(19) Europäisches Patentamt
European Patent Office
Office européen des brevets

(11) EP 2 584 035 A1

(12) **EUROPEAN PATENT APPLICATION**

(43) Date of publication:
**24.04.2013 Bulletin 2013/17**

(51) Int Cl.:
***C12N 9/08*** *(2006.01)* ***C12Q 1/28*** *(2006.01)*

(21) Application number: **11185833.8**

(22) Date of filing: **19.10.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Technische Universität Graz**
  **8010 Graz (AT)**
- **ACIB GmbH**
  **8010 Graz (AT)**
- **Karl-Franzens-Universität Graz**
  **8010 Graz (AT)**

(72) Inventors:
- **Krainer, Florian**
  **8010 Graz (AT)**
- **Naeaetsaari, Laura**
  **8010 Graz (AT)**
- **Glieder, Anton**
  **8200 Gleisdorf (AT)**
- **Kulterer, Martin**
  **8010 Graz (AT)**
- **Reichel, Victoria**
  **8010 Graz (AT)**

(74) Representative: **Gassner, Birgitta et al**
**REDL Life Science**
**Patent Attorneys**
**Donau-City-Straße 1**
**1220 Wien (AT)**

(54) **Horseradish peroxidase isoenzymes**

(57) The present invention relates to recombinant heme-containing horseradish peroxidase isoenzymes with improved technical properties.

EP 2 584 035 A1

## Description

The present invention relates to recombinant heme-containing horseradish peroxidase isoenzymes with improved properties.

## Background

Horseradish *(Armoracia rusticana)* is a herb cultivated worldwide in temperate regions, mainly due to the culinary use of its roots. However, the roots of *A. rusticana* are also used for the acquisition of highly versatile enzymes, the horseradish peroxidases (HRP) (Veitch, N.C. (2004) Phytochemistry 65, 249-259*).*

Peroxidases use hydrogen peroxide or various organic hydroperoxides as electron acceptors and are subsequently able to catalyze several oxidative reactions. They are encoded by various multigenic families, which are classified not only according to specific catalytic characteristics, but also according to structural properties and sequence information. A major mean for peroxidase taxonomy is the discrimination between heme-containing peroxidases and non-heme peroxidases. The latter contain five superfamilies, whereas the heme-containing peroxidases contain six superfamilies (Passardi, F., et al., (2007), Phytochemistry 68, 1605-11; Hofrichter, M., et al., (2010), Applied microbiology and biotechnology 87, 871-97).

All heme-containing peroxidases share some common features:

- A ferriprotoporphyrin IX prosthetic group at the active site with the key catalytic residues,
- Essential structural elements, such as two buried calcium-binding sites, four cysteine bridges and a buried salt-bridge,
- Hydrogen bonds and structural water molecules from the heme pocket to the distal calcium-binding site.

The non-animal heme peroxidases (synonymously referred to as plant peroxidases) comprise the majority of the so far identified heme peroxidase sequences. Several HRP isoenzymes have been described in literature. The horseradish peroxidase isoenzymes are referred to codes depending on their calculated isoelectric point (e.g. HRP A-isoenzymes have acidic isoelectric points, Band C-isoenzymes are neutral or neutral-basic, D- and E-isoenzymes are basic).

The isoenzyme HRP C as mentioned in literature corresponds to the Peroxidase C1A chain which is part of the peptide encoded by the gene *prxC1a* (GenBank: M37156.1; (17)). The corresponding C1A UniProt entry annotates a N-terminal hydrophobic leader peptide (positions 1-30), the Peroxidase C1A chain (31-338) and a C-terminal propeptide part (339-353) that is believed to convey vacuolar targeting (UniProtKB: P00433) (1). Further HRP nucleotide sequences published in the GenBank database encoded the following known HRP isoenzymes: C1 C (M60729.1), C1 B (M37157.1), C3 (D90116.1), C2 (D90115.1) and N (X57564.1) (17-19). These isoenzymes plus HRP A2 and E5 were also published at UniProt. The genes of nowadays published genomic DNA sequences encoding HRP isoenzymes are structured into four exons and three introns with identical splice site positions (1). Table 1 summarizes all isoenzymes published at UniProt or GenBank (effective March 2011):

Table 1

| isoenzyme | GenBank | UniProt | calculated I EP | calculated MW kDa |
|---|---|---|---|---|
| C1A | M37156.1 | P00433 | 5.67 | 38.82509 |
| C1B | M37157.1 | P15232 | 5.74 | 38.64586 |
| C1C | M60729.1 | P15233 | 6.21 | 36.54824 |
| C2 | D90115.1 | P17179 | 8.70 | 38.03538 |
| C3 | D90116.1 | P17180 | 7.50 | 38.17950 |
| A2 | - | P80679 | 4.72 | 31.89938 |
| E5 | - | P59121 | 9.13 | 33.72242 |
| N | X57564.1 | Q42517 | 7.48 | 35.12629 |

Applications of horseradish peroxidases are numerous and diverse. HRP is used in bioscience and biotechnology as well as in studies for medical applications. Thus, there is a need for HRP with improved properties.

## Summary of the Invention

The present invention relates to recombinant heme-containing horseradish peroxidase isoenzyme with improved technological properties such as altered glycosylation, improved catalytic properties or improved stability and different ranges of pH optima and improved surface interactions. In addition, the gene sequences of these new isoenzymes allow unlimited availability of pure isoenzymes by recombinant production, in contrast to environmentally dependent isoenzyme mixtures from plants or pure isoenzymes from expensive chromatographic purification.

The expression of recombinant plant peroxidases has been a matter of investigation since the early 1990s with the main focus lying on *E. coli* as expression host. However, the extremely low yields and formation of inclusion bodies turned out to be a major issue in the expression of eukaryotic proteins in *E. coli* and a lot of effort has been put into the dealing with this issue.

Alternatively to *E. coli,* various eukaryotic organisms have been used as expression hosts for plant peroxidases. Recombinant HRP has been expressed in an insect tissue culture via a baculovirus transfer vector as well as in *Saccharomyces cerevisiae.* In 2000, Morawski et al. (Protein engineering 13, 377-84) published the expression of HRP variants in *S. cerevisiae* and *Pichia pastoris* with significantly increased HRP activity in the culture supernatant. However the yields were still in the range of a few mg/L yeast culture. Further hosts for HRP expression are *Nicotiana tabacum, Spodoperta frugiperda* and *Beta vulgaris.*

Recombinantly expressed HRP has been subject to directed evolution in order to improve its expression and enzymatic properties such as specific activity or thermal stability. Morawski et al. (2000, 2001) described a 40-fold increase in specific activity after three rounds of random point mutagenesis and screening in a *S. cerevisiae* culture supernatant. The mutations were mainly located in either loop or surface regions. Even though the specific activity could be improved, the thermal stability was decreased. However, an additional single site mutation (N175S) led to a significant improvement in thermal stability.

Expression systems for expression of exogenous foreign genes in eukaryotic and prokaryotic cells are basic components of recombinant technology. Despite the abundance of expression systems and their wide-spread use, they all have characteristic disadvantages. For example, while expression in *E. coli* is probably the most popular as it is easy to grow and is well understood, eukaryotic proteins expressed therein are not properly modified. Moreover, those proteins tend to precipitate into insoluble aggregates and are difficult to obtain in large amounts.

The present invention encompasses the expression of the HRP isoenzmes (or a functional derivatives thereof) in either prokaryotic or eukaryotic cells.

Any cultivated prokaryotic or eukaryotic host cell, e.g. bacterial, fungal, plant, human and animal host cells, may be used as a host cell.

Preferred prokaryotic host cells may be e.g. derived from bacteria, such as *Escherichia coli, B. subtilis, salmonella, pneumococcus,* etc., or from algae, fungi, etc.

Alternatively, eukaryotic host cells, e.g. cells from multi-cellular organisms, may be selected. Compared to prokaryotic expression systems, the advantages of eukaryotic systems are adequate folding including correct disulphide bridge formation, processing and the ability to perform posttranslational modifications of heterologously expressed eukaryotic proteins. Cells from eukaryotic organisms are particularly preferred, if posttranslational modifications, e.g. glycosylation of the encoded proteins, are required (N and/or O linked). Typical examples of suitable eukaryotic host cells include yeasts such as *Saccharomyces cerevisiae, Schizosaccharomyces pombe Klyveromycis lactis, Yarrowia lipolytica, Arxula adenivorans, Pichia angusta* and *Pichia pastoris.* Yeast host cells systems offer an attractive alternative to prokaryotic host cells, because they combine many advantages of other eukaryotic host cells, like folding, processing and posttranslational modification of heterologous proteins, with the ability to produce high protein yields ranging up to grams per liter culture volume. Compared to higher eukaryotes, protein yields obtained in *Pichia pastoris* expression cultures are relatively high, ranging up to grams per liter culture volume. In *Pichia pastoris,* heterologously expressed proteins can be secreted into the culture medium by fusion to the yeast mating type $\alpha$-factor signal peptide. Other examples of suitable eukaryotic cells include mammalian host cells and further host cells, in particular host cells established for laboratory use, such as HEK293-, Sf9-, CHO(Chinese hamster ovary)- or COS-cells.

A number of plant expression systems exist as well. One advantage of plants or algae in an expression system is that they can be used to produce pharmacologically important proteins and enzymes on a large scale and in relatively pure form. In addition, micro-algae have several unique characteristics that make them ideal organisms for the production of proteins on a large scale.

Due to following advantages *P. pastoris* is the most preferred host for recombinant protein expression

- A broad knowledge base of this expression system due to the beforementioned decision of Phillips Petroleum (continued by RCT) to release the *P. pastoris* system to academic research laboratories (Cregg, J. M., et al., (2000) Molecular Biotechnology 16, 23-52).
- A high similarity of the methods for molecular genetic manipulation of *P. pastoris* to those applied to *Saccharamoyces*

*cerevisiae* which is one of the best-characterized systems in this field of science.

- Known and publicly available high quality annotated genome sequences of several most frequently used strains.
- A strong preference for respiratory growth facilitates the ability to culture *P. pastoris* to high cell density, which further enables the expression of foreign proteins for basic laboratory research, as well as for industrial manufacture up to concentrations of several grams per liter.
- An eukaryotic protein synthesis pathway and the ability to perform higher eukaryotic protein complex posttranslational modifications (polypeptide folding, glycosylation, acylation, methylation, disulfide bond formation, proteolytic processing, targeting to subcellular compartments).
- The AOX1 promoter of the gene encoding alcohol oxidase 1 has properties highly desirable for the controlled high-level expression of foreign protein: Strong repression on carbon sources like glucose or glycerol, but over 1000-fold induction when confronted with methanol as a sole carbon source.
- AOX1 promoter variants which allow high levelprotein expression without the use of methanol.
- The possibility to produce both, secreted and intracellular recombinant protein. Since *P. pastoris* only secretes very low levels of endogenous proteins, secreted recombinant protein comprises the vast majority of the total protein in the medium. Thus, the secretion of the heterologous protein serves as valuable first step in purification.
- The absence of endotoxins, oncogenic and viral DNA in *P. pastoris* products.
- The GRAS status of *Pichia pastoris.*
- *P. pastoris* is able to perform typical eukaryotic posttranslational modifications, such as the processing of signal sequences, correct folding, formation of disulfide bridges, O- and N-linked glycosylations.

The ability to process signal sequences plays a major role when a foreign gene is fused to the S. cerevisiae $\alpha$-factor prepro signal sequence which facilitates the secretion of the foreign protein.

Another important aspect of posttranslational modifications by *P. pastoris* is glycosylation. The glycosylation patterns of mammalian cells and *P. pastoris* differ greatly, which might have an impact on the activity of the recombinant protein and constitute a significant problem in the use of recombinant proteins by the pharmaceutical industry since they might be highly antigenic or affected in their biological activity. Efforts are made to genetically engineer *P. pastoris* to humanize its glycosylation behavior (Cregg et al. (2000)).

*P. pastoris* adds O-oligosaccharides to the hydroxyl groups of serine and threonine residues of secreted proteins. Unlike mammals, *P. pastoris* solely adds Man residues. O-glycosylation starts in the endoplasmatic reticulum (ER) where one Man residue is transferred from Man-P-dolichol to a Ser/Thr residue. The Ser/Thr residues used for O-glycosylation in *P. pastoris* are not necessarily the same residues as in the native host. In the Golgi apparatus, sugar transferases add further $\alpha$1,2-linked Man residues. The extent of O-glycosylation by *P. pastoris* differs among the produced recombinant proteins.

Asparagine residues found in the conserved amino acid pattern Asn-X-Ser/Thr are subject to possible N-glycosylation. The initial steps of N-linked glycosylation are the same in yeast and most higher eukaryotes: N-acetylglucosamine is transferred from uridine diphosphate-GlcNAc onto dolichol phosphate on the cytoplasmic face of the ER membrane. Further addition of GlcNAc and Man leads to the structure $Man_5GlcNAC_2$-P-dolichol. A flipase facilitates the translocation to the luminal face of the ER membrane where the Man5GlcNAc2-P-dolichol is further extended to $Glc_3MangGlcNAc_2$-P-dolichol. The sugars from this structure are transferred co-translationally to the Asn residue at the Asn-X-Ser/Thr motive by an oligosaccharyl-transferase complex. After removal of three Glc residues and one Man residue to $Man_8GlcNAc_2$, the glycopeptide is transported to the Golgi apparatus, which is where the glycosylation pathways of yeasts and mammals diverge. Contrary to mammals, *P. pastoris* does not reduce the $Man_8GlcNAc_2$ structure, but rather further extends it with additional Man residues catalyzed by Golgi mannosyltransferases. Also mannose phosphate diesters have been found in *P. pastoris* N-glycans.

An approach to increase the yield of functionally expressed recombinant protein in *P. pastoris* is the coexpression of proteins which either lead to an improvement in the host cell's metabolism or whose activity supports the functional expression of the actual target protein.

For example, Schroer et al. (2010, *Metabolic engineering 12*, 8-17) showed a significantly improved whole-cell biotransformation reaction (using the NADH-dependent butanediol dehydrogenase) by overexpressing the MUT pathway enzyme FLD, which facilitates NADH regeneration.

The tremendously high induction of AOX1 promoter-driven heterologous genes can lead to extremely high amounts of protein. These tend to activate the cell's unfolded protein response, indicating that correct folding of the peptides and disulfide bridge formation are limiting factors during high-level expression. In order to approach this bottleneck, protein disulfide isomerase (PDI) and other helper proteins can be coexpressed. PDI is described as an ER-residing protein of the thioredoxin superfamily with chaperone- and peptide binding functions. PDI overexpression has been shown to further improve the expression of secretory recombinant proteins by extending the secretory capacities of cells that already reached their limit in the production of functional recombinant protein without coexpressed PDI.

Horseradish peroxidase isoenzymes form a large group of extremely versatile enzymes with numerous applications these days and many more potential applications in future. Nonetheless, only a small number of isoenzymes has been characterized, published or even identified on the level of either amino acid sequence or nucleotide sequence.

In an aspect of this invention, novel recombinant HRP isoenzyme sequences on genome DNA level are provided.

In a further aspect of this invention, novel recombinant HRP isoenzymes are heterologously expressed.

A further aspect of this invention is a recombinant heme-containing horseradish peroxidase isoenzyme encoded by a nucleic acid comprising a sequence selected from the group consisting of SEQ ID NO:1 to SEQ ID NO:49, and functionally active variants thereof.

A further aspect of the invention is an isoenzyme, wherein said isoenzyme is less glycosylated as compared to the isoenzyme C1A.

A further aspect of the invention is an isoenzyme, wherein said isoenzmye has a thermal stability of at least 2 weeks at 20-25°C and at least several months at 4°C.

The term several months as used herein refers to a period of 1 to 24 months, preferred to a period of 3 to 18 months and most preferred to a period of 6 to 12 months.

A further aspect of the invention is an isoenzyme, wherein said isoenzmye has a biological acitvity of at least 500 U/mg, preferred of at least 750 U/mg, more preferred of at least 1.000 U/mg and most preferred of at least 1.200 U/mg in the ABTS assay. 1 unit is defined as the amount of enzyme that converts 1 $\mu$mol ABTS per minute with $K_M$ = 0.44 mM ABTS.

A further aspect of the invention is an isoenzyme, wherein said isoenzmye has a calculated isoelectric point of less than 5 or greater than 6.

A further aspect of the invention is an isoenzyme, wherein said isoenzmye has a lower $K_m$ when compared to the isoenzyme C1A.

A further aspect of the invention is a vector comprising a polynucleotide encoding an isoenzyme.

A further aspect of the invention is a host cell comprising a vector encoding a mutant of an isoenzyme displaying a reduced number of lysine residues.

A further aspect of the invention is a host cell comprising a vector encoding an isoenzyme.

A further aspect of the invention is a host cell, wherein said host cell is a prokaryotic cell, preferably *E. coli* or *B. subtilis* or eukaryotic cell, preferably a yeast cell.

A further aspect of the invention is a host cell, wherein said host cell is a Pichia cell, preferably a *P. pastoris* or *P. angusta* cell.

A further aspect of the invention is a method for producing a recombinant heme-containing horseradish peroxidase isoenzyme which comprises

a. providing a recombinant host cell engineered to express a polynucleotide,
b. culturing the host cell under conditions suitable for obtaining the isoenzyme, wherein the expression product of the host cell is reacting with heme; and
c. recovering the isoenzyme from the culture.

A further aspect of the invention is the use of a horseradish peroxidase isoenzyme according as a reagent for organic synthesis and biotransformation, preferably in polymerizations, coupled enzyme assays, chemiluminescent assays, and immunoassays.

A further aspect of the invention is the use of a horseradish peroxidase isoenzyme in a biosensor, in diagnostics, in bioremediation or in chemical synthesis.

A further aspect of the invention is a horseradish peroxidase isoenzyme for use in targeted cancer therapy.

A further aspect of the invention is a recombinant synPDI enzyme encoded by a nucleic acid comprising a sequence of SEQ ID NO:52 and functionally active variants thereof.

The HRP isoenzymes of the A2 group have the so far most acidic isoelectric point. A low isoelectric point is thought to indicate increased enzymatic activity and elevated stability at lower pH, which renders HRP A2 isoenzymes highly interesting isoenzymes, since (bio-) catalysis at low pH is of considerable industrial interest.

Thus, in one aspect of the invention the recombinant HRP isoenzyme is HRP A2A.

In a further aspect of this invention a protocol for the purification of the expressed HRP isoenzyme was established that allows enzyme purities comparable to or higher than commercially available HRP preparations.

In a further aspect of this invention the purified isoenzyme was characterized in terms of posttranslational modifications, its enzymatic activity towards the two standard substrates 2,2'-azino-bis(3-ethylbenzthiazoline-6-sulphonic acid) (ABTS) and guaiacol, and its stability in different buffers, at different temperatures and at different protein concentrations.

DESCRIPTION OF THE DRAWINGS

Figure 1: Aligned Sanger sequencing reads of C1 C: The double peak signals A/G1828 and C/G1861 can be seen in three out of seven reads.

Figure 2: Phylogenetic tree of aligned HRP isoenzymes. The published HRP isoenzymes are closer related to each other than to most of the newly discovered HRPs.

Figure 3: Sequence logos of signal peptide cleavage sites predicted by SignalP 3.0.

a) Sequence logo of all predicted signal peptide cleavage sites.

b) Sequence logo of the predicted signal peptide cleavage sites of the isoenzymes C1A, C1B, C1C, C2, A2A, E5, 01805, 22684 and 02021.

Figure 4: RNA secondary structure of the synthetic HRP A2A gene. The predicted RNA secondary structure with a total free energy of - 167 kcal/mol.

Figure 5: GC content of the synthetic HRP A2A gene. The GC content over the full sequence; calculated with an averaging window of 30 bp, sliding over the sequence with a stepping value of 1.

Figure 6: HRP activities from the first screen of A2A and coexpressed PDI genes. G4, B5, D6, A9 were done as duplicates. A2AMutSF5 shows the starting strain's activity. + empty plasmid shows the influence of the cotransformed empty plasmid. + PD1704, + synPDI, + synPDI N314H show the average of the measured HRP activity under PDI coexpression. These results have been obtained from one 96-DWP cultivation per PDI.

Figure 7: HRP activities from the rescreen of A2A + synPDI N314H coexpression. The activity control strains G4, B5, D6, A9 were done as duplicates. A2AMutSF5 and + empty plasmid represent the starting strain activity and its activity plus "coexpressed" empty plasmid, respectively. + synPDI N314H shows the measured activities of clones coexpressing A2A + synPDI N314H (average of quadruplicates).

Figure 8: Total HRP activities of the cultivated strains. The MutS and MutS synPDI N314H strains were measured as negative controls for HRP activity. The A2AMutSF5 and A2AMutSF5 synPDI N314H strains showed edincreasing activity over time. The measured activity from A2AMutSF5 synPDI N314H was slightly higher than the ones from the A2AMutSF5 strains.

Figure 9: Total HRP activities of the fractions from StrepTactin affinity chromatography. HRP A2ANstrep did not bind to the StrepTactin column and eluted in the first fractions. Already in the flowthrough fraction, collected during the loading of the sample, HRP activity was measured. In the subsequent two washing fractions the remaining HRP activity was detected.

Figure 10: HIC chromatogram and measured HRP activities. Functional HRP eluted from ~ 200 mL - 475 mL. Proteins without HRP activity eluted from ~ 560 - 600 mL.

Figure 11: The 10 different pHs (9.5 - 5.0) are indicated above the columns (2 - 11). In the rows A and B, HRP A2A was changed to the respective buffers (without QFF), then the standard ABTS assay was performed. In the rows C and D, 15 $\mu$L of the test tube supernatant were used for the ABTS assay: Only at pH 9.5, no HRP activity could be detected. In the rows E and F, 15 $\mu$L of the supernatant of the buffers at pH 9.5, 9.0 and 8.5 were used for the ABTS assay after eluting HRP A2A from the QFF material.

Figure 12: Anion exchange chromatogram and measured HRP activities. The fractions exposing HRP activity are enlarged. HRP A2A eluted from ~ 70 - 115 mL.

Figure 13: HRP activities and Rz values of the fractions from ammonium phosphate precipitation. (a) shows the HRP activities measured from the pellets of the various precipitation steps redissolved in buffer QFF-A and the supernatant of the last precipitation step. (b) shows the Rz values of the fractions.

Figure 14: Size exclusion chromatogram and measured HRP activities. The fractions exposing HRP activity are enlarged below. HRP A2A eluted from ~ 80 - 90 mL.

Figure 15: SDS-PAGE gel picture of the purified HRP A2A. The PageRuler prestained Protein Ladder was used as protein standard and was run until the ~25 kDa band reached the lower end of the gel. The band at ~ 35 kDa in the second gel lane was the size exclusion chromatography fraction that contained the purified HRP A2A.

Figure 16: IEF gel picture of commercially available HRP preparations and the purified HRP A2. The IEF Marker 3-10, Liquid Mix from SERVA Electrophoresis GmbH was used as standard and was run in the flanking lanes. HRP A2A showed an IEP of pH 3.5- 4.2. The Sigma preparations II, VI, VI-A and XII seemed to contain a similar HRP isoenzyme, among others. Type I contained predominantly isoenzymes with an IEP at ~ pH 5. The Toyobo HRP seemed to contain an isoenzyme slightly more basic than A2A (MS-MS analysis of the Toyobo HRP revealed the sequence of isoenzyme C1 a as the major component of this commercial preparation from the plant. This isoenzyme has a calculated isoelectric point of 5,67).

Figure 17: pH profile of HRP A2A with ABTS. pH 4.5 was identified as optimum for the catalysis of ABTS by HRP A2A. By the photometric assay no activity was measured lower than pH 3.5 or higher than pH 9.0. All points were measured as triplicates.

Figure 18: Standard curves from BSA and HRP VI-A dilutions. BSA and HRP VI-A gave comparable results in the performed BCA assay for protein quantitation and were equally suitable for determining the concentration of HRP A2A. Each point was measured as triplicate.

Figure 19: Kinetics of HRP A2A with ABTS as saturation curve and as Lineweaver-Burk plot. (a) The calculated ABTS units/mg were plotted against the respective ABTS concentration. (b) The reciprocal values of the calculated reaction rates were plotted against the reciprocal respective ABTS concentration. The trendline crosses the x-axis at the reciprocal negative value of $K_M$ and the y-axis at the reciprocal value of $V_{max}$. All points were measured as triplicates.

Figure 20: pH profile of HRP A2A with guaiacol. The optimal pH for the conversion of guaiacol by HRP A2A was found to be at pH 5.0. The activity stayed high until pH 7.0. No activity was measured lower than pH 3.5 or higher than pH 9.0. All points were measured as triplicates.

Figure 21: Kinetics of HRP A2A with guaiacol as saturation curve and as Lineweaver-Burk plot. (a) The calculated guaiacol units/mg were plotted against the respective guaiacol concentrations. (b) The reciprocal values of the calculated reaction rates were plotted against the reciprocal respective guaiacol concentrations. The trendline crosses the x-axis at the reciprocal negative value of $K_M$ and the y-axis at the reciprocal value of $V_{max}$. All points were measured as triplicates.

Figure 22: Influence of pH on the stability of HRP A2A. The optimal pH range for storing HRP A2A was pH 7.0 - 10.0. At lower pH the activity was significantly decreased over time. All points were measured as triplicates.

Figure 23: Influence of temperature on the stability of HRP A2A. At 50 °C and 65 °C the activity was completely abrogated within 10 min and 3 min, respectively. At 37°C, HRP activity could be detected for 30 min. At 20°C, the activity decreased to 27 % of the initial activity in 60 min. At 4°C, 75 % of the starting activity could still be measured after 60 min. All measuring points were done as triplicates.

Figure 24: Influence of protein concentration on the stability of HRP A2A. HRP A2A stayed stable at concentrations $\geq$ 0.6 ng/$\mu$L. At lower concentrations, the HRP activity decreased within one day. All points were measured as triplicates.

Figure 25: HRP sequences from next-generation sequencing. The HRP sequences found in the transcriptome and their corresponding published sequences are shown. Differences between the contig-derived translated transcriptome sequences and the published sequences are marked in grey.

Figure 26: Annealed adaptor for genome walking: Adaptor strand 1 was annealed either to adaptor strand 2.a, 2.b or 2.c, depending on the desired 5'-overhang (red). The last nucleotide at the 3'-end of the adaptor strand 2 did not match to adaptor strand 1, in order to prevent elongation from that 3'-end on. Marked in yellow+green is the binding site of the AdaptorPrimer1, marked in green+blue is the binding site of the AdaptorPrimer2.

Figure 27: The two Sanger-verified sequences 08562.1 and 08562.4 are shown in an alignment with the corresponding transcriptome contigs.

Figure 28: Sequence alignment of 5'-UTR (marked in grey) of the Sanger verified C1 C plus its N-terminus to the protein sequence of the N-terminus of C1 B.

Figure 29: Sequence alignment of synPDI, synPDI N314H and PDI704.

The term "variant" or "functionally active variant" of an enzyme as used according to the invention herein means a sequence resulting from modification of the parent sequence by insertion, deletion or substitution of one or more amino acids or nucleotides within the sequence or at either or both of the distal ends of the sequence, and which modification does not affect (in particular impair) the activity of this sequence. In a preferred embodiment the functionally active variant

a) is a biologically active fragment of the amino acid or the nucleotide sequence, the functionally active fragment comprising at least 50% of the sequence of the amino acid or the nucleotide sequence, preferably at least 60%, preferably at least 70%, more preferably at least 80%, still more preferably at least 90%, even more preferably at least 95% and most preferably at least 97%, 98% or 99%;

b) is derived from the amino acid or the nucleotide sequence by at least one amino acid substitution, addition and/or deletion, wherein the functionally active variant has a sequence identity to the amino acid or the nucleotide sequence or to the functionally active fragment as defined in a) of at least 50%, preferably at least 60%, preferably at least 70%, preferably at least 80%, still more preferably at least 90%, even more preferably at least 95% and most preferably at least 97%, 98% or 99%; and/or c) consists of the amino acid or the nucleotide sequence and additionally at least one amino acid or nucleotide heterologous to the amino acid or the nucleotide sequence, preferably wherein the functionally active variant is derived from or identical to any of the variants of any of the sequences of SEQ ID NO: 1 - 49.

"Percent (%) nucleotide sequence identity" with respect to the polynucleotide sequences identified herein is defined as the percentage of nucleotide residues in a candidate sequence that are identical with the nucleotide residues in the specific polynucleotide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

The functionally active variant may be obtained by sequence alterations in the amino acid or the nucleotide sequence, wherein the sequence alterations retains a function of the unaltered amino acid or the nucleotide sequence, when used in combination of the invention. Such sequence alterations can include, but are not limited to (conservative) substitutions, additions, deletions, mutations and insertions.

In a specific embodiment of the invention the polypeptide or the nucleotide sequence as defined above may be modified by a variety of chemical techniques to produce derivatives having essentially the same activity (as defined above for fragments and variants) as the modified polypeptide or the nucleotide sequence, and optionally having other desirable properties. Other desirable properties are, for example, the increase in stability as measured by the pH stability and/or temperature stability of the enzyme, the increase in acitivity as measured by the ABTS assay or increased stability at elevated hydrogen peroxide concentrations.

The variant of the polypeptide or the nucleotide sequence is functionally active in the context of the present invention, if the activity of the composition of the invention including the variant (but not the original) amounts to at least 50%, preferably at least 60%, more preferred at least 70%, still more preferably at least 80%, especially at least 90%, particularly at least 95%, most preferably at least 99% of the activity of the enzyme as used according to the invention including the amino acid or the nucleotide sequence without sequence alteration (i.e. the original polypeptide or the nucleotide sequence).

Functionally active variants may be obtained by changing the sequence as defined above and are characterized by having a biological activity similar to that displayed by the respective sequence of SEQ ID NO: 1 - 49 from which the variant is derived, including the ability of HPR isoenzymes to show higher stability in technical applications, the ability to react with substrates at low concentrations, to show improved interaction with other proteins and surfaces, higher catalytic efficiency, advantages for commercial manufacturing and improved properties for *in vivo* applications.

Applications of horseradish peroxidases are numerous and diverse. HRP is used in bioscience and biotechnology as well as in studies for medical applications.

A very common application of HRP is nowadays in biosensors, predominantly for the real-time quantification of $H_2O_2$ but also for the in vivo detection of glucose, ethanol or tumor markers via co-immobilization with a $H_2O_2$-producing enzyme.

HRP is further used in numerous diagnostic applications. Generally, a chromogen is oxidized by HRP with $H_2O_2$, which is generated by a substrate-specific oxidase (e.g. glucose oxidase in colorimetric diagnostic kits for the determination of glucose in blood serum or plasma). Alternatively HRP is conjugated to specific antibodies to be used in diagnostic applications, generating a dye or fluorescence signal in case the antibodie binds to a target.

There is also a trend for miniaturization in diagnostics, for example, a portable sequential cross-flow immunoanalytical device using HRP for signal generation and hereby simplified the complex traditional ELISA procedure.

Another application for horseradish peroxidases is in bioremediation, such as the detoxification of phenolic wastewater. Efforts are made to immobilize HRP in order to facilitate reusability and increase enzyme stability: HRP was used for the removal of natural and synthetic estrogens from wastewater.

Chemical synthesis is another field in which HRP-catalyzed reactions can be applied. Oxidative reactions, such as oxidative dehydrogenation and polymerization of aromatic compounds, heteroatom oxidation and epoxidation constitute interesting enzymatic abilities for potential applications. Also N- and O-dealkylation or selective hydroxylation are nowadays used for small-scale organic synthesis.

A highly interesting reaction of HRP is the formation of cytotoxic radical species from the non-toxic plant hormone indole-3-acetic acid (IAA), also known as auxin, via a one-electron oxidation step without additional $H_2O_2$. An application of this reaction lies in targeted cancer therapy (e.g. antibody-, gene- or polymer-directed enzyme-prodrug therapies), where the glycosylation of HRP is an important factor regarding the clearance of the antibody from the cells.

## Examples

### 1. Kits, protocols and methods

1.1 Agarose gel electrophoresis

1 % agarose gels were made with 250 mL 1x TAE buffer and approximately 2 μL EtBr (≥ 98 % ethidium bromide). DNA samples were mixed with 6x or 2x Orange Loading Dye and pipetted to the wells of the gel, next to 5 μL

or 10 μL of 0.1 μg/μL GeneRuler™ 1 kb DNA Ladder. Electrophoresis was run for approximately 50 min at a voltage of 120 V. After the run, the samples were analyzed with the GelDoc-It™ Imaging System. Preparative agarose gels were run for approximately 2 h at a voltage of 90 V. The samples were cut out off the gel at the Chroma 43.

1.2 SDS-PAGE

Protein samples were prepared as recommended by Invitrogen™ life technologies and pipetted to the wells, next to 5 μL PageRuler™ Prestained Protein Ladder. Electrophoresis was run for approximately 1 h at a voltage of 200 V. Staining of the gels was done with approximately 100 mL SDS gel staining solution for 20 min after heating the staining solution plus the gel close to the boiling point in the microwave. Destaining was done three times with 100 - 200 mL SDS gel destaining solution for 20 min each after heating the destaining solution plus the gel close to the boiling point.

1.3 Isoelectric focusing gel electrophoresis

For isoelectric focusing, Novex IEF gels were used. Protein samples were handled in accordance to the recommendations from Invitrogen™ life technologies. As a reference, 5 μL of the IEF Marker 3-10, Liquid Mix from SERVA Electrophoresis GmbH were used. Buffers and run conditions were used as recommended. After the run, the gel was fixed for 15 min in 12 % TCA (50.0 g ≥ 99.5 % trichloroacetic acid dissolved in 500 mL $dH_2O$). For staining, the SimplyBlue SafeStain solution from Invitrogen life technologies was used as recommended.

1.4 Plasmid minilysate preparation

The GeneJET Plasmid Miniprep Kit (Fermentas GmbH, St. Leon-Rot, Germany) was used for plasmid isolation according to the manufacturer's recommendations with the following deviations: Cells were abraded from half an agar plate with a toothpick, all incubation times were doubled, plasmid elution was done with 40 μL $dH_2O$ (heated to 65 °C).

1.5 DNA purification

DNA purification was done after PCR, preparative agarose gel electrophoresis and DNA restriction with the Wizard SV Gel and PCR Clean-Up System (Promega GmbH, Mannheim, Germany). Applied deviations from the manufacturer's recommendations were doubled incubation times and elution of DNA with 40 μL $dH_2O$ (heated to 65 °C).

1.6 Molecular cloning

**Standard Ligations**

Ligation of an insert to a vector was done over night at 16 °C with T4 DNA Ligase (400 u/μL, 10x T4 DNA Ligase Reaction Buffer/New England BioLabs® Inc., Ipswich, MA, USA) according to the manufacturer's recommendations.

**Cloning to the pJET1.2blunt vector**

The CloneJET™ PCR cloning Kit (Fermentas GmbH, St. Leon-Rot, Germany) was used for cloning PCR products to the pJET1.2blunt vector (see 3.4.2 Plasmid constructs) according to the manufacturer's recommendations. The incubation time for ligation was 30 minutes at room temperature with a molar vector: insert ratio of 1:3. Desalting was done for 20 min against $dH_2O$ at room temperature prior to electroporation (see below).

1.7 Applied PCR techniques

**Standard PCR**

Standard PCRs were performed with Phusion® High-Fidelity DNA-Polymerase, and genomic DNA from *A. rusticana* was used as template DNA. The annealing temperature was by default chosen to be 2 °C below the primer DNA melting temperature, calculated with EditSeq (Davis, Botstein, Roth melting temperature). Primers were used in a final concentration of 400 nM, dNTPs in a final concentration of 200 μM. The elongation time was calculated in dependence of the expected PCR product length, considering a processivity of 1 kb per 10 - 30 s of the Phusion® High-Fidelity DNA-Polymerase.

In case the PCR did not yield any product under standard conditions (either buffer HF or buffer GC), the reaction conditions were changed: The reaction was repeated with addition of 100 % DMSO and 50 mM $MgCl_2$ (final concentration of 2 % DMSO and 2 mM $MgCl_2$). Further, the annealing temperature was varied +/- 5 °C of the primer DNA melting temperature calculated with EditSeq (Davis, Botstein, Roth melting temperature).

The PCR products were cut from a preparative agarose gel, purified and sent to LGC Genomics GmbH, Berlin, Germany for Sanger sequencing.

**Genome Walking**

Approximately 2 μg genomic DNA from *A. rusticana* were digested over night with Bsp1431, BsaWI, PsuI, XhoII, HindIII with the corresponding buffer in order to get fragments of 1 - 5 kb size. The digestion was stopped by heat inactivation of the enzymes, the fragmented DNA was precipitated with EtOH (cf. 3.6.13 Genomic DNA isolation and determination of gDNA quality and quantity) and the pellet was dissolved in 30 μL $dH_2O$.

An adaptor was created by annealing adaptor strand 1 (primer gWalking-AdaptorStrand1) either to adaptor strand 2.a (primer gWalkingAdaptorStrand2a), 2.b (primer gWalkingAdaptorStrand2b) or 2.c (primer gWalkingAdaptorStrand2c), depending on the 5'-overhang created by the respective restriction enzyme (see figure 26).

Adaptor strand 1 was annealed either to adaptor strand 2.a, 2.b or 2.c, depending on the desired 5'-overhang (red). The last nucleotide at the 3'-end of the adaptor strand 2 did not match to adaptor strand 1, in order to prevent elongation from that 3'-end on. Marked in yellow+green is the binding site of the AdaptorPrimer1, marked in green+blue is the binding site of the AdaptorPrimer2.

In the annealing reaction, adaptor strand 1 was mixed in 1:1 molar ratio with adaptor strand 2.a / 2.b / 2.c (i.e. 13.7 μL 100 μM adaptor strand 1 + 4.0 μL 100 μM adaptor strand 2) and heated to 95 °C for 5 min. Afterwards, the thermomixer was turned off and the mixtures were left to cool to room temperature over night. The three differently annealed adaptors (1 +2a, 1+2b, 1+2c) were ligated for 3 h at room temperature with T4 DNA Ligase to the digested gDNA fragments, considering the specific 5' overhangs that have been created by the used restriction enzymes (i.e. adaptor 1+2a was ligated to gDNA digested with either XhoII, PsuI or Bsp143I, adaptor 1+2b was ligated to gDNA digested with BsaWI, adaptor 1+2c was ligated to gDNA digested with HindIII). The ligation reaction was stopped by incubation for 5 min at 70 °C and 70 μL TE buffer were added.

Two gene-specific primers and two adaptor primers were designed. The gene-specific primers were designed to bind approximately 100 bp away from the end of the known sequence, considering that no restriction site of the restriction enzymes used for gDNA digestion lied between the primer-binding site and the end of the known sequence. AdaptorPrimer1 (5'GTAATACGACTCACTATAGGGC3') (SEQ ID NO:54) and GeneSpecificPrimer1 were used as a primer pair for a first PCR with 1 μL of the gDNA+adaptor ligation product as template DNA. 1 μL of the first PCR mix was used as template for a second PCR with AdaptorPrimer2 (5'ACTATAGGGCACGCGTGGT3') (SEQ ID NO:55) and GeneSpecificPrimer2 as primer pair. This second primer pair was designed to bind within the first PCR product. Both PCR steps were performed with an elongation time of 50 s.

A gene-specific DNA fragment as product from the second PCR was isolated from a preparative agarose gel, purified and sent to Sanger sequencing (LGC Genomics GmbH, Berlin, Germany), using AdaptorPrimer2 and the corresponding GeneSpecificPrimer2.

**Colony PCR**

Template DNA was isolated from single *P. pastoris* colonies using the Bust n' Grab protocol (see chapter 3.6.13). GoTaq® polymerase was used in accordance to the manufacturer's recommendations with addition of $MgCl_2$ to a final concentration of 2 mM $MgCl_2$ in the PCR mix. The primers were used at a final concentration of 100 nM each. The PCR product was analyzed via agarose gel electrophoresis.

**qPCR**

Quantitative PCR was performed similarly to the protocol described by Abad et al. ((2010) Biotechnology journal 5, 413-20):

Genomic DNA isolated from *P. pastoris* as described below was used as template DNA. The 2x Power SYBR Green Master Mix (Applied Biosystems, Foster City, CA, USA) was used for the PCR reactions. The ARG4 gene was used as an internal reference, the KanMX6 gene and the synPDI gene were used in parallel as target genes for determination of the PDI copy number, the Zeocin syn gene was used as an indirect target gene for the determination of the HRP copy number (all vectors used for HRP transformation also carried the Zeocin syn gene). Strains with verified single copy integration of the target gene were used as reference strains. The used primers and their concentrations are depicted in table 2. All reactions were done as duplicates.

Table 2

| gene | primer number | final concentration mM |
|---|---|---|
| *ARG4* | P09-478 | 200 |
| | P09-479 | 250 |
| Zeocin syn | P09-338 | 250 |
| | P09-337 | 250 |
| syn*PDI* | P07-515 | 200 |
| | P07-514 | 250 |
| KanMX6 | P10-828 | 250 |
| | P10-829 | 250 |

Primers used for qPCR with ARG4 as reference gene and Zeocin syn/synPDI/ KanMX6 as target genes and final concentrations of the various primers in the PCR reaction mix.

1.8 Transformation to *Escherichia coli* Top 10F'

DNA (approximately 100 ng, maximum volume of 20 μL) and competent cells (80 μL E. coli Top 10F') were mixed and incubated for 5 min on ice in electroporation cuvettes. The cells were pulsed with of 2.5 kV, 200 Ω, 25 μF. 500 μL SOC medium were added immediately. The cell suspension was incubated for 1 h, 37 °C, 600 rpm. 10 μL, 100 μL and the rest of the cell suspension were plated on LB-agar plates containing the respective antibiotic. Incubation of the plates was done at 37 °C over night.

1.9 Transformation to *Pichia pastoris*

Transformation of *P. pastoris* was done as described by Lin-Cereghino et al. ((2005) BioTechniques 38, 44-48):

> Competent cells were prepared by inoculating a single colony of the respective strain to 50 mL YPD medium and grown over night at 28 °C, 110 rpm. From this pre-culture a main-culture was grown from $OD_{600}$ = 0.2 to a final $OD_{600}$ of 0.8 - 1.0 (again in YPD medium). The cells were harvested and 80 μL of them were mixed with 3 - 4 μg DNA (maximum volume of 25 μL) and incubated for 5 min in electroporation cuvettes on ice. After pulsing the cells with 2.0 kV, 200 Ω, 25 μF, 500 μL 1 M sorbitol were added immediately, followed by 500 μL YPD medium. The cells were incubated for 2 h, 28 °C, 110 rpm for regeneration, then plated on YPD-agar plates (10 μL, 100 μL, rest) containing the respective antibiotic(s) and incubated at 28 °C for 2 - 3 days.

1.10 Micro-scale cultivation of *Pichia pastoris* in 96-deep well plates

In order to screen for *P. pastoris* clones expressing active HRP, a micro-scale cultivation in 96-deep well plates (DWP) was done similar to the protocol described by Weis et al. ((2004) FEMS yeast research 5, 179-89):

> Cells from a single colony were transfered to 250 μL BMD1% per well. Two wells per plate were left empty as negative controls for cell growth, two wells were inoculated with *P. pastoris* MutS as negative controls for the recombinant enzyme's activity and two wells per strain were inoculated with *P. pastoris* strains already successfully expressing the inquired recombinant enzyme as positive controls for its activity. They were incubated at 28 °C, 320 rpm, 80% humidity. After approximately 60 h, 250 μL BMM2 were added per well for the induction of recombinant protein expression. 12 h, 24 h and 36 h after the start of induction, 50 μL BMM10 were added per well. The cultivation was stopped 48 h after the start of induction, the cells were centrifuged at 3.000 x g for 10 min and the supernatant was checked for HRP activity with the ABTS assay.

1.11 Small scale cultivation of *Pichia pastoris* in 2 L-shake flasks

A single colony was inoculated to 200 mL BMD1 % and incubated for 60 h at 28 °C, 110 rpm, 80% humidity. Induction of protein expression was done by addition of 20 mL BMM10 and further additions of 2 mL MeOH 12h, 24 h and 36 h after the start of induction. 48 h after induction start, the cells were pelleted by centrifugation at 3.000 x g for 10 min. The supernatant was checked for HRP activity by applying the ABTS assay.

1.12 <u>Cultivation of *Pichia pastoris* in 1.5-L bioreactors using the DASGIP system</u>

One single colony per strain of interest was inoculated to 50 mL BMGY as a pre-pre-culture and incubated for 24 h at 28 °C, 110 rpm, 80% humidity. The pre-pre-cultures were used to inoculate pre-cultures with a starting $OD_{600}$ = 0.2 in 60 mL BMGY. The pre-cultures were incubated over night at 28 °C, 110 rpm, 80% humidity to reach $OD_{600}$ =10-20. Corresponding volumes of the pre-cultures were added to 450 mL of sterile BSM to a final $OD_{600}$ = 1.0 in the DASGIP reactors.

Using these culture conditions the Batch phase was started with the following settings: Minimum stirring of 500 rpm and minimum of 30 % dissolved oxygen (DO) in the fermentation medium. The stirring was coupled to the oxygen level and set to increase, in case the DO went below 30 %. The Batch phase was run until all nutrients in the starting medium were used by the cells, indicated by the "starvation peak" in the oxygen levels.

During the FedBatch phase, the cultures were controlledly grown to high cell densities by setting a glycerol feed medium (~ 65 % glycerol) flow of 5 mL/h per reactor.

Approximately 6 h after the start of the FedBatch phase, the Induction phase was started. A methanol feed medium flow of 3 mL/h was set and the Induction phase was run for 90 h.

1.13 <u>Genomic DNA isolation and determination of gDNA quality and quantity</u>

*Armoracia rusticana*

Genomic DNA was isolated from leaves of *A. rusticana* with the nexttec™ Genomic DNA Isolation Kit for Plants maxi (nexttec Biotechnologie GmbH, Leverkusen, Germany) according to the manufacturer's recommendations.

The quantity of the isolated gDNA was assessed by estimation from an agarose gel. The quality was assessed by verification of the absorption ratios A260:A280 > 1.8, A260:A230 = 2.1 and A260:A270 = 1.2, measured on the NanoDrop 2000c Spectrophotometer.

*Pichia pastoris*

In order to isolate gDNA from *P. pastoris* for qPCR the following protocol was applied (Hoffman et al. ((1987) Gene 57, 267-272)):

> A single colony of the desired strain was inoculated to 25 mL sterile YPD and incubated over night at 28 °C, 110 rpm. The grown culture ($OD_{600}$ < 10) was centrifuged for 5 min at 500 x g, the pellet was resuspended in 500 μL sterile $dH_2O$ and transferred to a sterile micro-centrifuge tube. The cells were spinned down and the pellet was resuspended in 200 μL yeast lysis buffer. 200 μL phenol:chloroform:isoamyl alcohol (25:24:1) plus approximately 300 mg acid-washed glass beads were added and the suspension was vortexed for 5 min. After addition of 200 μL TE buffer, the suspension was centrifuged for 5 min, 16.100 x g and the aqueous phase was transferred to a new microcentrifuge tube. The DNA was precipitated by addition of 1 mL ice cold absolute EtOH and incubation for 10 min at -20 °C, spinned down and air dried. The DNA pellet was resuspended in 400 μL TE plus 5 μL 2 mg/mL RNaseA and incubated for > 4 h at 37 °C. 10 μL 4 M ammonium acetate and 1 mL absolute EtOH were added and the DNA was precipitated as described above. The DNA pellet was washed with 70 % EtOH, spinned down and dissolved in a final volume of 50 μL TE buffer.

In order to isolate gDNA from *P. pastoris* for colony PCR, the Bust n' Grab protocol described by Harju et al. ((2004) BMC biotechnology 4:8) was applied with the following modifications:

> Instead of pelleted cells of an overnight culture, a *P. pastoris* colony was dissolved in 200 μL lysis buffer. In order to lyse the cells, liquid nitrogen was used instead of dry ice-ethanol. After the isolation steps, RNase treatment was done with 0.125 μL 2 μg/μL RNaseA for 1 h at 37 °C.

The assessment of quality and the quantity of the isolated gDNA from P. *pastoris* was done applying the same criteria as for the isolated gDNA from A. *rusticana.*

1.14 HRP activity assay and evaluation of HRP purity

**ABTS assay**

This standard assay for analyzing the enzymatic activity of HRP was done similar to the ABTS assay described by Morawski et al. (2000):

15 μL of supernatant from either micro-scale cultivation, fermenter culture or purified enzyme were mixed with 140 μL ABTS assay solution (1 mL 20x ABTS stock, 19 mL 50 mM NaOAc, pH 4.5, 1.75 pL 30% (v/v) $H_2O_2$) and the increase in absorption at 405 nm (ε of oxidized ABTS is 34,700 $M^{-1}$ $cm^{-1}$) was followed with the Spectramax Plus 384. The assay conditions were varied in order to characterize A2A. These reactions were performed as triplicates. In case of obvious outliers, one out of three measured points was discarded.

**Guaiacol assay**

The guaiacol assay was done similar to the guaiacol assay described by Morawski et al. (2000):

15 μL of HRP solution were mixed with 140 μL guaiacol assay solution (11.1 μL guaiacol, 20 mL 20 mM phosphate buffer, pH 7.0, 0.605 μL 30% (v/v) $H_2O_2$) and the increase in absorption at 470 nm (ε of oxidized guaiacol is 26,000 $M^{-1}$ $cm^{-1}$) was followed with the Spectramax Plus 384. The characterization of the enzymatic performance of HRP A2A with guaiacol as substrate was done with various deviations from the assay conditions.

**Assessment of HRP purity by verifying the Rz value**

As a criterium of purity the Rz value was measured as described by Morawski et al. (2000): The absorption at 404 nm and at 280 nm was measured on the NanoDrop 2000c Spectrophotometer in order to calculate the Rz value, being the ratio A404:A280.

1.15 Protein quantitation

In order to quantify dissolved protein, the microplate procedure protocol of the Pierce® BCA Protein Assay Kit (Thermo Scientific Fisher, Waltham, MA, USA) was applied. 250.0, 125.0, 50.0, 25.0, 5.0, 2.5 and 1.0 ng/μL BSA dilutions and 250.0, 120.0, 62.5, 31.25, 15.625 and 7.8125 ng/μL HRP VI-A dilutions for the generation of standard curves were used. The suggested incubation time was prolonged to 1 h at 37 °C prior to measure the absorption at 562 nm.

1.16 Protein purification

**Buffer change and concentration of protein solution**

The Sartorius Vivaflow 50 system (30,000 MWCO cut-off) was used for sample concentration and buffer change. In order to concentrate samples of volumes smaller than 50 mL, the Sartorius Vivaspin 20 system (3,000 MWCO cut-off) was used.

**Hydrophobic interaction chromatography**

For hydrophobic interaction chromatography, the buffer HIC-A (20 mM Tris-HCl, pH 7.0, 1 M $(NH_4)_2SO_4$) was used as starting buffer. Buffer HIC-B (20 mM Tris-HCl, pH 7.0) was used for elution.

Cleaning and equilibration of Phenyl Sepharose® 6 Fast Flow (packed in a XK26/20 column) was done as recommended by the manufacturer. Elution was done by replacing buffer HIC-A with buffer HIC-B in a linear gradient from 0 % HIC-B to 100% HIC-B over 23x column volumes (CV) with a flowrate of 15 mL/min (~ 169.5 cm/h). Changes in absorption at 280 nm and 404 nm, conductivity and the concentration of buffer HIC-B on the column were followed with the UNICORN™ software. The collected fractions were checked for HRP activity with the ABTS assay.

**Anion exchange chromatography**

For determination of the pH of the QFF-buffer A at which HRP A2A binds completely to the Q Sepharose® Fast Flow material, a test tube experiment was performed similarly to the protocol described by GE Healthcare (http-//www.gelife-sciences.com/aptrix/upp00919.nsf/Content/TT%3ATest+tube+metho %28150431776-C520%29?

OpenDocument&hometitle=tech_support_service (24.02.2011).):

About 200 µL Q Sepharose® Fast Flow (QFF) material were transferred to 10 microcentrifuge tubes and spinned down. The supernatant was discarded and the sedimented beads were washed ten times for equilibration. The following 10 different buffers were used in the 10 tubes: Tris-HCl, pH 9.5, Tris-HCl, pH 9.0, Tris-HCl, pH 8.5, Tris-HCl, pH 8.0, Tris-HCl, pH 7.5, citrate-phosphate, pH 7.6, citrate-phosphate, pH 7.0, citrate-phosphate, pH 6.4, citrate-phosphate, pH 5.8, citrate-phosphate, pH 5.0. After equilibration of the beads with 285 µL of the corresponding buffers, 15 µL of HRP solution were added to each microcentrifuge tube. This mixture was vortexed and incubated for 1 min at 4 °C. The beads were spinned down and 15 µL of the supernatant were checked for HRP activity by applying the standard ABTS assay at pH 4.5.

In case no HRP activity could be detected in the supernatant, a 1 M NaCl solution of the respective buffer was added and mixed with the HRP-binding beads. After another 1 min incubation at 4 °C, the supernatant was checked again for restored HRP activity due to HRP elution from the beads.

For anion exchange chromatography, the buffer QFF-A (50 mM Tris-HCl, pH 9.5) was used as starting buffer and buffer QFF-B (50 mM Tris-HCl, pH 9.5, 1 M NaCl) was used for elution.

20 mL Q Sepharose® Fast Flow material were packed to a XK26/20 column, washed and equilibrated with buffer QFF-A in accordance with the manufacturer's recommendations. Elution was done by changing the ratio of QFF-A:QFF-B in a step-gradient.

Changes in absorption at 280 nm and 404 nm, conductivity and the concentration of buffer QFF-B on the column were followed with the UNICORN™ software. Collected fractions were checked for HRP activity via the ABTS assay and the fractions with highest HRP activity were checked for their Rz values.

**Size exclusion chromatography**

For size exclusion chromatography, the Superdex buffer (3.5 mM citrate, 32.9 mM $Na_2HPO_4$, pH 7.0) was used.

The HiLoad™ 16/60 Superdex™ 200 prep grade column, prepacked with 120 mL matrix material, was washed and equilibrated as recommended by the manufacturer. The sample was concentrated using the Vivaspin 20 system and loaded onto the column with a flowrate of 0.3 mL/min (~ 9.0 cm/h). The same flowrate was applied for the size exclusion run over 2x CV. Changes in absorption at 280 nm and 404 nm and conductivity were followed with the UNICORN™ software. The fractions were checked for HRP activity with the ABTS assay and the fractions with highest HRP activity were assessed for their Rz values.

**Fractional precipitation with ammonium sulfate**

100 µL sample were incubated in $(NH_4)_2SO_4$ solutions of stepwise increasing molarity at 4 °C for 45 min per step. After incubation, the samples were spinned down at 16,100 x g for 15 min. The supernatant was used for the next precipitation step, the pellet was resuspended in buffer QFF-A. The buffer conditions of the various steps are shown in table 3:

Table 3

| molarity mol/L | saturation at 4 °C % |
|---|---|
| 0.00 | 0 |
| 0.39 | 10 |
| 0.79 | 20 |
| 1.18 | 30 |
| 1.57 | 40 |
| 1.96 | 50 |
| 2.36 | 60 |
| 2.75 | 70 |
| 3.14 | 80 |
| 3.54 | 90 |

The fractions, being the resuspended pellets and the supernatant of the last precipitation step, were checked for HRP activity by applying the ABTS assay and their Rz values were determined.

### Affinity chromatography

For affinity chromatographic purification via a fused StrepTagII, the Strep-tag® purification protocol by IBA GmbH was applied according to the manufacturer's recommendations.

## Example 1. HRP sequences from next-generation sequencing

### 454 sequencing of the *Armoracia rusticana* transcriptome

In order to identify and verify nucleotide sequences of horseradish peroxidase isoenzymes, a search for GenBank- or UniProt-published HRP sequences in the transcriptome was conducted.

High quality total RNA has been isolated from horseradish, normalized in terms of RNA abundance and length, and sequenced by LGC Genomics GmbH (Berlin, Germany) by using the Roche Applied Science GenomeSequencer FLX Titanium technology. A *de novo* assembly using the Newbler Assembler 2.01 was done by LGC Genomics that provided an alignment of approximately 590,000 reads to a total of ~ 27,000 contigs with ~ 13,000 being longer than 500 bp.

The BLAST-NCBI (default settings) was used implemented in the ClusterControl system at the Institute of Genomics and Bioinformatics at the Graz University of Technology to check the transcriptome contigs for HRP sequences published either at GenBank or UniProt. Furthermore, also the published HRP sequences were BLASTed against the transcriptome raw read sequences, the hits isolated and manually assembled using ClustalW2.

In order to identify new, so far unknown HRP isoenzyme sequences, the total contig number of approximately 27,000 contigs was minimized by performing a tBLASTn search with the known HRP protein sequences as well as 90 % identity-clustered Arabidopsis thaliana peroxidase sequences against all contigs. An e-value of $10^{-5}$ and the BLOSUM62 matrix were chosen in order to include many false positives. The longest open reading frame in each of the found contigs was translated and the encoded protein sequence was classified using the NCBI Conserved Domains Database (CDD). The contigs identified as secretory peroxidases were manually analyzed with the DNAstar/SeqMan software, presumedly misassembled reads were split to separate contigs, trimmed reads were extended if possible.

### 454 sequencing of the *Armoracia rusticana* genome

In addition to next-generation sequencing of the horseradish transcriptome the 454 sequencing of a shot-gun FLX Titanium library as well as a 3 kb paired-end library of the horseradish genome at the ZMF Molecular Biology Core Facility (Center for Medical Research, Graz, Austria) were ordered. Assuming the horseradish genome to be of similar size as the Arabidopsis thaliana genome (157 Mbp), which most probably underestimates the true genome size, a putative double coverage in average was expected.

The genomic DNA (gDNA) was isolated from the leaves of horseradish and sequenced by 454 sequencing.

The sequencing provided a total of approximately 286 Mb (linker / primer sequences excluded) in ~ 676,000 shotgun reads and ~ 464,000 paired-end reads. Assembling of the reads was approached with the Newbler assembler (standard settings, primer/linker trimming), as well as with MIRA (after Newbler extraction and trimming of the reads). Since both approaches featured just a small part of the total assumed genome size, the previously identified transcriptome contigs were sought directly in the genome sequencing reads via tBLASTn (e-value 10-5, matrix PAM30) of the protein sequences derived from the transcriptome. By displaying the matches of all nearly identical high-scoring segment pairs (> 10 residues) and by using the GeneWise tool (http://www.ebi.ac.uk/Tools/Wise2/ index.html, 01.03.2011) for intron finding and the Needleman-Wunsch algorithm (http://www.ebi.ac.uk/Tools/emboss/align/ index.html, 01.03.2011) for the alignments (gap open penalty 100.0, gap extension penalty 0.0005), exons with adjacent intron sequences were supposed to be found.

### Verification of HRP sequences with Sanger sequencing

In addition to the identification of published and new HRP isoenzymes from next-generation sequencing approaches, those sequences have been verified on genomic DNA level via Sanger sequencing, which allows the determination of full gene sequences (i.e. exonic and intronic sequences) at highly reliable quality.

Primer pairs that bind in the 3'- and 5'-untranslated regions (UTR) of those isoenzymes published at GenBank (i.e. C1A, C1 B, C1 C, C2, C3, N) were designed. In order to design primers for isoenyzmes published at UniProt, those sequences that were successfully deduced from the transcriptome (i.e. E5, A2) could be used. Primers for two transcriptome contigs encoding new HRPs (i.e. 22684, 01805) that were found in the BLAST search with the published HRP sequences were designed.

For some isoenzymes, there was too little distinctive sequence information available in the UTRs to design specific primers. A Genome Walking approach in order to verify the C-terminal encoding regions of E5 and contig 22684 as well as the N-terminal encoding regions of C1 C and N was chosen. The respective gene-specific primers were: B2CtermSpecific1 a and B2CtermSpecific2, ESCtermSpecific1 and ESCtermSpecific2, newC1 CNtermSpecific1 a and newC1 CNtermSpecific2, newNNtermSpecific1 a and newNNtermSpecific2. The primers labelled "B2" correspond to 22684.

Further primers were designed to bind within the HRP genes, in order to ensure an overall sequence coverage of at least two times. These primers were used in PCRs and / or for Sanger sequencing. In case a certain region of an isoenzyme could not be amplified unambiguously or did not yield explicit sequencing data due to unspecific primer bindings, the PCR product was ligated to the pJET1.2blunt vector, transformed to *Escherichia coli* and the clonally amplified plasmids from approximately eight different clones were isolated and sent to Sanger sequencing separately, in order to guarantee distinct sequence information of the inquired isoenzyme. Otherwise, the PCR product was sent directly to Sanger sequencing. In order to verify the full gene sequences of the new additional isoenzymes that were found in the CDD-associated approach (i.e. contigs 23190 (SEQ ID NO:15), 04663 (SEQ ID NO:16), 06351 (SEQ ID NO:17), 06117 (SEQ ID NO:18), 04791 (SEQ ID NO:10), 17517 (SEQ ID NO:20), 01350 (SEQ ID NO:21), 02021 (SEQ ID NO:27), 05508 (SEQ ID NO:23), 08562 (SEQ ID NO:24), 22489 (SEQ ID NO:26)), gene-flanking primers were used for PCR amplification. In case the primer pairs yielded distinctive PCR products, they were ligated to pJET1.2blunt vector, amplified in *E. coli* and eight plasmid isolations per isoenzyme were sent to Sanger sequencing.

The sequence reads were aligned to a reference sequence if available (i.e. published sequences and transcriptome sequences) or assembled *de novo* using SeqMan. Intron finding was achieved by applying the PlantGDB GeneSeqer web tool.

**Analysis of verified HRP sequences**

The whole encoded protein sequences of the Sanger-verified isoenzymes were aligned to each other using ClustalW2 in order to get a picture of how closely the isoenzymes are related to each other. Moreover, SignalP 3.0 was used for the prediction of signal peptide cleavage sites and WebLogo 3 for a visual representation of the identified cleavage sites. Furthermore, a theoretical isoelectric point (IEP) was calculated with the Expasy Compute pl/Mw tool of all verified HRP isoenzymes using the whole protein sequences, in order to allow an assignment of them to an isoenzyme group.

**454 sequencing of the *Armoracia rusticana* transcriptome**

By BLASTing the published HRP sequences separately against the transcriptome contigs, the contigs 15901 (SEQ ID NO:28), 25148 (SEQ ID NO:29), 04627 (SEQ ID NO:30) and 00938 (SEQ ID NO:32) were successfully identified to which the HRP isoenzymes C1 B, C1C, C2 and E5 were assigned, respectively. Further, two contigs, 01805 (SEQ ID NO:31) and 22684 (SEQ ID NO:33), encoding yet unpublished HRP isoenzymes were found. Contig 01805 showed 84 % identity to the published coding sequence of C1A, contig 22684 was 90 % identical with the published coding sequence of HRP C3. The identity percentage of the published coding sequences of C1A and C1 B, C1A and C1C, C1 B and C1C was 89 %, 90 %, 94%, respectively.

BLASTing published HRP sequences against all transcriptome sequencing raw reads allowed the identification of reads that could be manually assembled to match the published HRP isoenzyme A2.

The alignments of the identified translated isoenzyme sequences against the amino acid sequences from UniProt and - if available - the translated sequences from GenBank are shown in figure 25. The published and transcriptome-derived sequences of C1 B and C2 were identical. Since the A2 sequence at UniProt lacks the 31 aa N-terminal signal peptide sequence, the positions of the affected amino acids in the table differ by the number 31. All differences are described in table 4.

Table 4

| HRP | translated transcriptome sequence | UniProt / translated GenBank sequence |
|---|---|---|
| C1B | = | = |
| C1C | Arg40 | Ser40 |
| C2 | = | = |

(continued)

| HRP | translated transcriptome sequence | | UniProt / translated GenBank sequence | |
|---|---|---|---|---|
| E5 | extra N-terminal signal peptide and C-terminal propeptide sequence | | only main chain | |
| A2 | extra N-terminal signal peptide | | only main chain | |
| | Asn78 | Thr284 | Asp47 | Leu253 |
| | Gly221 | Asn334 | Asn190 | Asp303 |
| | Asn222 | | Gly191 | |

In the search for new HRP sequences, 48 contigs could be identified after the first BLAST search step. Checking for a CDD classification as secretory peroxidase of the protein sequences encoded in these 48 contigs further reduced the contig number to 16. Manual reviewing of these contigs facilitated the identification of a total number of 19 contigs that featured a full secretory peroxidase domain (i.e. contig23190 (SEQ ID NO:15), contig04663 (SEQ ID NO:16), contig06351 (SEQ ID NO:17), contig06117 (SEQ ID NO:18), contig03523 (SEQ ID NO:19), contig17517 (SEQ ID NO: 20), contig00938 (SEQ ID NO:32), contig01805 (SEQ ID NO:31), contig04627 (SEQ ID NO:30), contig15901 (SEQ ID NO:28), contig22684 (SEQ ID NO:33), contig01350 (SEQ ID NO:21), contig01350_ALTERNATIVE (SEQ ID NO:22), contig02021 (SEQ ID NO:27), contig25148 (SEQ ID NO:29), contig05508 (SEQ ID NO:23), contig08562 (SEQ ID NO: 24), contig08562_ALTERNATIVE (SEQ ID NO-25),contig22489 (SEQ ID NO:26)). Among these were again the contigs encoding the isoenzymes E5, C2, C1 B and C1 C and the two contigs 01805 and 22684 which have already been found before. Inconsistencies of assembled reads in one contig were mainly considered to be due to allelic variations.

**454 sequencing of the *Armoracia rusticana* genome**

The assemblies of the genome sequencing reads featured ~ 64,000 contigs with 23 Mb from the Newbler approach, and ~ 23,000 contigs with 12 Mb from the MIRA approach. The following results were achieved by checking the genome sequencing reads directly for HRP isoenzymes: One full exonic sequence and two partial intronic sequences corresponding to the C1 B-encoding transcriptome contig 15901 were found in 12 reads of the genome Newbler contig 38504. One full exon and 2 partial intronic sequences belonging to the transcriptome contig 08562 could be identified on a single genome sequencing read. Three genome reads represented the sequences of two full exons, one full and one partial intron associated to the contig 08562_AL TERNATIVE (SEQ ID NO:25). One genome sequencing read could be identified to feature a partial exonic and a partial intronic sequence belonging to the transcriptome contig 01351 (SEQ ID NO:34) which has been characterized by CDD criteria to contain a C-terminally truncated part of a conserved domain of the plant peroxidase-like superfamily.

**Verification of HRP sequences with Sanger sequencing**

A total of 15 HRP isoenzyme sequences on genomic DNA level via Sanger sequencing with double coverage at the minimum were successfully verified. The verified isoenzymes and the deviations of the Sanger sequences from the transcriptome sequences, as well as from GenBank and UniProt sequences, and the influence of these deviations on the amino acid sequence are shown in table 5:

Table 5

| HRP | Sangersequence | | transcriptome sequence | | GenBank sequence | | UniProt sequence |
|---|---|---|---|---|---|---|---|
| | nt | aa | nt | aa | nt | aa | aa |
| C1A (SEQ ID NO:51) | TA 109-110 | Tyr | - | - | AT 109-110 | Ile | Tyr |
| | C1159 | intron | - | - | G1159 | intron | - |
| C1B (SEQ ID NO:1) | T/C253 | intron | none | none | T253 | intron | - |
| | T/C859 | intron | none | none | C859 | intron | - |

(continued)

| HRP | Sangersequence | | transcriptome sequence | | GenBank sequence | | UniProt sequence |
|---|---|---|---|---|---|---|---|
| | nt | aa | nt | aa | nt | aa | aa |
| C1C (SEQ ID NO:2) | C118 | Arg | C118 | Arg | A118 | Ser | Ser |
| | A/T1275 | intron | - | - | - | - | - |
| | A/G1828 | Thr/Ala | G1828 | Ala | G1828 | Ala | Ala |
| | C/G1861 | Gln/Glu | G1828 | Glu | G1828 | Glu | Glu |
| C2 (SEQ ID NO:3) | A/C636 | intron | none | none | A636 | intron | - |
| | C1315 | intron | none | none | * | intron | - |
| | A1333 | intron | none | none | * | intron | - |
| | C1250 | intron | none | none | T1250 | intron | - |
| A2A (SEQ ID NO:4) | 1-93 | signal | 1-93 | signal | - | - | - |
| | AAT 231-234 | Asn | AAT 231-234 | Asn | - | - | Asp |
| | GGA 996-998 | Gly | GGA 661-663 | Gly | - | - | Asn |
| | AAT 999-1001 | Asn | AAT 664-666 | Asn | - | - | Gly |
| | ACG 1185-1187 | Thr | ACG 850-852 | Thr | - | - | Leu |
| | G/A1203 | Ala/Thr | A1203 | Ala | - | - | Ala |
| | AAT 1335-1337 | Asn | AAT 999-1002 | Asn | - | - | Asp |
| E5 (SEQ ID NO:5) | T419 | Leu | C419 | Leu | - | - | Leu |
| | C422 | Asp | T422 | Asp | - | - | Asp |
| 01805 (SEQ ID NO:6) | none | none | none | none | - | - | - |
| 22684 (SEQ ID NO:7) | G1611 | Arg | A1611 | Lys | - | - | - |
| | TGA 1627-1629 | Asp | CGG 1627-1629 | Gly | - | - | - |
| 01350 (SEQ ID NO:8) | none | none | none | none | - | - | - |
| 02021 (SEQ ID NO:9) | none | none | none | none | - | - | - |
| 04791 (SEQ ID NO:10) | none | none | none | none | - | - | - |
| 06117 (SEQ ID NO:11) | none | none | none | none | - | - | - |
| 17517 (SEQ ID NO:12) | none | none | none | none | - | - | - |

(continued)

| HRP | Sangersequence | | transcriptome sequence | | GenBank sequence | | UniProt sequence |
|---|---|---|---|---|---|---|---|
| | nt | aa | nt | aa | nt | aa | aa |
| 08562.1 (SEQ ID NO:13) | see alignment below | | | | - | - | - |
| 08562.4 (SEQ ID NO:14) | | | | | - | - | - |

This direct comparison of the data from Sanger sequencing using a gDNA template and 454 sequencing from a transcriptome cDNA library reveals that the HRP isoenzyme C1A, even though present in the genome, could not be found in the transcriptome. This finding was highly unexpected, since C1A has been described in literature as the most abundant isoenzyme in horseradish. It was especially surprising, that C1A was not expressed in the tissue used for the generation of the cDNA library, since tissue from both, leaves and roots was used thereto. However it might be possible that the presumedly highly abundant C1A transcript was completely degraded during the cDNA normalization via kamchatka crab duplex-specific nuclease (Zulidov et al., (2004) Nucleic acid research 32, e37). In order to prove this hypothesis, primers specific for C1A could be used for a PCR using the cDNA library as template for the verification of the presence or absence of C1A transcripts. Alternatively the used horseradish variety might show different expression of isoenzymes than the plant material studied so far or the expression of C1 a is dependent on the season or specific environmental conditions such as temperature or day length in the natural environment.

The double peak signals A/G1828 and C/G1861 (see table 5) in the alignment of the Sanger sequencing reads matched to isoenzyme C1 C are shown in figure 1. All allelic sequences seem to have been assembled as a mixture in the transcriptome contig identified as C1 C. Underlining this assumption, both putatively allelic sequences could be found in the transcriptome raw reads as separate reads.

The two Sanger-verified sequences 08562.1 and 08562.4 are shown in an alignment with the corresponding transcriptome contigs. All observed differences and the homopolymeric region from position 98-104 of contig 08562 and contig 08562_ALTERNATIVE are marked in grey. Non-coding sequence is written in grey letters (see figure 27).

Identified differences between 08562.1 and 08562.4 on amino acid level were Lys88 versus Arg88 and Ser279 versus Asn279 in 08562.1 versus 08562.4, respectively.

The alignment of the presumably allelic sequences of 08562.1 and 08562.4 from Sanger sequencing with the transcriptome contigs 08562 and 08562_ALTERNATIVE illustrates important issues in the assembly of next-generation sequencing reads of isoenzyme sequences. Moreover, it emphasizes the clear need for manual sequence verification in order to clarify assembly mistakes and homopolymeric regions, which constitute a known problem that is inherent to the 454 sequencing method.

The transcriptome reads from the two verified isoenzymes 08562.1 and 08562.4 have been found to be mixed and misassembled. Hence, more restrictive assembly settings might be helpful in order to distinguish between these two isoenzymes and assemble them to the two correct contigs. However, by applying more restrictive settings, the tolerance for sequencing mistakes decreases in an inversely proportional way which might lead to the identification of further "isoenzymes" that actually originate from errors in the sequencing procedure. Separate PCR amplifications of 08562.1 and 08562.4 from the cDNA library and subsequent Sanger sequencing could be done to prove the assumed misassembly.

Furthermore, the T-oligomeric region at position 98-104 of the two transcriptome contigs, which at first has been manually "corrected" with an insertion X in order to account for a frameshift mutation, has been shown to actually consist of a pentamer instead of the assumed heptamer.

The existence of allels might be an explanation for the observed variations in the sequences of (putatively) one isoenzyme gene. Even though there is no information available on the actual degree of ploidy of *A. rusticana,* this is a very likely possibility, especially taking the obtained sequencing data of C1 C or 08562 into consideration. Bearing this in mind, the high number of different isoenzymes observed via isoelectric focusing could be explained by the detection of two (or more) expressed allels of "one" isoenzyme gene. This illustrates an issue in the nomenclature and classification of isoenzymes: In general, all HRP molecules present in an extract from horseradish have been separated by isoelectric focusing and counted as individual isoenzymes. Hence, the verified sequence of A2A for example, might be both, an allel of the published A2 gene, but just as well one of the also observed isoenzymes A1 or A3. And again, A1 and A2 might be allels of the same gene or actually two separate genes on one chromosome. This question remains unanswered due to the low quality of the obtained genome sequence.

Since gene duplication is a proposed mechanism for the evolution of HRP isoenzymes, highly similar HRP gene sequences can also be explained hereby and do not necessarily have to be allels.

A further explanation for the observed differences in all verified sequences might be a variance in the HRP genes among locally separated *A. rusticana* populations.

In summary, 15 HRP isoenzymes on genomic DNA level were successfully identified and verified, using the data from the 454 transcriptome sequencing, Genbank and UniProt. Moreover, at least nine of the 15 HRPs are new isoenzymes that have not been published yet.

## Analysis of verified HRP sequences

The data provided from the multiple sequence alignment of the verified HRP amino acid sequences could be visualized in a phylogenetic tree (see figure 2).

The published HRP isoenyzmes are closer related to each other than to the new isoenzymes. A2A seems to be the most distant isoenzyme of the published ones. Just 01805 and 22684 of the new isoenzymes are closely related to the published HRPs. The other new isoenzymes cluster to two groups: 02021, 17517 and 04791 form one group, 01350, 06117, 08562.1 and 08562.4 form the second group.

By aligning a translation of the (presumed) 5'-UTR (marked in grey) of the Sanger-verified C1 C plus its N-terminus to the protein sequence of the N-terminus of C1 B, a strikingly high similarity could be observed (see figure 28).

This is most probably not part of the 5'-UTR of C1C, but rather an N-terminal signal peptide, whose full amino acid sequence has not been published at UniProt. Considering the high similarity to the signal peptide of C1 B, it can be assumed that C1 C and C1 B target to the same destination.

Additionally, MHFSTSSSSLSTWTTLITLGCLMLHSFKSSA (SEQ ID NO:56) was identified as N-terminal signal peptide of 01805 with 51 % similarity to the newly found C1 C signal peptide, MGFSPSFSSSSIGVLILGCLLLQASNSNA (SEQ ID NO:57) as signal peptide of 22684 with 75 % similarity to the annotated signal peptide of the C3 isoenzyme at UniProt, and MVVSPFFSCSAMGALILGCLLLQASNA (SEQ ID NO:58) as signal peptide of E5 with 77 % similarity to the C3 signal peptide.

Moreover, the C-terminal peptide LLHDMVEVVDFVSSM (SEQ ID NO:59) of C1A was found, which is annotated as propeptide at UniProt, not only present in C1A, but also in C1 B and C1 C with 93 % similarity and in 01805 with 86 % similarity. It is thus very likely, that these isoenzymes are processed to mature enzymes in a similar way as C1A.

In addition to these similarity-based findings of signal peptides, the predicted signal peptides and the corresponding cleavage sites from the SignalP 3.0 server are shown in table 6 (the predicted cleavage sites of the verified isoenzymes. 1: Most likely cleavage site position. 2: Cleavage site sequence from neural networks prediction. 3: Cleavage site probability from hidden Markov models prediction.):

Table 6

| isoenzyme | cleavage site | | |
|---|---|---|---|
| | pos[1] | seq[2] | prob[3] |
| C1A | 30/31 | SDA/QL | 0.849 |
| C1B | 28/29 | SDA/QL | 0.897 |
| C1C | 29/30 | SNA/QL | 0.903 |
| C2 | 24/25 | SHA/QL | 0.891 |
| A2A | 31/32 | SSA/QL | 0.738 |
| E5 | 27/28 | SNA/QL | 0.920 |
| 01805 | 31/32 | SSA/QL | 0.647 |
| 22684 | 29/30 | SNA/KL | 0.613 |
| 01350 | 20/21 | VQG/NY | 0.550 |
| 02021 | 29/30 | SEA/QL | 0.810 |
| 04791 | 22/23 | I ES-RL | 0.877 |
| 06117 | 22/23 | CIC/DD | 0.805 |
| 17517 | 23/24 | VTA/RR | 0.919 |

(continued)

| isoenzyme | cleavage site | | |
|---|---|---|---|
| | pos1 | seq2 | prob3 |
| 08562.1 | 22/23 | CLC/DK | 0.989 |
| 08562.2 | 22/23 | CLC/DK | 0.989 |

The following eleven amino acids surrounding the predicted signal peptide cleavage sites were used to create a sequence logo (figure 3).

The cleavage sites of C1A, C1 B, C1C, C2, A2A, E5, 01805, 22684 and 02021 were found to be highly alike and were thus used for the creation of another sequence logo (figure 3b) which allows the fast identification of highly conserved amino acids in this signal peptide cleavage site, e.g. the alanine (position 4 in fig. 3b) before the glutamine after which the signal peptide is cleaved.

By examining the gene structure it was observed that all published HRP isoenzyme sequences showed a structure of four exons and three introns. Most of the sequences presented here match this structure, however isoenzyme 04791 does not have any introns and 17517 has only two introns. Both genes are within a cluster of three closely related HRP genes (see fig. 2).

The calculation of the theoretical IEP showed that most of the newly identified isoenzymes have a basic theoretical IEP. 01350, 04791, 08562.1 and 08562.4 may be assigned to the basic HRP isoenzyme groups D and E. 06117, 22684 and 01805 might be further members of the isoenzyme group C, or belong to group B (i.e. neutral, neutral-basic). HRP A2A showed the lowest IEP so far with 4.82, whereas the highest IEP was found for HRP 17517 with 9.57.

The calculated IEPs of the verified HRP isoenzymes are shown in table 7:

Table 7

| isoenzyme | IEP | isoenzyme | IEP |
|---|---|---|---|
| A2A | 4.82 | C2 | 8.70 |
| C1A | 5.67 | E5 | 8.72 |
| 06117 | 5.69 | 04791 | 8.86 |
| C1B | 5.74 | 08562.1 | 8.95 |
| 22684 | 6.32 | 08562.4 | 8.96 |
| 01805 | 6.37 | 02021 | 9.57 |
| C1C | 6.94 | 17517 | 9.57 |
| 01350 | 8.66 | | |

### Example 2. Expression of HRP A2A in *Pichia pastoris*

#### 2.1 Experimental

##### 2.1.1 Optimizing the HRP A2A gene for heterologous expression

HRP isoenzyme A2A was expressed in *Pichia pastoris* due to its acidic isoelectric point.

In order to maximize the yield of expressed HRP the A2A gene was optimized, using its protein sequence derived form the Sanger-verified nucleotide sequence. Upstream the mature A2A sequence, an EcoRI restriction site was added, the *P. pastoris* Kozak sequence and the $\alpha$-factor signal sequence to facilitate secretion. For later purification, the StrepTagII sequence was fused via a Ser-Ala linker to the C-terminus of the mature A2A, followed by a Stop codon and a NotI resctriction site.

The Gene Designer software from DNA2.0 Inc., Menlo Park, CA, USA was used. The codon usage table designed for high level expression during methanol induction in *Pichia pastoris* published by Abad et al. ((2010) Microbial cell factories 9, 24) was applied. Further, common restriction sites were excluded and pentameric or higher A/T combinations in order to allow unhindered cloning and to avoid destabilizing repeats, respectively. Further it was assured a balanced GC content (i.e. min. 30%, max. 70%) over the whole sequence, as well as a free energy higher than - 15 kcal/mol in RNA secondary structure predictions for the avoidance of any impairment with the ribosomes' processivity.

The GC content was determined with the online EMBOSS 6.3.1: freak tool (averaging window: 30 bp, stepping value: 1), RNA secondary structure prediction was done with the GeneBee web tool.

The gene was synthetized and cloned into a pUC vector.

### 2.1.2 Cloning of the HRP A2A gene

Primers (A2StrepRemovalfw, A2StrepRemovalrv) were designed for the removal of the StrepTagII sequence from A2Cstrep via PCR and to end up with an untagged A2A sequence. Furthermore, primers were designed to fuse the StrepTagII sequence via a Ser-Ala linker in between the $\alpha$-factor signal sequence and the N-terminus of the mature HRP A2A protein sequence and thus create A2ANstrep in an overlap PCR: The first PCR was done with the primers A2_Nterm_Strepfw1 plus A2StrepRemovalrv. The PCR product was cut from a preparative agarose gel, purified and used as template in a second PCR with the primers A2_Nterm_Strepfw2 plus A2StrepRemovalrv.

A2ACstrep and A2A were cloned to pPpT4_SmiI, A2ANstrep was cloned to pPpT4Alpha_SmiI via their EcoRI and NotI restriction sites. The three plasmids were transformed to E. coli Top 10F' for plasmid amplification and subsequently sent to Sanger sequencing for sequence verification, prior to their transformation to *P. pastoris* MutS for recombinant protein expression.

### 2.1.3 Expression of HRP A2A in *P. pastoris* and coexpression of PDI

The *P. pastoris* strains expressing A2ACstrep and A2ANstrep were screened for HRP activity via the ABTS assay subsequent to micro-scale cultivation in 96-DWP. The two best expressing A2ANstrep clones were picked for small scale cultivation in 2 L-shake flasks.

In order to screen for *P. pastoris* A2AMutS clones expressing suffiently high levels of functional HRP, micro-scale cultivations in 96-DWPs was done and the HRP activity of approximately 200 clones evaluated via the ABTS assay. Moreover, the PDI genes synPDI, synPDI N314H and PDI704 were transformed in the pPpKan_SmiI plasmid to the A2AMutS clone that yielded the highest HRP activity and also studied the influence of the coexpressed genes on HRP activity in 96-DWPs. The PD1704 gene was isolated from gDNA of *P. pastoris* CBS704 at the Research Centre Applied Biocatalysis, Graz, Austria. synPDI represents the gene for a synthetic PDI gene based on the sequence of a *P. pastoris* CBS7435 PDI. synPDI N314H is a mutant of the synPDI gene with a N314H spontaneous mutation and was identified at the Institute of Molecular Biotechnology, Graz, Austria. The aligned protein sequences of the mentioned PDIs are shown in figure 29.

In order to evaluate the mere influence of a second transformation event itself (e.g. molecular interactions between the first and the second plasmid or alterations in the host genome by the integration of the second plasmid), the empty pPpKan_SmiI plasmid was transformed. Approximately 200 clones were screened and a selection of active clones was picked for rescreening. Hereto, these candidate clones were streaked to single colonies and four clones per candidate were picked for a second micro-scale cultivation and subsequent ABTS assay.

Selection of transformants was performed by streaking the cells onto YPD agar plates containing either zeocin™ (from pPpT4_SmiI or pPpT4Alpha_SmiI), kanamycin (from pPpKan_SmiI) or both. The HRP C1A expressing *P. pastoris* strains 110G4, 110B5, 107D6 and 107A9 were co-cultivated as positive controls for HRP activity. Two wells per 96-DWP were not inoculated with any strain as sterile controls. A duplicate of the non-transformed starting strain *P. pastoris* MutS was also co-cultivated as negative control for HRP activity. In the rescreen of the coexpressing strains, the starting A2AMutS strain was co-evaluated in quadruplicate in order to be able to assess an increase in the HRP acitivity of the coexpression strains.

### 2.1.4 Cultivation of *P. pastoris* expressing HRP A2A in DASGIP bioreactors

In order to enable future transcriptome analyses of HRP expressing strains, four strains were chosen for cultivation in DASGIP bioreactors. A2AMutSF5 and A2AMutSF5 synPDI N314H were used as HRP expressing strains. MutS ZeoR and MutS synPDI N314H were used as reference strains. The latter was generated by transforming pPpKan_SmiI_synPDI N314H to *P. pastoris* MutS. The hereby resulting clones were verified for containing the synPDI N314H gene via colony PCR with the primers RT-synPDI-fw and AOXseq_rv.

Prior to the actual cultivation process, the copy numbers of A2A and synPDI N314H were determined in the respective strains via qPCR (see 1.7), in order to ensure comparable genetic conditions and thus comparability of the obtained HRP yields.

The cultivation procedure was performed as described in 1.12. Samples for the assessment of HRP activity and future RNA isolation were drawn at the end of the FedBatch phase (0 h) and four times during the Induction phase (4 h, 24 h, 70 h, 90 h). At every time point, approximately 1.4 mL of the culture were drawn from each reactor. The $OD_{600}$ was measured and aliquots corresponding to a number of $3 \times 10^8$ cells (under the assumption that $3 \times 10^8$ cells correlate

with 15 mL culture of $OD_{600}$ = 1) were transferred to three microcentrifuge tubes per reactor and time point. The samples were spinned down for 2 min, 12,000 x g, 4°C, the supernatant was discarded and the pellet was flash frozen in liquid nitrogen and stored at -80°C for eventual total RNA isolation. The remaining sample culture was spinned down for 5 min, 3,000 x g, 4 °C and the supernatant was frozen at -20°C for later assessment of HRP activity via the ABTS assay. All handling was done on ice.

After the full cultivation procedure the cultures expressing HRP were transferred to 500 mL PPCO centrifuge bottles and spinned down for 15 min at 3,000 x g, 4°C in the Beckman Coulter Avanti centrifuge J-20 XP with the Beckman Coulter JA-1 0 Rotor. The supernatant was stored at -20°C for upcoming analyses.

## 2.2 Results and discussion

### 2.2.1. Expression of the optimized HRP A2A gene

The sequence of A2ACstrep was successfully optimized for expression in *Pichia pastoris* during methanol induction. The predicted RNA secondary structure and the GC distribution over the optimized sequence are shown in figure 4 and figure 5, respectively. The optimized sequence of A2ACstrep and the applied codons are depicted in SEQ ID NO:60.

The synthetic A2ANstrep was successfully modified to the untagged A2A and the C-terminally tagged A2ACstrep and these three genes were successfully transformed to *P. pastoris.*

Coexpression of the PDI genes *PDI704,* syn*PDI* and *syn*PDI N314H yielded interesting results in the screening in 96-DWPs. Coexpressed *PDI*704 and syn*PDI* did not seem to affect the HRP activity significantly. However, coexpressed synPDI N314H increased the measured activity up to four fold, compared to the activity of the starting strain A2AMutSF5 (see figure 6).

Syn*PDI* N314H, being the most promising candidate for coexpression, was chosen for rescreening, the results are depicted in figure 7. The obtained HRP activities of A2AMutSF5 strains coexpressing syn*PDI* N314H were significantly increased from the starting strain and are comparable to the activities of the optimized HRP C1A expressing control strains.

The mechanism underlying the specific increase in HRP activity of the N314H mutant of syn*PDI* is not yet understood and remains to be elucidated. Classification of the PDI amino acid sequence applying the NCBI Conserved Domain Search web tool, showed position 314 to lie in a domain classified to the PDI b' family (redox inactive TRX-like domain b') which is a member of the thioredoxin-like superfamily. The b' domain is described as the primary substrate binding site and to be implicated in chaperone activity. Probably, the N314H mutation in this domain increased the recognition of HRP as a substrate and promoted its correct folding, resulting in elevated yields of functional HRP A2A.

By screening *P. pastoris* clones expressing A2ACstrep for HRP activity, it was noticed that the HRP activity was abolished by the C-terminally fused StrepTagII, which probably interfered with the enzyme's folding to its native conformation. Thus it was focused on the expression of A2ANstrep, which showed HRP activity comparable to the untagged A2A. The two clones with the highest activity were picked and A2ANstrep was successfully expressed in small scale cultivation. The hereby obtained A2ANstrep-containing supernatant could be used for an affinity purification approach.

### 2.2.2 Cultivation of *P. pastoris* expressing HRP A2A in DASGIP bioreactors

The generation of the strain MutS synPDI N314H was successfully verified via agarose gel electrophoresis of the colony PCR product. By applying qPCR the gene copy numbers of HRP A2A was determined as well as of PDI in the corresponding strains. The qPCR results are shown in table 8. The Arg/Zeo data represents the HRP copy number, Arg/Pdi and Arg/Kan both represent the PDI copy number and are equally suitable for this purpose. The average values were calculated from the two values obtained from absolute and relative copy number quantification. Normalized data accounts for fluctuations in the method and normalizes the raw data to the reference's value.

Table 8

| Arg/Zeo | | | | |
|---|---|---|---|---|
| **raw data** | | | | |
| sample | absolute | relative | average | stdv |
| A2AMutSF5 | 0,85 | 0,89 | **0,87** | 0,03 |
| A2AMutSF5 synPDI N314H | 0,89 | 0,91 | **0,90** | 0,02 |
| reference | 0,84 | 0,85 | **0,85** | 0,01 |

(continued)

| Arg/Zeo | | | | |
|---|---|---|---|---|
| **raw data** | | | | |
| sample | absolute | relative | average | stdv |
| | | | | |
| **normalized data** | | | | |
| sample | absolute | relative | average | stdv |
| A2AMutSF5 | 1,01 | 1,04 | **1,02** | 0,02 |
| A2AMutSF5 synPDI N314H | 1,06 | 1,07 | **1,06** | 0,01 |
| reference | 1,00 | 1,00 | **1,00** | 0,00 |
| | | | | |
| **Arg/Pdi** | | | | |
| **raw data** | | | | |
| sample | absolute | relative | average | stdv |
| A2AMutSF5 synPDI N314H | 0,59 | 0,59 | **0,59** | 0,00 |
| MutS synPDI N314H | 1,20 | 1,20 | **1,20** | 0,00 |
| reference | 0,96 | 0,96 | **0,96** | 0,00 |
| | | | | |
| **normalized data** | | | | |
| sample | absolute | relative | average | stdv |
| **A2AMutSF5** synPDI N314H | 0,62 | 0,61 | **0,61** | 0,00 |
| MutS synPDI N314H | 1,24 | 1,24 | **1,24** | 0,00 |
| reference | 1,00 | 1,00 | **1,00** | 0,00 |
| | | | | |
| **Arg/Kan** | | | | |
| **raw data** | | | | |
| sample | absolute | relative | average | stdv |
| A2AMutSF5 synPDI N314H | 0,62 | 0,65 | **0,64** | 0,02 |
| MutS synPDI N314H | 0,99 | 1,00 | **1,00** | 0,01 |
| reference | 1,02 | 1,04 | **1,03** | 0,01 |
| | | | | |
| **normalized data** | | | | |
| sample | absolute | relative | average | stdv |
| A2AMutSF5 synPDI N314H | 0,61 | 0,63 | **0,62** | 0,01 |
| MutS synPDI N314H | 0,97 | 0,97 | **0,97** | 0,00 |

(continued)

| normalized data | | | | |
|---|---|---|---|---|
| sample | absolute | relative | average | stdv |
| reference | 1,00 | 1,00 | **1,00** | 0,00 |

The analyzed strains were successfully used in the DASGIP cultivation, the measured HRP activities are shown in figure 8.

The coexpression of A2A plus synPDI N314H yielded the highest activities, yet not as strikingly higher than sole A2A, as in micro-scale cultivations.

### Example 3. Purification of HRP A2A

### 3.1 Experimental

#### 3.1.1 Affinity chromatography

Using the Vivaspin 20 system, the Strep-tagged HRP A2A sample from small scale cultivation supernatant was concentrated to ~ 1000 μL and mixed with 14 U avidin in order to bind interfering biotin from the cultivation medium. The protein solution was dialyzed over night at 4 °C against 1 L of the IGA GmbH buffer W (100 mM Tris-HCl, pH 8.0, 150 mM NaCl, no EDTA), using a dialysis tube with 8,000 - 10,000 MWCO cut-off. The same preparation was performed without the preceding avidin treatment in order to exclude the possibility of column blockage by the used avidin.

The dialyzed enzyme solution was concentrated with the Vivaspin 20 system to 500 μL - 1000 μL and loaded onto the Gravity flow Strep-Tactin® MacroPrep® column.

The collected fractions of the run were analyzed for HRP activity by applying the ABTS assay.

#### 3.1.2 Hydrophobic interaction chromatography

The supernatant of A2AMutSF5 was concentrated from the cultivation in the 5 L-BIOSTAT bioreactor to a volume of 50 mL and changed the buffer to HIC-A, subsequent to tangential flow filtration using the Centramate 500 S system with a corresponding filtration membrane cassette with a 30 kDa cut-off.

These 50 mL of HRP A2A in buffer HIC-Awere loaded onto the HIC column with a flowrate of 5 mL/min (~ 56.5 cm/h) and the purification run was performed as described in 1.16. During the run, fractions of 5.0 mL to 15 mL PP-tubes were collected.

#### 3.1.3 Anion exchange chromatography

Prior to the actual anion exchange chromatography run, the test tube experiment was performed as described in 1.16, in order to identify the pH at which HRP A2A binds to the QFF material.

For the anion exchange chromatography, all HIC fractions were pooled that showed HRP activity, changed the buffer to QFF-A and concentrated the enzyme solution to approximately 50 mL.

This volume was loaded onto the anion exchange column with a flowrate of 5 mL/min (~ 56.5 cm/h). Throughout the run, fractions of 1.2 mL were collected to 96-DWPs.

#### 3.1.4 Fractional precipitation with ammonium sulfate

The QFF fraction with the highest Rz value was picked for fractional precipitation with ammonium sulfate as described in 1.16.

#### 3.1.5 Size exclusion chromatography

All QFF-fractions were pooled that showed HRP activity, changed to Superdex buffer and concentrated the sample to a volume of approximately 800 μL. This volume was loaded onto the column with a flow of 0.3 mL/min (~ 9.0 cm/h). Fractions of 800 μL were collected to 96-DWPs throughout the whole run.

The fraction giving the highest Rz value was stored at 4 °C and evaluated its purity via SDS-PAGE.

### 3.2 Results and discussion

#### 3.2.1 Affinity chromatography

By applying the StrepTactin protocol to HRP A2ANstrep, no purification could be achieved. HRP activity was predominantly measured in the flowthrough fraction collected during sample loading and in the first washing fractions. The actual elution fractions did not exhibit any significant HRP activity any more (see figure 9). HRP A2ANstrep did not bind to the StrepTactin column and eluted in the first fractions. Already in the flowthrough fraction, collected during the loading of the sample, HRP activity was measured. In the subsequent two washing fractions the remaining HRP activity was detected.The sample without avidin treatment provided the same results.

#### 3.2.2 Hydrophobic interaction chromatography

By using the HIC approach, it was possible to separate a lot of undesirable proteins from HRP in the cultivation supernatant. The chromatogram and the measured HRP activities are depicted in figure 10.

The measured HRP activity exposed a double peak, which might represent monomeric and aggregated oligomeric HRP A2A species that featured slightly different binding behaviors.

No significant amounts of HRP were lost in this purification step. The obtained Rz value after HIC purification was < 0.2. The fractions from 300 mL - 425 mL were used for further purification. Hereto, they had to be pooled, concentrated and changed to the buffer of the following purification step.

#### 3.2.3 Anion exchange chromatography

The test tube experiment provided the following data: By incubating A2A at different pH with the QFF material, full HRP activity was verified in the supernatants at pH 5.0 - 8.5. However, decreased HRP activity in the supernatant at pH 9.0, and no activity in the supernatant at pH 9.5 could be measured, indicating binding of A2A to QFF. By washing the QFF material and the bound HRP A2A with the same buffer and additional 1 M NaCl, HRP was successfully eluted from the QFF material and thus the HRP activity in the supernatant restored. Incubation of A2A in all 10 buffers without QFF material and subsequent activity verification via ABTS assay was successful in all buffers and thus loss of HRP activity at a certain pH could be excluded (see figure 11).

By running the anion exchange chromatography at pH 9.5, HRP A2A was further purified and a max. Rz value of approximately 0.5 achieved (see figure 12).

Again, HRP A2A eluted in two peaks, pointing to two conformational species. For maximum recovery of HRP A2A, all fractions exposing HRP activity (~ 70 - 115 mL) were used for final purification via size exclusion chromatography. Moreover, the fraction at 100 mL run volume was partially used for fractional precipitation with ammonium sulfate.

#### 3.2.4 Fractional precipitation with ammonium sulfate

By performing fractional precipitation with ammonium sulfate of the purest anion exchange chromatography fraction HRP A2A was further purified to a Rz value of ~ 1.8, which is already comparable to a commercially available HRP preparation (Peroxidase from horseradish Type II/ Sigma-Aldrich Handels GmbH, Vienna, Austria; denoted Rz ≥ 1.8).

The ABTS activities of the obtained fractions and the corresponding Rz values are depicted in figure 13.

The fractional precipitation with ammonium sulfate constitutes a fast approach for the purification of HRP A2A to a medium level of purity.

#### 3.2.5 Size exclusion chromatography

Alternatively to the "quick-and-dirty" precipitation approach, size exclusion chromatography was performed that yielded a high level of purity of HRP A2A with Rz value ≥ 3.5, which is even higher than the denoted Rz ~ 3.0 of the purest commercially available HRP preparation from Sigma (Peroxidase from horseradish Type VI/Sigma-Aldrich Handels GmbH, Vienna, Austria).

HRP A2A eluted in the fractions from approximately 80 - 90 mL. The chromatogram of the size exclusion chromatography run and the measured HRP activities are shown in figure 14:

The fraction with the highest Rz value and the highest HRP activity (at 84 mL total run volume) was determined to have a protein concentration of 60 ng/μL (see 4.2.3). Considering the fraction volume of 800 μL, the total yield of pure HRP A2A from one 5 L-BIOSTAT reactor was 50 μg.

SDS-PAGE of this fraction confirmed the high level of purity and the successful purification of the recombinantly expressed HRP A2A (see figure 15).

**Example 4. Characterization of HRP A2A**

### 4.1 Experimental

#### 4.1.1 Posttranslational modifications of HRP A2A

In order to study the purified recombinant HRP A2A for posttranslational modifications, a mass spectrometric analysis at the ZMF Mass Spectrometry - Proteomics Core Facility was conducted. Further, the PredictProtein web tool was used on the mature HRP A2A amino acid sequence in order to compare the predicted results with those from the mass spectrometry.

#### 4.1.2 Isoelectric focusing of HRP A2A

The binding of HRP A2A to QFF at pH 9.5 happened at a much more basic pH as expected (considering the theoretical IEP of A2A at pH 4.82).

Thus, an isoelectric focusing (see chapter 1.6.3) of HRP A2A was performed and compared to the commercially available HRP preparations Type I, Type II, Type VI, Type VI-A, Type XII from Sigma-Aldrich Handels GmbH and a HRP preparation from Toyobo Co., Ltd.

#### 4.1.3 HRP A2A and ABTS

**pH optimum of HRP A2A plus ABTS**

The interaction of HRP A2A was verified with the HRP standard substrate ABTS. The influence of pH on the catalysis of ABTS was studied. The ABTS assay was performed with the purified HRP A2A in buffers of different pH:

The 50 mM NaOAc, pH 4.5 standard buffer was used (Morawski *et al.*(2000)), 20 mM citrate-NaOH buffers at pH 3.0, 3.5, 4.0, 4.5, 5.0, 6.0, 7.0 and 20 mM Tris-HCI buffers at pH 7.0, 8.0, 9.0, 10.0. By performing the assay at the same pH with different buffer systems (i.e. 50 mM NaOAc and 20 mM citrate-NaOH at pH 4.5; 20 mM citrate-NaOH and 20 mM Tris-HCI at pH 7.0), any influences of the buffer systems on the reaction was excluded, other than the pH. Changing to the corresponding pH was achieved by 1:200 dilution of the purified A2A in the respective buffer.

**Michaelis-Menten kinetics of HRP A2A plus ABTS**

Since the optimal pH of the reaction of HRP A2A with ABTS was identified. A buffer at this pH was used for determining the kinetic constants $V_{max}$ and $K_M$ for the HRP catalyzed conversion of ABTS. Herefore the assay was performed with varying concentrations of ABTS: 0.1, 0.33, 0.5, 0.75, 1.0, 1.5, 2.0 and 3.0 mM. The measured activities in mAU/min were used to calculate the corresponding ABTS units/mL (1 ABTS unit is defined as the amount of enzyme that converts 1 μmol ABTS per minute) via the equation depicted below.

$$ABTS\ units/\mathrm{mL} = \frac{\frac{mAU}{min} * f}{\varepsilon * \mathrm{d}} * \frac{V}{v}$$

**Equation for the calculation of ABTS units per milliliter.**

f = dilution factor = 200.

$\varepsilon_{ABTS}$ = absorption coefficient of ABTS = 34,700 M-1 cm-1.

d = path length = 0.42 cm.

V = total reaction volume = 155 μL.

v = enzyme solution volume = 15 μL.

In order to be able to measure the increase in the absorption at 404 nm in the ABTS reaction the purified A2A 1:200 was diluted. The dilution factor f is taken into consideration in all calculations. The path length d was verified with the corresponding PathCheck function of the SoftMax Pro 4.8 software.

The specific activity of HRP A2A for ABTS was seeked to be verified. Hereto the purified A2A was quantified via the Pierce BCA Protein Assay Kit as described in chapter 1.6.15. Using this data the specific activity of HRP A2A in ABTS units/mg was calculated via dividing the ABTS units/mL by the protein concentration in μg/mL and multiplying with 1000.

#### 4.1.4 HRP A2A and guaiacol

pH optimum of HRP A2A plus guaiacol

Similarly to the verification of the pH optimum of the HRP-catalyzed ABTS reaction, another assay was performed in buffers of different pH with a second standard substrate, guaiacol. As standard buffer 20 mM phosphate-KOH buffer, pH 7.0 as published by Morawski et al. (2000) was tested. Moreover, further 20 mM phosphate-KOH buffers were tested at pH 5.0, 6.0 and 8.0, 20 mM citrate-NaOH buffers at pH 3.0, 3.5, 4.0, 4.5, 5.0, 6.0, 7.0 and 20 mM Tris-HCl buffers at pH 7.0, 8.0, 9.0, 10.0 and 10.5.

**Michaelis-Menten kinetics of HRP A2A plus guaiacol**

Using the gathered data on the optimal pH for the conversion of guaiacol by HRP A2A kinetic studies were performed in analogy to those of A2A with ABTS with the following accommodations in the calculations:
f = dilution factor = 65 and
$\varepsilon_{guaiacol}$ = absorption coefficient of guaiacol = 26,000 M-1 cm-1.
Thus guaiacol units were aimed to be obtained corresponding to varying concentrations of the substrate (0.5, 0.75, 1.0, 3.0, 5.0, 7.0, 10.0, 20.0 mM). 1 guaiacol unit is defined as the amount of enzyme that converts 1 μmol guaiacol per minute.

#### 4.1.5 Stability studies with HRP A2A

**Optimal storage pH of HRP A2A**

The influence of pH on the storage stability of HRP A2A was evaluated. Therefore, the purified HRP A2A was diluted 1:20 in buffers of different pH (20 mM citrate-NaOH buffers at pH 3.0, 4.0, 5.0, 6.0 and 7.0 and 20 mM Tris-HCl buffers at pH 8.0, 9.0 and 10.0) and stored the different tubes at 4°C. In order to measure the starting / remaining activities at the time points 0 d, 2 d, 6 d, 10 d, 20 d (for pH 3.0 also measured after 1 d), and at the same assay conditions, aliquots from the tubes were further diluted with the different pHs 1:10 in 50 mM NaOAc buffer, pH 4.5 and the ABTS assay performed.

**Temperature stability of HRP A2A**

Pure A2A 1:200 (i.e. 0.3 ng/μL) was diluted in 50 mM NaOAc buffer, pH 4.5 and incubated at different temperatures: 20°C, 37 °C, 50°C, 65 °C. For each incubation temperature aliquots were taken after several time points and stored on ice until the end of the whole experiment. Finally, the HRP activities from all time points of one incubation temperature were measured at once with the ABTS assay.

**Dependence of the stability of HRP A2A on protein concentration**

HRP A2A stays stable for long time (>> 20 d) as long as it is undiluted. The purified HRP A2A was diluted with the Superdex buffer to the following concentrations: 60.0 ng/μL, 2.0 ng/μL, 1.2 ng/μL, 0.8 ng/μL, 0.6 ng/μL, 0.46 ng/μL and 0.38 ng/μL. The HRP activity was measured after 0 h, 1 h, 5 h, 1 d, 3 d and 10 d. Hereto, aliquots were taken from the various dilutions and separately further diluted to a final total dilution of 1:200 (i.e. 0.3 ng/μL) and the ABTS assay performed.
A 6.7 ng/μL concentrated solution of HRP A2A was examined for the existence of nanoparticles using the ZetaPlus Zeta Potential Analyzer.

### 4.2 Results and discussion

#### 4.2.1 Posttranslational modifications of HRP A2A

The acquired MALDI-TOF MS (matrix-assisted laser desorption/ionization - time-of-flight mass spectrometry) data for intact mass determination revealed two main species of A2A.

Top-down sequencing via MALDI-TOF-TOF MS provided data that suggested an unprocessed C-terminus and two varying N-termini: They differed in one amino acid from the C-terminal end of the α-factor signal sequence, which obviously has been heterogeneously cleaved from the mature HRP A2A peptide. One species had a N-terminal AEA upstream the mature HRP A2A sequence, the other species had EAEA. Thus the Ste13 site was not correctly processed in *P. pastoris.*

Moreover, the gathered data suggested Cys11 to build a disulfide bridge, which also has been predicted by the PredictProtein tool and which is also annotated for the HRP A2 published at UniProt.

PredictProtein further predicted the same seven N-glycosylation sites (N3, N13, N147, N185, N197, N211, N267) that are also annotated at UniProt. The mass spectrometric analysis detected a HexNAc (N-acetylhexose) residue on N3, N13 and N147. N185 did not seem to be modified, the other N-glycosylation sites were not covered by the acquired data.

Assuming a total of seven HexNAc residues per molecule, the two species with the differing N-termini fitted perfectly to the determined intact masses. Probably N197, N211 or N267 carries not one, but two HexNAc residues and thus accounts for the seemingly unmodified N185. This small extent of glycosylation was also reflected as a discrete protein band on the SDS gel (see figure 17).

Having a homogeneously glycosylated HRP A2A might significantly simplify eventual crystallization and structure analysis, which has been hindered so far by the N-glycans' heterogeneity of HRP A2 directly isolated from horseradish.

### 4.2.2 Isoelectric focusing of HRP A2A

The isoelectric focusing of HRP A2A indicated an IEP at pH 3.5 - 4.2 (see figure 16) which was close to the calculated theoretical IEP at pH 4.82. Most probably, the ionizable amino acid groups were predominantly not present on the enzyme's surface, which could explain its unexpected binding behavior towards QFF.

The IEP of the isoenzymes in the Sigma type I preparation was mainly around pH 5.0, the type II preparation showed two predominant isoenzymes at pH 5.3 - 6.0 and 3.5 - 4.2. The latter also seemed to be present in type VI, VI-A and XII with additional isoenzymes at 5.3 - 6.0 in type VI. The Toyobo preparation also showed an isoenzyme at pH 3.5 - 4.2, but slightly more basic than the A2A and the isoenzyme identified in the Sigma preparations.

### 4.2.3 HRP A2A and ABTS

#### pH optimum of HRP A2A plus ABTS

The pH optimum for the conversion of ABTS by HRP A2A at pH 4.5 was successfully identified (see figure 17). Interestingly, Hiner et al. reported in 2001 a broad pH optimum of HRP A2 for the conversion of ABTS at pH 5.5 - 6.5 (Journal of biological inorganic chemistry: JBIC: a publication of the Society of Biological Inorganic Chemistry 6, 504-16). However, the HRP A2 used for that publication was the preparation HRP-5 from Biozyme Labs (Blaenavon, UK) and has just been identified as A2 by isoelectric focusing (but not by sequencing). Most likely, this HRP also differs from the present recombinant A2A in its glycosylation pattern, which might also influence its enzymatic properties. In addition, the verified sequence used for the recombinant production of A2A differed from the published A2, which could also explain the varying results.

No activity could be detected at pH lower than 3.5 or higher than 9.0. Since HRP activity is also absent at pH lower than 3.5 or higher than 9.0 for the conversion of guaiacol (see 4.2.4), it might be that the enzyme itself is impaired in its activity at this pH.

In analogy to current literature on pH profiling using ABTS as substrate (Hiner et al. (2001)), the influence of pH on the absorption spectrum of ABTS was not taken into consideration for these studies. Consequently, in order to be able to evaluate the mere enzymatic activity of HRP A2A in the conversion of ABTS at a certain pH, further experiments need to be performed (e.g. the quantification of formed product by A2A at a certain pH after a given time via HPLC).

#### Michaelis-Menten kinetics of HRP A2A plus ABTS

The performed BCA assay for protein quantitation revealed that both, BSA and HRP-VI-A were suitable as standard proteins for calculating the concentration of the purified HRP A2A (see figure 18).

HRP A2A quantitation by using the BSA standard curve yielded a concentration of 56.9 ng/μL and 63.1 ng/μL by using the HRP VI-A standard curve, giving an average HRP A2A concentration of 60.0 ng/μL, which was used for all further calculations.

By plotting the calculated ABTS units/mg of the measured activities against the respective ABTS concentrations a saturation curve following Michaelis-Menten kinetics was obtained (see figure 19a). Plotting of the reciprocal values of the reaction rates against the reciprocal values of the corresponding ABTS concentrations yielded the Lineweaver-Burk plot (figure 19b) which allowed the determination of $V_{max}$ and $K_M$ with ABTS as substrate by calculating the reciprocal negative value of the crossing point of the trendline and the x-axis (i.e. $K_M$) and the reciprocal value of the crossing point of the trendline and the y-axis (i.e. $V_{max}$):

$$V_{max} = 793 \text{ ABTS units/mg}$$

$$K_M = 0.44 \text{ mM ABTS.}$$

As for the pH profile of HRP A2A, the values Hiner et al. published for their A2 isoenzyme differed from this one: They reported a $V_{max}$ = 1,432 ABTS units/mg and a $K_M$ = 4.0 mM at pH 4.5 (34). The HRP A2A was already in saturation at a concentration of 3.0 mM ABTS. Interestingly, this means a two times faster maximum conversion speed, but a ten times lower substrate affinity of their isoenzyme.

#### 4.2.4 HRP A2A and guaiacol

#### pH optimum of HRP A2A plus guaiacol

The pH optimum for the conversion of guaiacol by HRP A2A at pH 5.0 was verified, however the measured activities stayed at comparable levels until pH 7.0. In contrast to the corresponding ABTS profile, the enzyme's pH optimum seemed to dominate over the assay's pH optimum, since the profile much more resembled a normal distribution. Similarly to the ABTS profile, no significant activity could be measured below pH 3.5 or above pH 9.0 (see figure 20).

Contrary to the pH profile with ABTS, the findings for the guaiacol pH profile matched the data published by Hiner et al. (2001). They also reported similar results for the conversion of guaiacol with HRP C1A. Probably, the guaiacol pH profile generally does not show bigger variations among the isoenzymes, which could explain the differences between A2A and their A2 in the ABTS assay, but not in the guaiacol assay. As for the pH profile of ABTS, the dependence of the absorption spectrum of guaiacol on pH was neglected and remains to be verified in further studies.

#### Michaelis-Menten kinetics of HRP A2A plus guaiacol

As for the graphical representation and determination of the kinetic constants of A2A with ABTS, the calculated guaiacol units/mg of the measured activities were plotted against the corresponding substrate concentrations and thus obtained a curve that goes into saturation at a certain guaiacol concentration. $V_{max}$ and $K_M$ for the conversion of guaiacol by HRP A2A were again determined by creating a Lineweaver-Burk plot (figure 21):

$$V_{max} = 59 \text{ guaiacol units/mg,}$$

$$K_M = 1.03 \text{ mM guaiacol.}$$

Hiner et al. (2001) published a comparable $V_{max}$ = 81 guaiacol units/mg for the conversion of guaiacol by their HRP A2. Unlike for the conversion of ABTS, their isoenzyme seems to show catalytic properties towards guaiacol, that are comparable to the obtained data with recombinant A2A.

#### 4.2.5 Stability studies with HRP A2A

#### Optimal storage pH of HRP A2A

The activity of HRP A2A seems to be best preserved in buffers of pH 7.0 - 10.0 (see table 9). In pH 3.0 and 4.0, the HRP activity was completely abrogated; in pH 5.0 and 6.0, the activity was significantly decreased; in pH 7.0 - 10.0, the activity was equivalently well preserved.

Table 9

| pH | residual HRP activity after 20 d % | pH | residual HRP activity after 20 d % |
|---|---|---|---|
| 3.0 | 0.0 | 7.0 | 76,7 |
| 4.0 | 0.0 | 8.0 | 77,7 |
| 5.0 | 10,4 | 9.0 | 80,6 |
| 6.0 | 50,9 | 10.0 | 77,3 |

The total measured activities in all pHs over 20 d are depicted in figure 22. In pH 3.0, the HRP activity is completely abrogated in short time (no activity measurable after 1 d). In pH 4.0, the activity also continuously decreased and was below detection limits after 10 d. However, even at this relatively low pH still a third of its original activity was detected after two days. HRP A2A still yielded measurable, but significantly decreased activity after 20 d in pH 5.0, and with a further descending trend. In pH 6.0, the activity was decreased to 50 % of the starting activity after 20 d, however it seemed to stay stable at this level. The best preserved HRP activity was detected in buffers of pH 7.0 - 10.0. The residual HRP activity in these four buffers was approximately 78 % of the initial activity and stayed stable at this level.

This data extends the findings from the pH profiles: HRP A2A might have shown no activity at pH 3.5, because it was inactivated immediately, when transferred to the buffer of this pH. However, enzyme inactivation at pH higher than 9.0 seems not to be an explanation for the absence of HRP activity at that pH. The decrease in activity at basic pH seems to be rather due to impairments of the assay's reaction itself (e.g. an altered absorption spectrum of the measured product at higher pH).

**Temperature stability of HRP A2A**

The 0.3 ng/μL diluted HRP A2A met the expectations and lost its activity over time the faster, the higher the incubation temperature was (see figure 23). Storage for 60 min at 4 °C decreased the activity to 75 % of the starting activity (4°C data was taken from the experiment investigating the influence of protein concentration on stability, see below; data from 0.38 ng/μL HRP A2A). After 60 min, the diluted HRP A2A showed 50 % of the initial activity with a descending trend at room temperature (i.e. 20°C). At 37 °C, HRP activity was measurable for 30 min. At 50°C and 65 °C, the activity was abrogated after 10 min and 3 min, respectively.

The finding, that HRP activity decreased relatively fast even at 4°C did not match the observations on HRP stability made so far: Not any significant loss of activity was seen over months of HRP A2A stored at 4°C. However, contrary to the A2A used for this temperature stability study, the stored A2A was undiluted (i.e. 60 ng/μL), hence it is hypothesized, that protein concentration might play a crucial role in HRP stability (see below).

**Dependence of the stability of HRP A2A on protein concentration**

It is assumed that the stability of HRP A2A depends on the protein concentration of the solution it is dissolved in. By studying the HRP activities over time at different concentrations, the hypothesis was successfully confirmed and the crucial "point-of-no-return" in protein concentration identified above which HRP stability is guaranteed, and below which HRP activity decreases rather fast.

After storing HRP A2A at 4 °C for 10 d in concentrations ≥ 0.6 ng/μL no significant loss of HRP activity could be detected. However the two lower concentrated HRPs completely lost activity within one day (see figure 24).

**Example 5. Surface variants for oriented enzyme immobilization**

Increased stability and the possibility for oriented immobilization of HRPs are desired features for the diverse uses of HRPs in biotechnological applications. In this study, 2 single and 12 double mutants of solvent exposed lysine residues were created, codon optimized and successfully expressed in *Pichia pastoris.* The mutations and expression levels of each variant enzyme after standard cultivation and methanol induction are depicted in Table 10. The values from 96-well deep-well cultivations were not normalized and thus not directly comparable between mutants, but demonstrated that substantial amounts (>100mABS/min using standard ABTS assay) of 12 of the 14 surface variants can be produced in *P. pastoris.*

The production of three surface variants HRPC1AsynK232N_K241N, HRPC1AsynK174R_K241 N and HRPC1AsynK174Q_K241 F was up-scaled to 5 l volume (BiostatC fermenta), yielding expression levels of 34.1U/ml; 30.2U/ml and 44.3U/ml, respectively. The yield of codon optimized C1A without modifications was 5.3U/ml and wt C2 2.2U/ml. The aforementioned yields are not directly comparable due to varying feeds used. Nevertheless, these data demonstrate the active expression of these surface lysine variants.

Table 10

| Variant # | SEQ ID NO: | AA variation | Activity mABS/min |
|---|---|---|---|
| 0 | 50 | HRPC1Asyn | 363 |
| 1 | 35 | HRPC1AsynK232Q_K241N | 236 |
| 2 | 36 | HRPC1AsynK232Q_K241F | 146 |
| 3 | 37 | HRPC1AsynK232N | 1428 |
| 4 | 38 | HRPC1AsynK232N_K241N | 293 |
| 5 | 39 | HRPC1AsynK232N_K241F | 77 |
| 6 | 40 | HRPC1AsynK174R_K241N | 135 |
| 7 | 41 | HRPC1AsynK174R_K241F | 45 |
| 8 | 42 | HRPC1AsynK174R_K232Q | 234 |
| 9 | 43 | HRPC1AsynK174R_K232N | 307 |
| 10 | 44 | HRPC1AsynK174Q_K241N | 157 |
| 11 | 45 | HRPC1AsynK174Q_K241F | 108 |
| 12 | 46 | HRPC1AsynK174Q_K232Q | 196 |
| 13 | 47 | HRPC1AsynK174Q_K232N | 133 |
| 14 | 48 | HRPC1Asyn_T110V | 56 |
| 15 | 49 | HRPC1Asyn_K241F | 134 |

## Claims

1. A recombinant heme-containing horseradish peroxidase isoenzyme encoded by a nucleic acid comprising a sequence selected from the group consisting of SEQ ID NO:1 to SEQ ID NO:49 and functionally active variants thereof.

2. An isoenzyme according to claim 1, wherein said isoenzyme is less glycosylated as compared to the isoenzyme C1A.

3. An isoenzyme according to claim 1 or 2, wherein said isoenzyme has a thermal stability of at least 2 weeks at 20-25°C and at least several months at 4 °C.

**4.** An isoenzyme according to any one of claims 1 to 3, wherein said isoenzyme has a biological acitvity of at least 500 U/mg, preferred of at least 750 U/mg, more preferred of at least 1.000 Umg and most preferred of at least 1.200 U/mg in the ABTS assay.

**5.** An isoenzyme according to any one of claims 1 to 4, wherein said isoenzyme has a calculated isoelectric point of less than 5 or greater than 6.

**6.** An isoenzyme according to any one of claims 1 to 5, wherein said isoenzmye has a lower $K_m$ when compared to the isoenzyme C1A.

**7.** The isoenzyme according to any one of claims 1 to 6, wherein said isoenzymy is A2A.

**8.** A vector comprising a polynucleotide according to claim 1.

**9.** A host cell comprising a vector according to claim 8.

**10.** A host cell according to claim 9, wherein the host cell is a prokaryotic cell, preferably *E. coli* or *B. subtilis* or a eukaryotic cell, preferably a yeast cell.

**11.** A host cell according to claim 10, wherein the host cell is a *Pichia* cell, preferably a *P. pastoris* or *P. angusta* cell.

**12.** A method for producing a recombinant heme-containing horseradish peroxidase isoenzyme according to any one of claims 1 to 7, which comprises

a. providing a recombinant host cell engineered to express a polynucleotide according to claim 1,
b. culturing the host cell under conditions suitable for obtaining the isoenzyme, wherein the expression product of the host cell is reacting with heme; and
c. recovering the isoenzyme from the culture.

**13.** Use of a horseradish peroxidase isoenzyme according any one of claims 1 to 7 as a reagent for organic synthesis and biotransformation, preferably in coupled enzyme assays, chemiluminescent assays, and immunoassays.

**14.** Use of a horseradish peroxidase isoenzyme according to any one of claims 1 to 7 in a biosensor, in diagnostics, in bioremediation or in chemical synthesis.

**15.** A horseradish peroxidase isoenzyme according to any one of claims 1 to 7 for use in targeted cancer therapy.

**Figure 1**

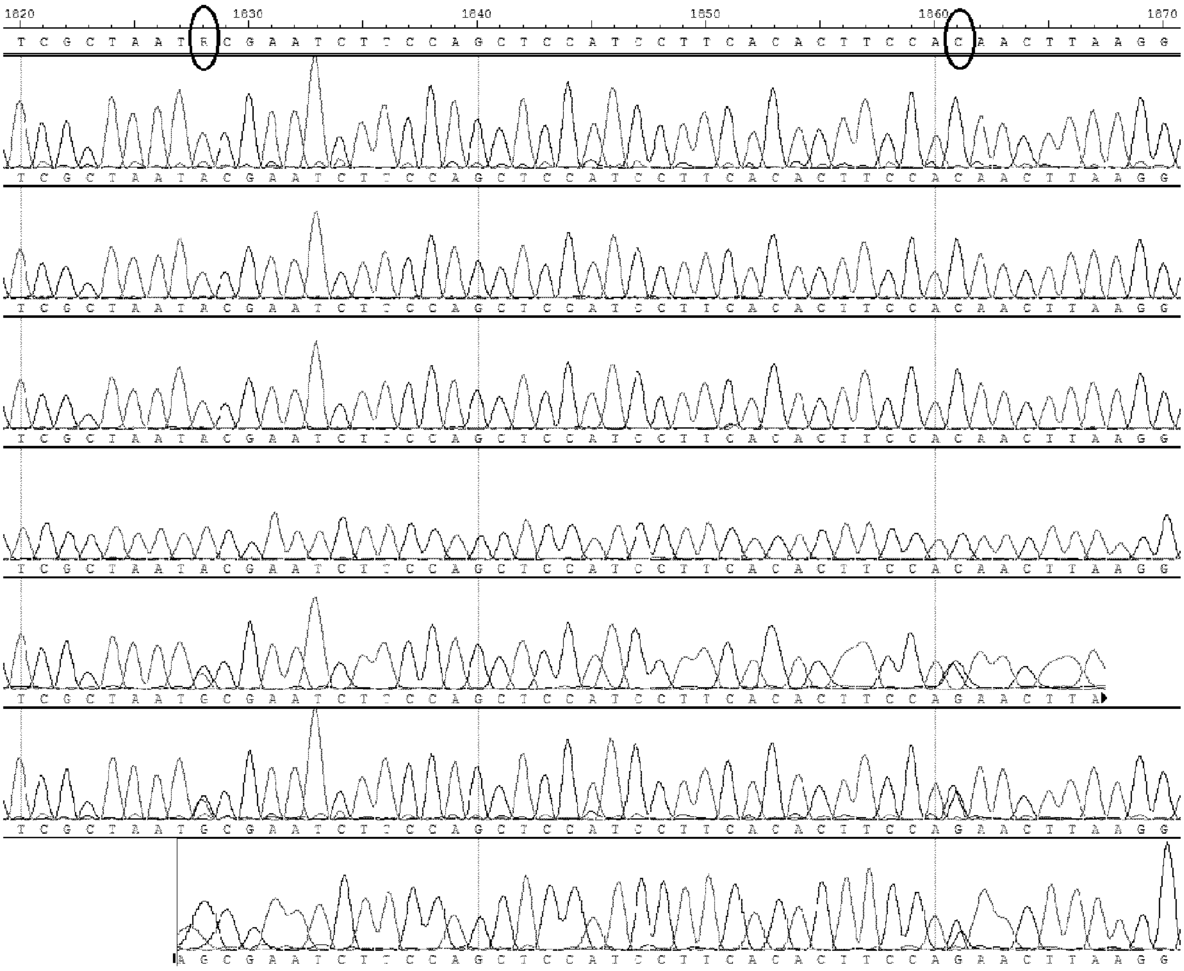


Figure 2

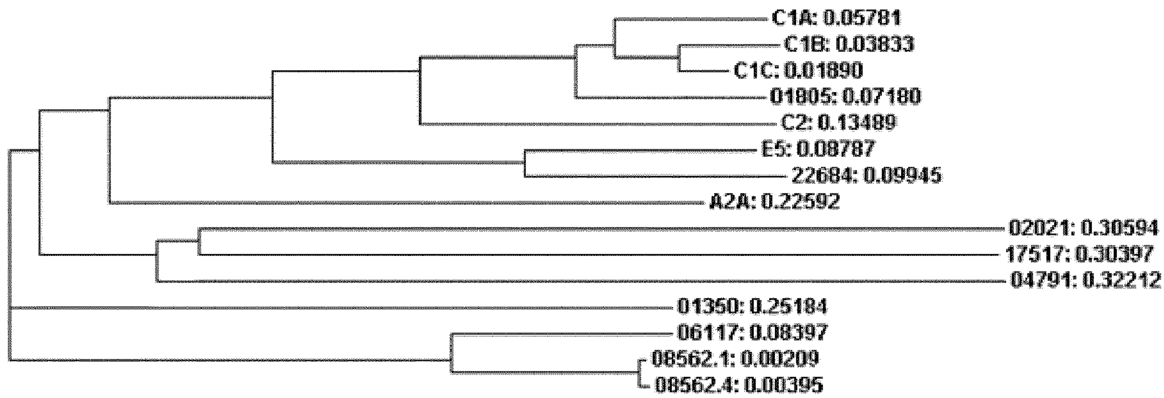

Figure 3:

| | | | |
|---|---|---|---|
| C1A | A2A | 01350 | 17517 |
| SDAQLTPTFYD | SSAQLNATFYS | VQGNYNNVVEA | VTARRPRVGFY |
| C1B | E5 | 02021 | 08562.1 |
| SDAQLTPTFYD | SNAQLRPDFYS | SEAQLQMNFYA | CLCDKSYGGKL |
| C1C | 01805 | 04791 | 08562.4 |
| SNAQLTPTFYD | SSAQLTPTFYD | IESRLTTNFYS | CLCDKSYGGKL |
| C2 | 22684 | 06117 | |
| SHAQLSPSFYD | SNAKLRPDFYL | CICDDESNYGG | |

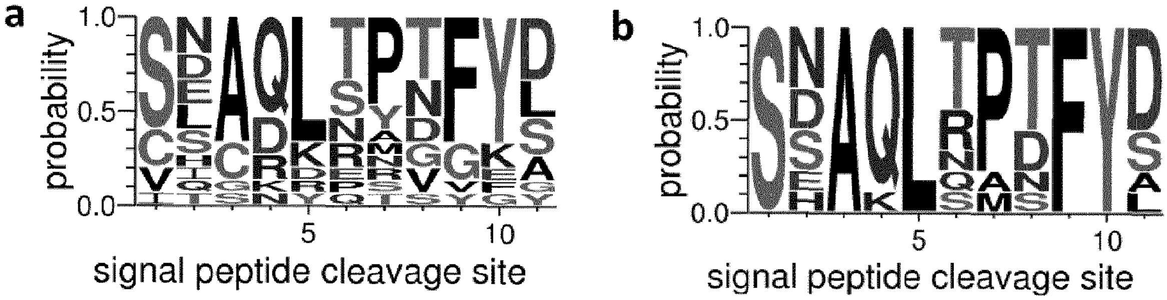


Figure 4

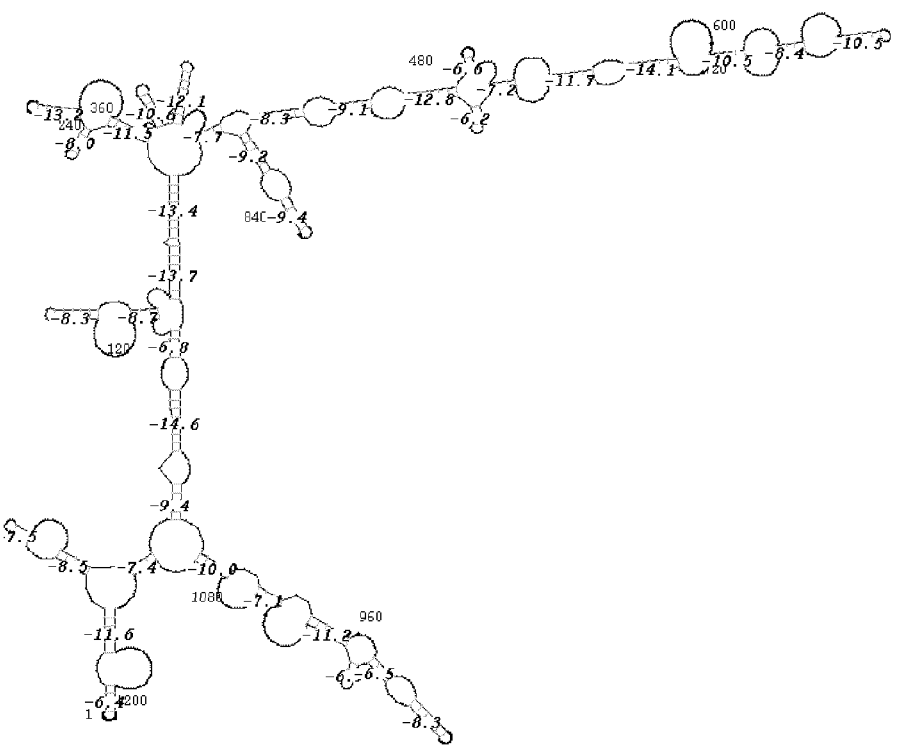

Figure 5

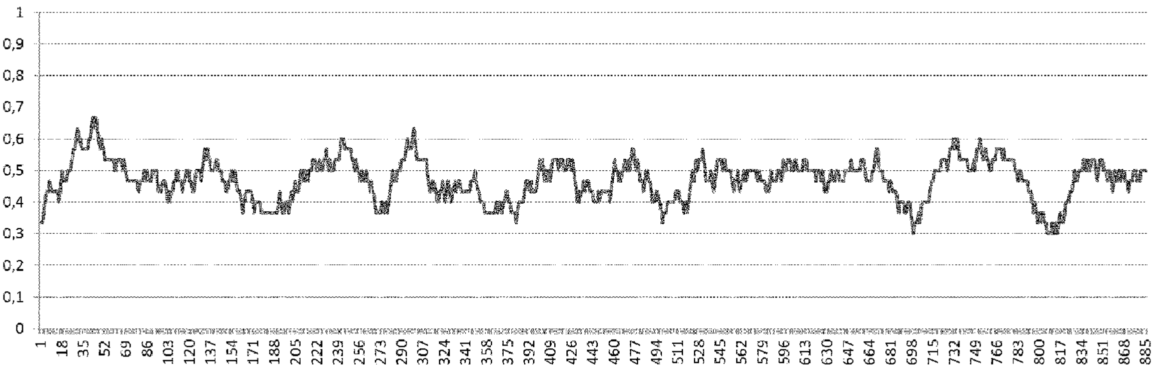

Figure 6:

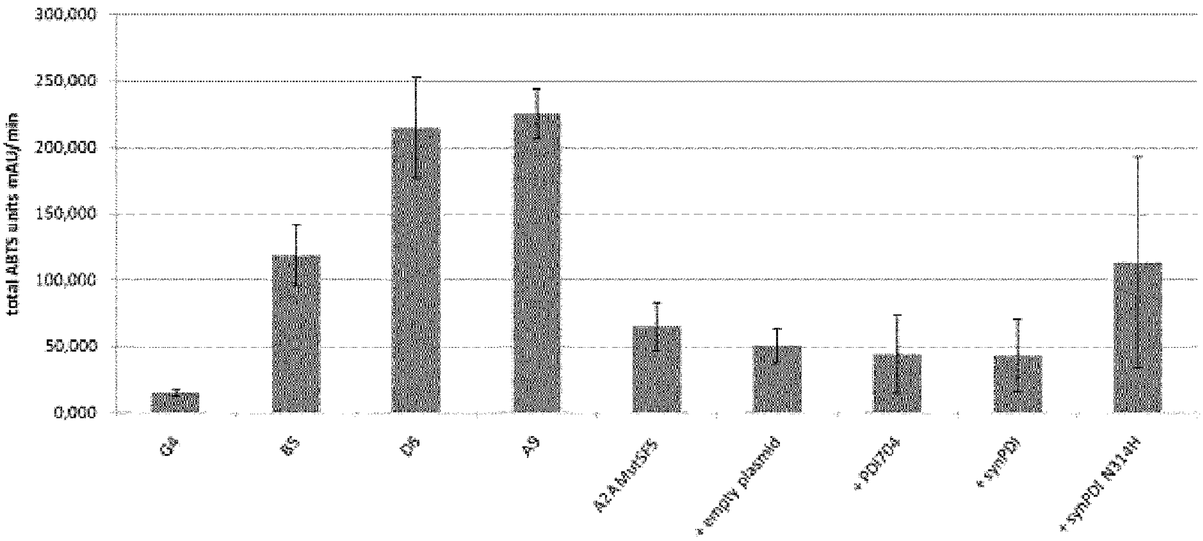
total ABTS units mAU/min
300,000
250,000
200,000
150,000
100,000
50,000
0,000
G4
B5
D5
A9
A2AMutSFS
+ empty plasmid
+ PDI704
+ synPDI
+ synPDI N314H

Figure 7:

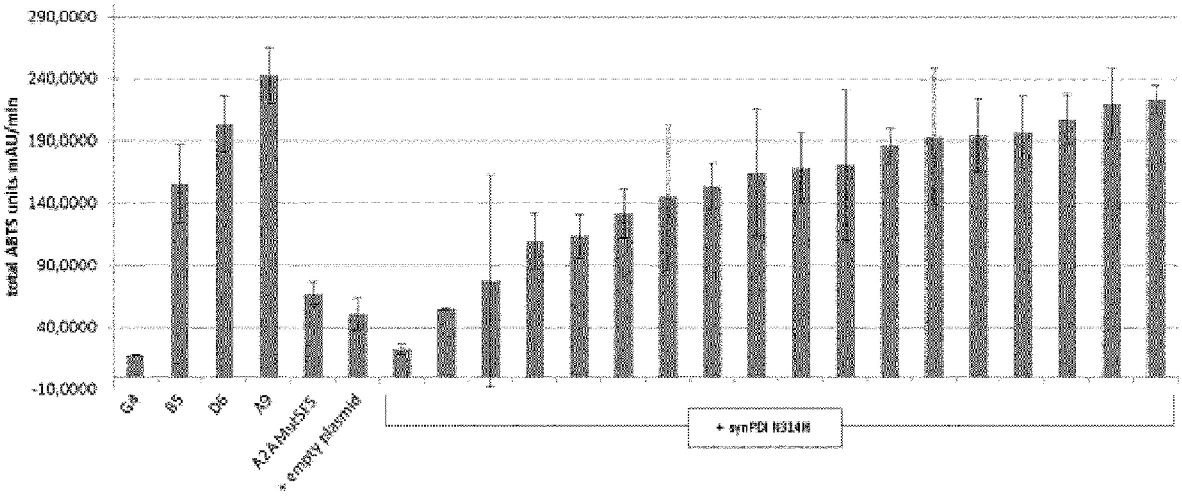
290,0000
240,0000
190,0000
140,0000
90,0000
40,0000
-10,0000
total ABTS units mAU/min
G4
B5
D6
A9
A2A MutSFS
+ empty plasmid
+ synPDI H314H

Figure 8:

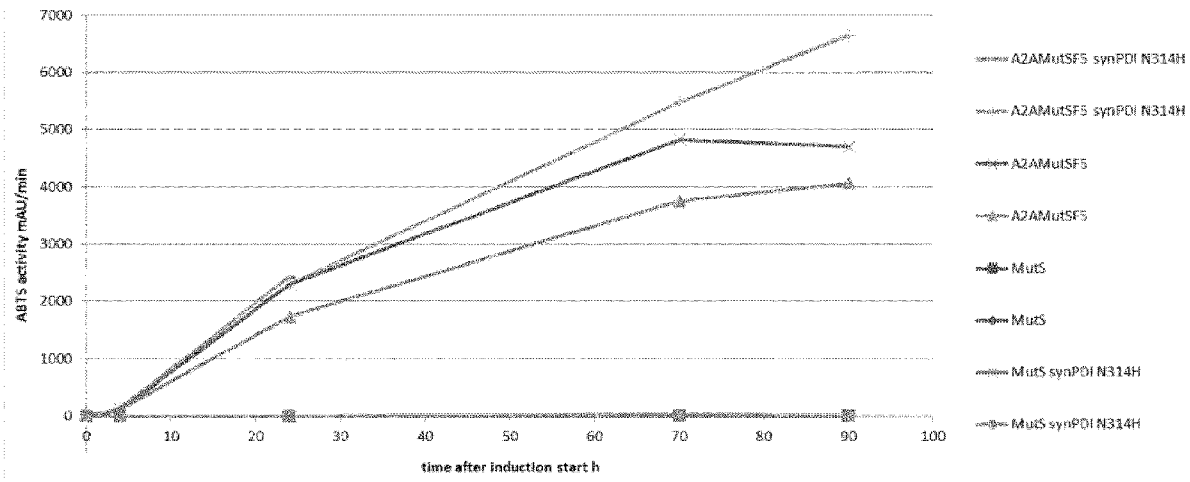
7000
6000
5000
4000
3000
2000
1000
0
ABTS activity mAU/min
0
10
20
30
40
50
60
70
80
90
100
time after induction start h
A2AMutSF5 synPDI N314H
A2AMutSF5 synPDI N314H
A2AMutSF5
A2AMutSF5
MutS
MutS
MutS synPDI N314H
MutS synPDI N314H

Figure 9

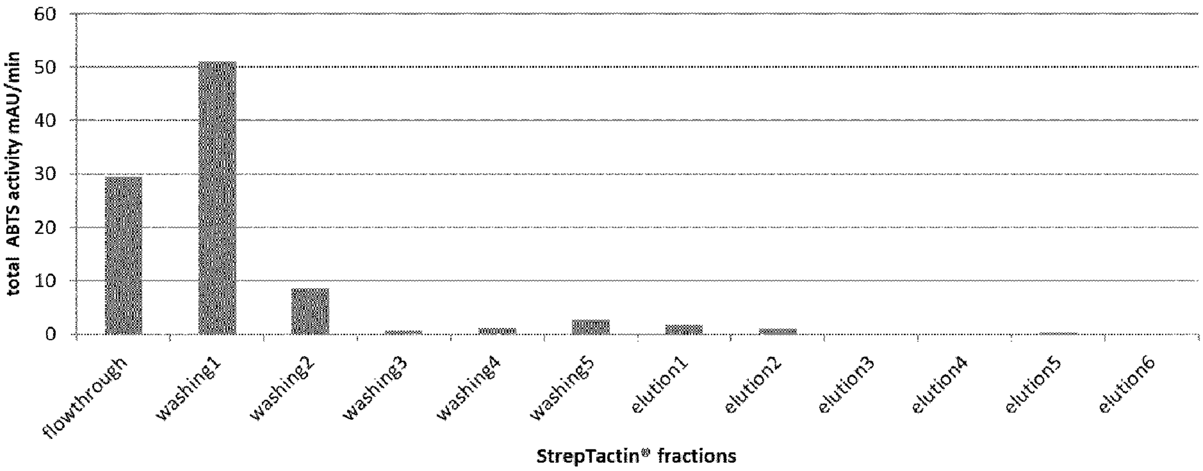
60
50
40
30
20
10
0
total ABTS activity mAU/min
flowthrough
washing1
washing2
washing3
washing4
washing5
elution1
elution2
elution3
elution4
elution5
elution6
StrepTactin® fractions

Figure 10

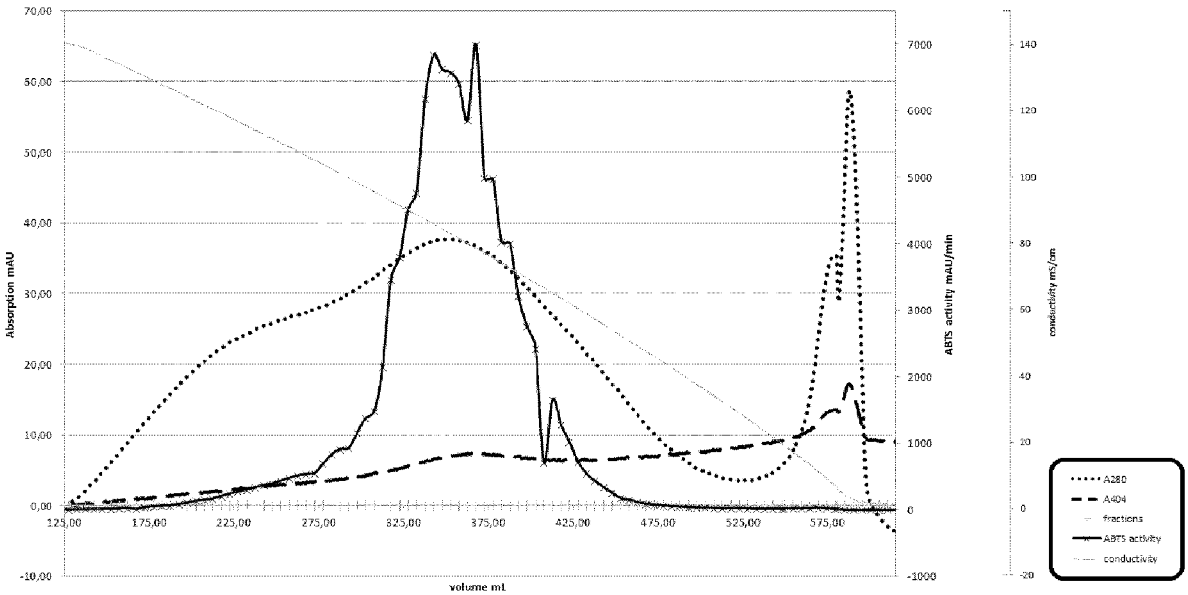
Absorption mAU
70,00
60,00
50,00
40,00
30,00
20,00
10,00
0,00
-10,00
125,00
175,00
225,00
275,00
325,00
375,00
425,00
475,00
525,00
575,00
volume mL
ABTS activity mAU/min
7000
6000
5000
4000
3000
2000
1000
0
-1000
conductivity mS/cm
140
120
100
80
60
40
20
0
-20
A280
A404
fractions
ABTS activity
conductivity

Figure 11:

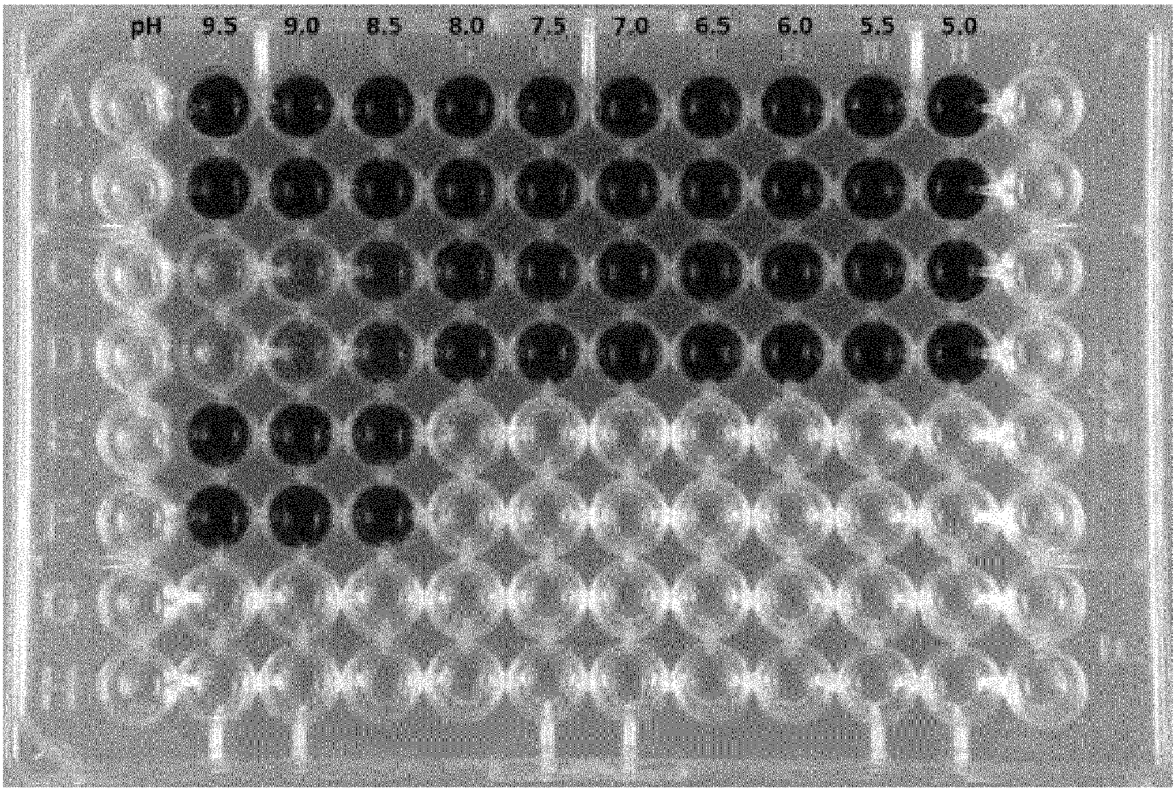

Figure 12:

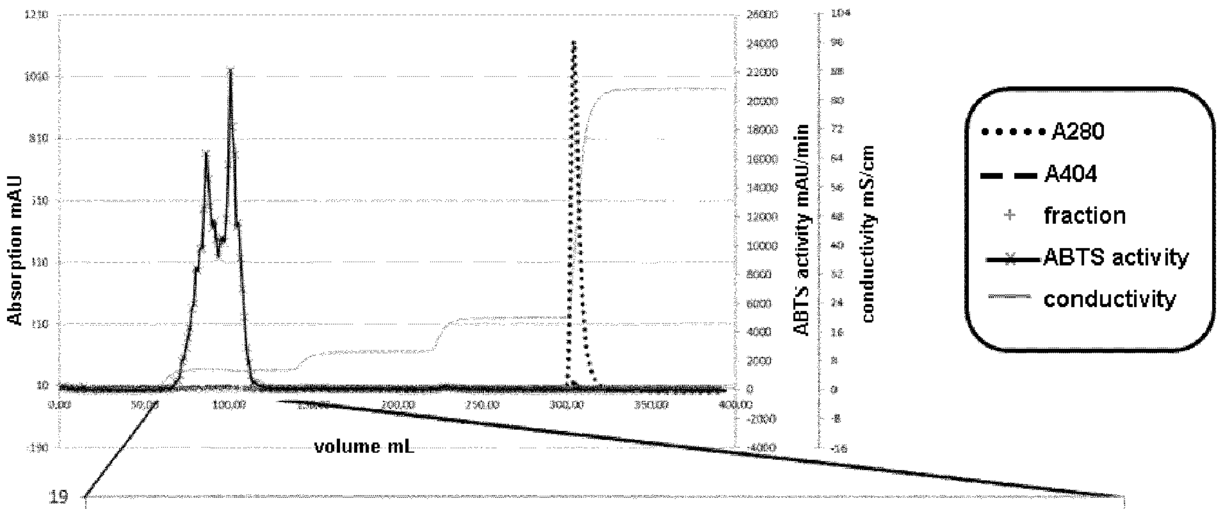
Absorption mAU
ABTS activity mAU/min
conductivity mS/cm
volume mL
A280
A404
fraction
ABTS activity
conductivity
19

Figure 12 continued:

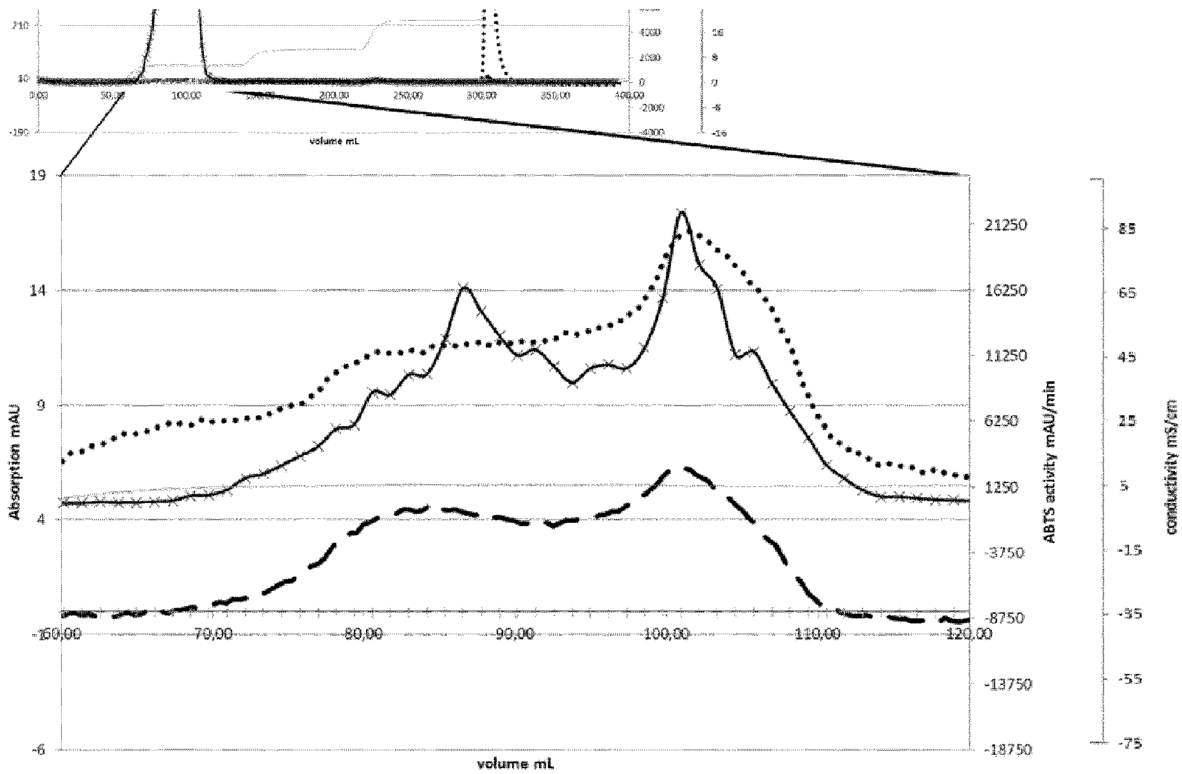

Figure 13:

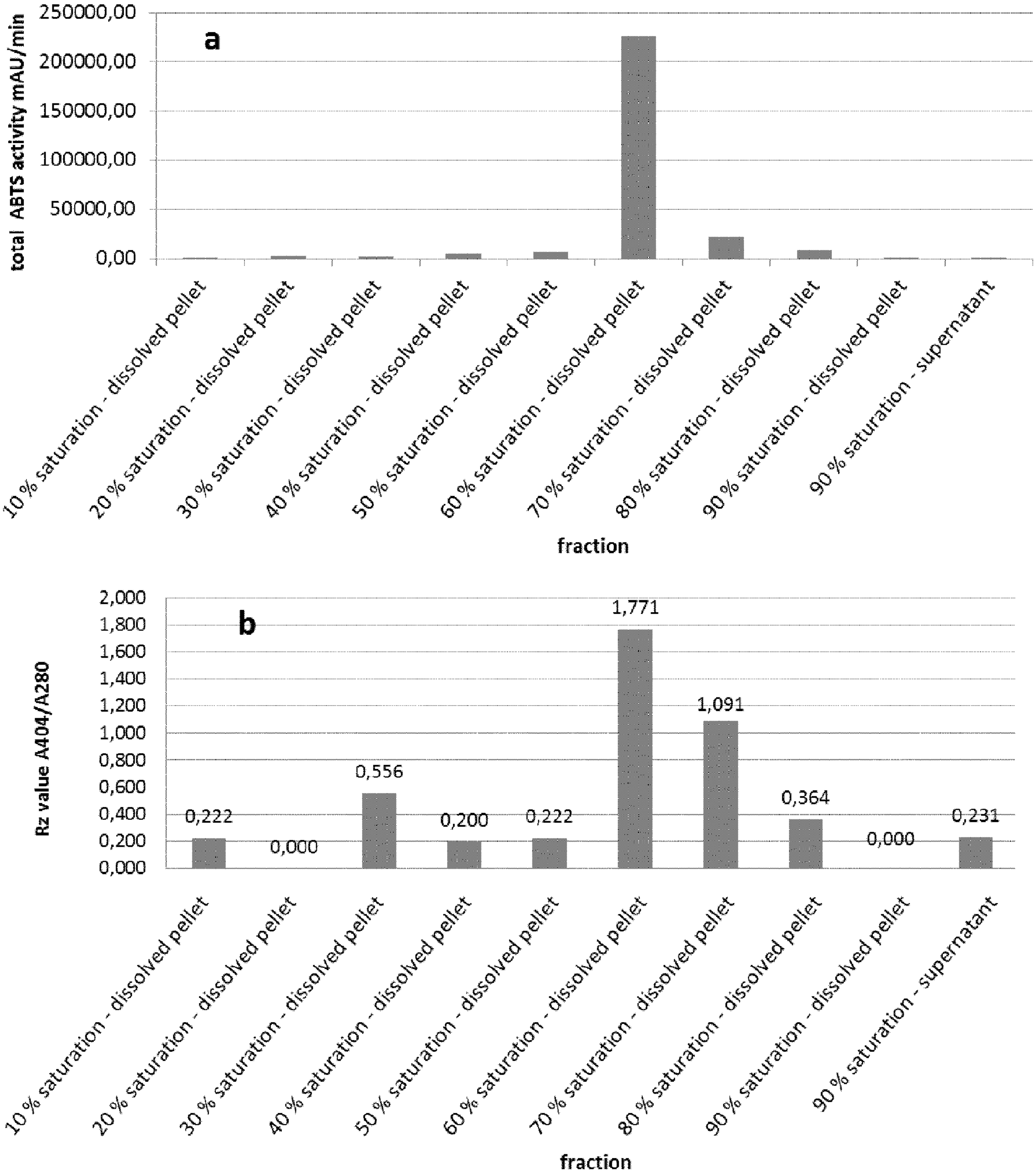
a
total ABTS activity mAU/min
250000,00
200000,00
150000,00
100000,00
50000,00
0,00
10 % saturation - dissolved pellet
20 % saturation - dissolved pellet
30 % saturation - dissolved pellet
40 % saturation - dissolved pellet
50 % saturation - dissolved pellet
60 % saturation - dissolved pellet
70 % saturation - dissolved pellet
80 % saturation - dissolved pellet
90 % saturation - dissolved pellet
90 % saturation - supernatant
fraction
b
Rz value A404/A280
2,000
1,800
1,600
1,400
1,200
1,000
0,800
0,600
0,400
0,200
0,000
0,222
0,000
0,556
0,200
0,222
1,771
1,091
0,364
0,000
0,231
10 % saturation - dissolved pellet
20 % saturation - dissolved pellet
30 % saturation - dissolved pellet
40 % saturation - dissolved pellet
50 % saturation - dissolved pellet
60 % saturation - dissolved pellet
70 % saturation - dissolved pellet
80 % saturation - dissolved pellet
90 % saturation - dissolved pellet
90 % saturation - supernatant
fraction

Figure 14

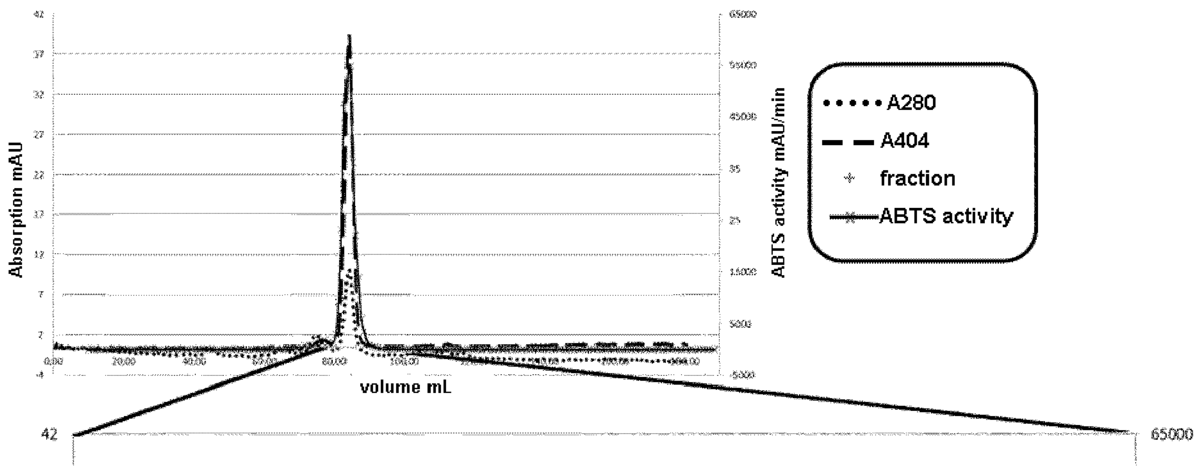
Absorption mAU
volume mL
ABTS activity mAU/min
A280
A404
fraction
ABTS activity
42
65000

Figure 14 continued:

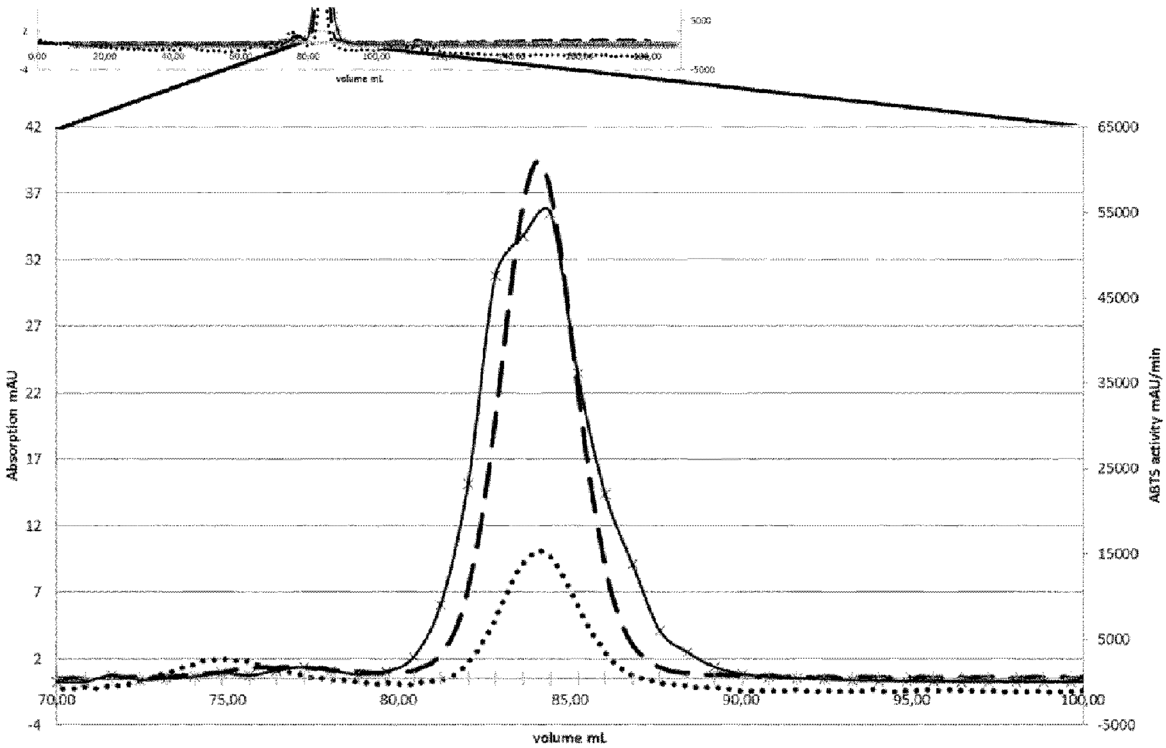

Figure 15

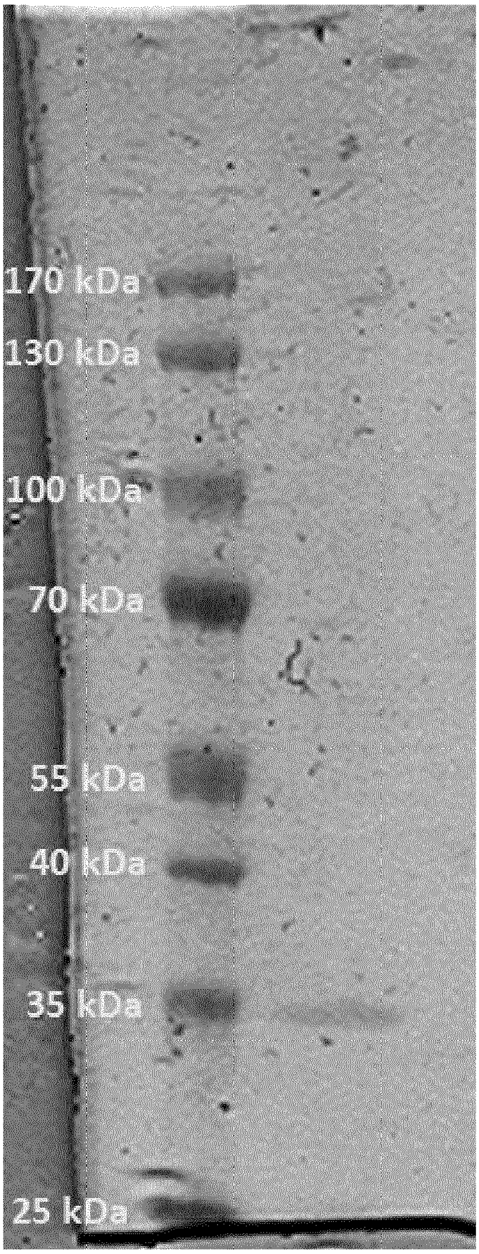

Figure 16

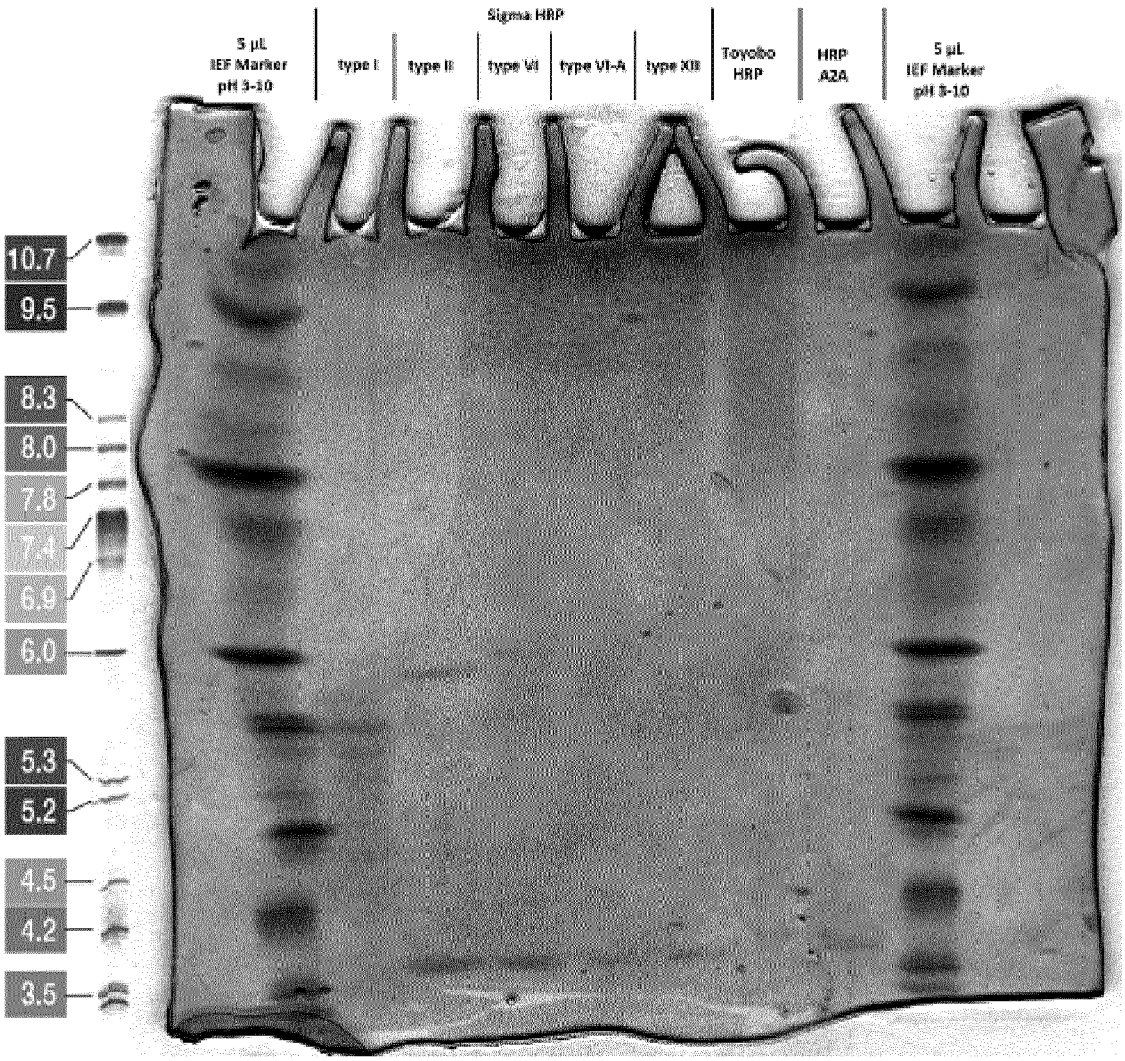
Sigma HRP
5 µL IEF Marker pH 3-10
type I
type II
type VI
type VI-A
type XII
Toyobo HRP
HRP A2A
5 µL IEF Marker pH 3-10
10.7
9.5
8.3
8.0
7.8
7.4
6.9
6.0
5.3
5.2
4.5
4.2
3.5

Figure 17

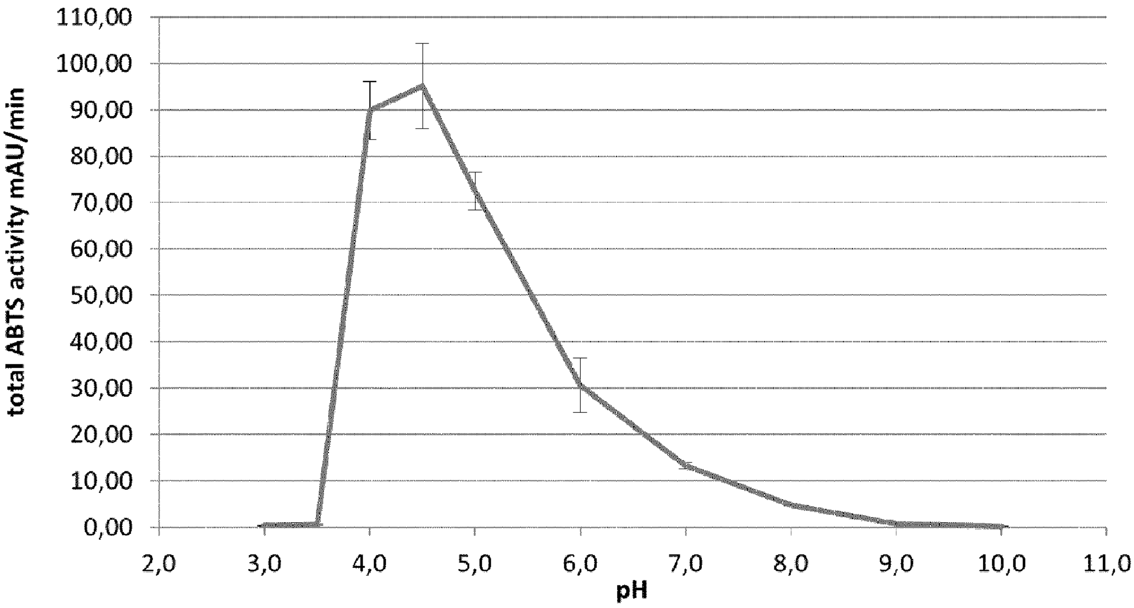
total ABTS activity mAU/min
110,00
100,00
90,00
80,00
70,00
60,00
50,00
40,00
30,00
20,00
10,00
0,00
2,0
3,0
4,0
5,0
6,0
7,0
8,0
9,0
10,0
11,0
pH

Figure 18

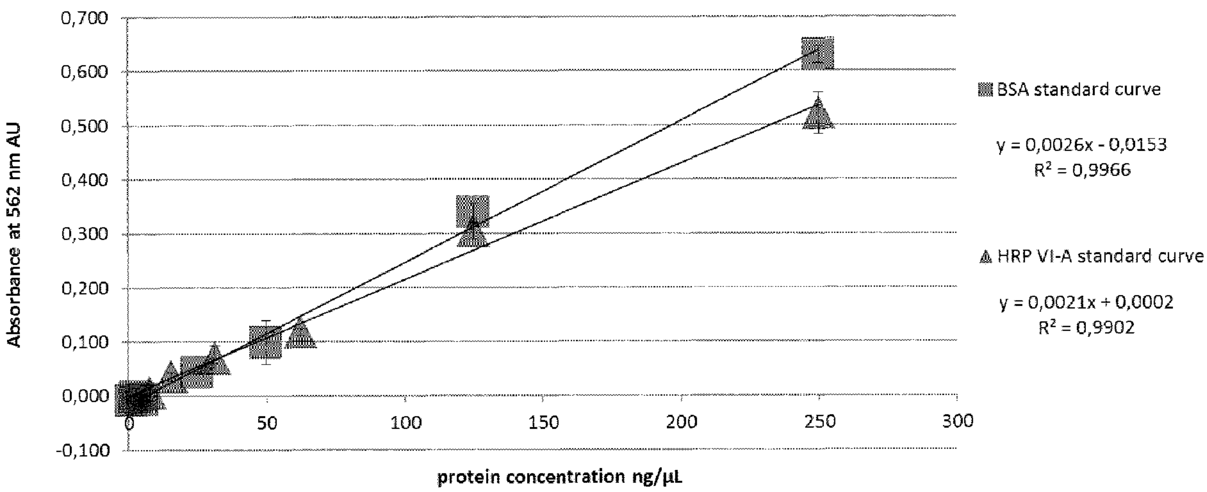
0,700
0,600
0,500
0,400
0,300
0,200
0,100
0,000
-0,100
Absorbance at 562 nm AU
0
50
100
150
200
250
300
protein concentration ng/µL
BSA standard curve
y = 0,0026x - 0,0153
R² = 0,9966
HRP VI-A standard curve
y = 0,0021x + 0,0002
R² = 0,9902

Figure 19

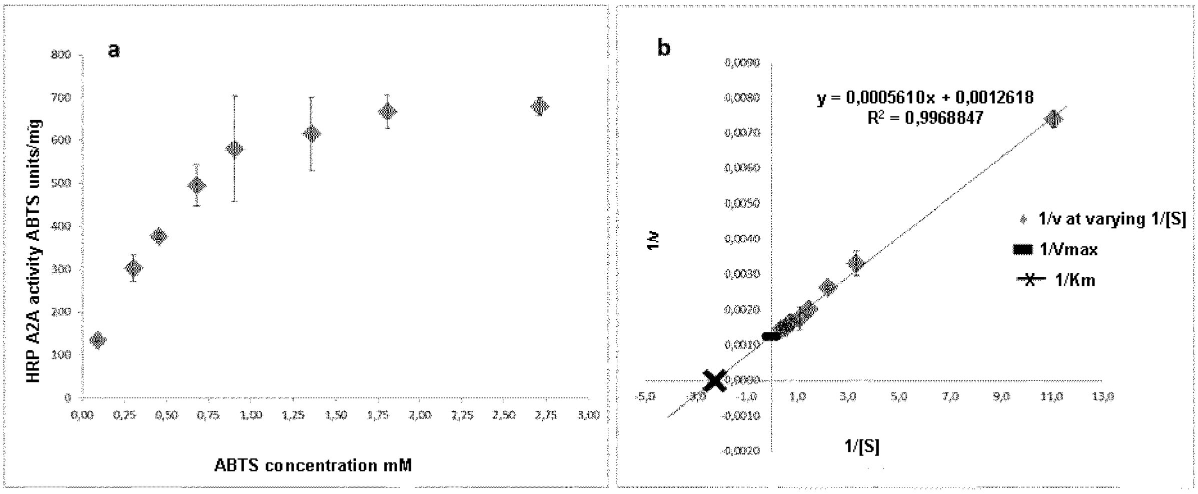
a
HRP A2A activity ABTS units/mg
ABTS concentration mM
b
y = 0,0005610x + 0,0012618
$R^2$ = 0,9968847
1/v
1/[S]
1/v at varying 1/[S]
1/Vmax
1/Km

Figure 20

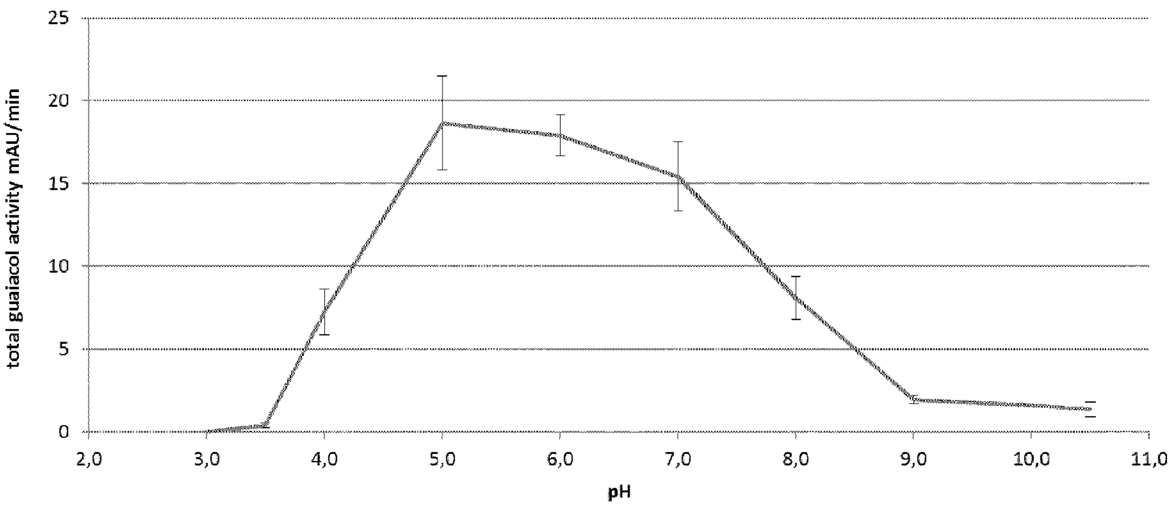
25
20
15
10
5
0
total guaiacol activity mAU/min
2,0
3,0
4,0
5,0
6,0
7,0
8,0
9,0
10,0
11,0
pH

Figure 21

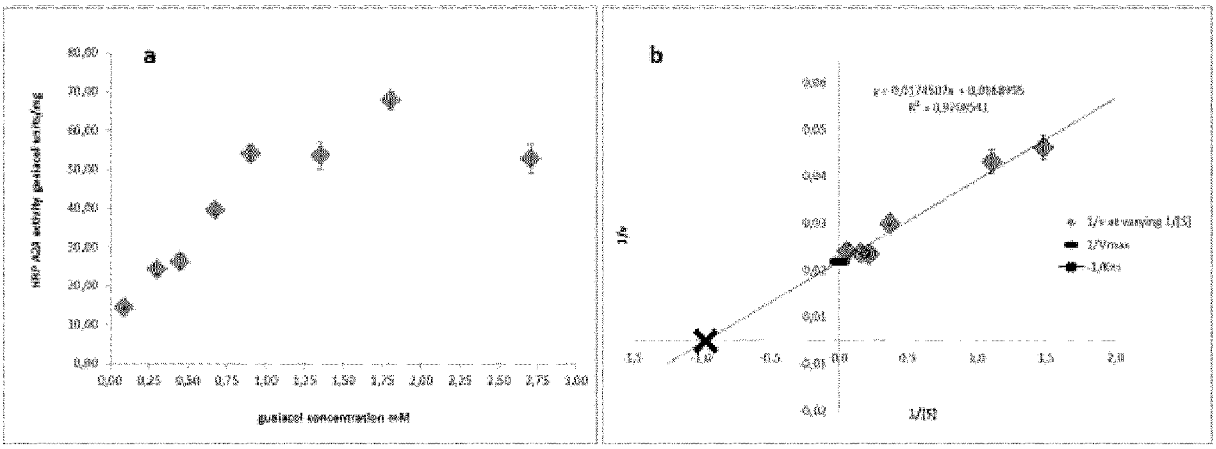

Figure 22

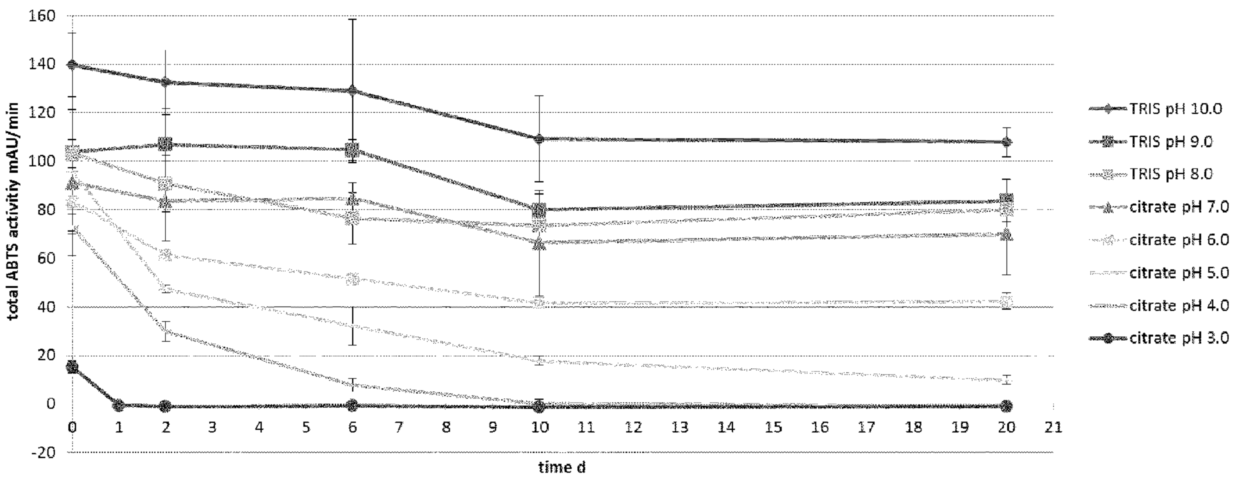

160
140
120
100
80
60
40
20
0
-20
total ABTS activitiy mAU/min
0 1 2 3 4 5 6 7 8 9 10 11 12 13 14 15 16 17 18 19 20 21
time d
TRIS pH 10.0
TRIS pH 9.0
TRIS pH 8.0
citrate pH 7.0
citrate pH 6.0
citrate pH 5.0
citrate pH 4.0
citrate pH 3.0

Figure 23

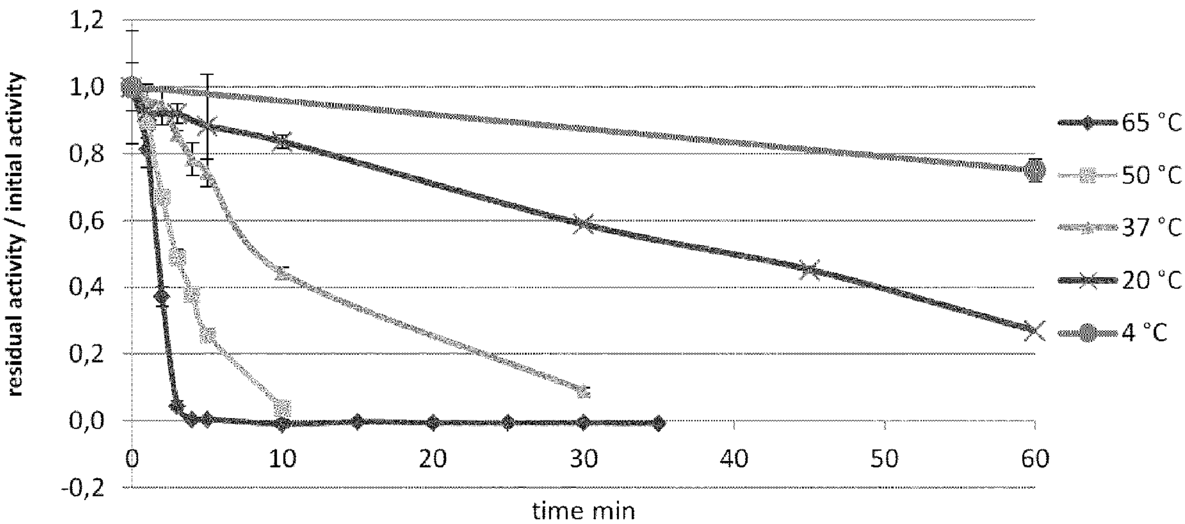
1,2
1,0
0,8
0,6
0,4
0,2
0,0
-0,2
0
10
20
30
40
50
60
time min
residual activity / initial activity
65 °C
50 °C
37 °C
20 °C
4 °C

Figure 24

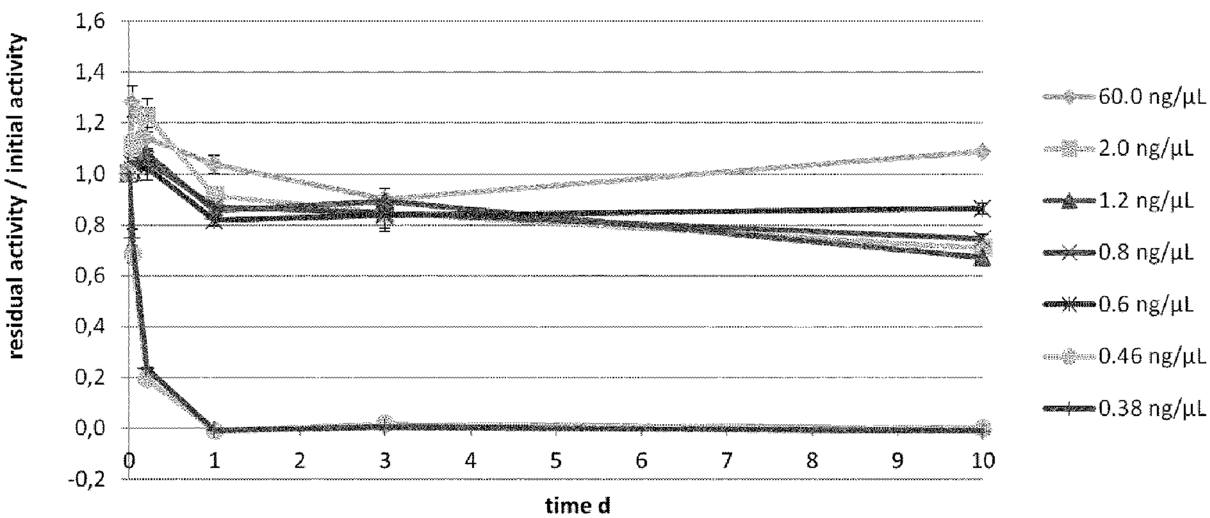
1,6
1,4
1,2
1,0
0,8
0,6
0,4
0,2
0,0
-0,2
residual activity / initial activity
0
1
2
3
4
5
6
7
8
9
10
time d
60.0 ng/µL
2.0 ng/µL
1.2 ng/µL
0.8 ng/µL
0.6 ng/µL
0.46 ng/µL
0.38 ng/µL

## Figure 25

```
C1B_transcriptome     MHSPSSTSFTWILITLGCLAFYASLSDAQLTPTFYDTSCPNVSNIVRDIIINELRSDPRI 60
C1B_genbank           MHSPSSTSFTWILITLGCLAFYASLSDAQLTPTFYDTSCPNVSNIVRDIIINELRSDPRI 60
C1B_uniprot           MHSPSSTSFTWILITLGCLAFYASLSDAQLTPTFYDTSCPNVSNIVRDIIINELRSDPRI 60
                      ************************************************************

C1B_transcriptome     TASILRLHFHDCFVNGCDASILLDNTTSFLTEKDALGNANSARGFPTVDRIKAAVERACP 120
C1B_genbank           TASILRLHFHDCFVNGCDASILLDNTTSFLTEKDALGNANSARGFPTVDRIKAAVERACP 120
C1B_uniprot           TASILRLHFHDCFVNGCDASILLDNTTSFLTEKDALGNANSARGFPTVDRIKAAVERACP 120
                      ************************************************************

C1B_transcriptome     RTVSCADVLTIAAQQSVNLAGGPSWRVPLGRRDSLQAFLDLANANLPAPFFTLPQLKDAF 180
C1B_genbank           RTVSCADVLTIAAQQSVNLAGGPSWRVPLGRRDSLQAFLDLANANLPAPFFTLPQLKDAF 180
C1B_uniprot           RTVSCADVLTIAAQQSVNLAGGPSWRVPLGRRDSLQAFLDLANANLPAPFFTLPQLKDAF 180
                      ************************************************************

C1B_transcriptome     AKVGLDRPSDLVALSGGHTFGKNQCRFIMDRLYNFSNTGLPDPTLNTTYLQTLRQQCPLN 240
C1B_genbank           AKVGLDRPSDLVALSGGHTFGKNQCRFIMDRLYNFSNTGLPDPTLNTTYLQTLRQQCPLN 240
C1B_uniprot           AKVGLDRPSDLVALSGGHTFGKNQCRFIMDRLYNFSNTGLPDPTLNTTYLQTLRQQCPLN 240
                      ************************************************************

C1B_transcriptome     GNQSVLVDFDLRTPTVFDNKYYVNLKEQKGLIQSDQELFSSPNATDTIPLVRSFADGTQK 300
C1B_genbank           GNQSVLVDFDLRTPTVFDNKYYVNLKEQKGLIQSDQELFSSPNATDTIPLVRSFADGTQK 300
C1B_uniprot           GNQSVLVDFDLRTPTVFDNKYYVNLKEQKGLIQSDQELFSSPNATDTIPLVRSFADGTQK 300
                      ************************************************************

C1B_transcriptome     FFNAFVEAMNRMGNITPLTGTQGEIRLNCRVVNSNSLLHDIVEVVDFVSSM 351
C1B_genbank           FFNAFVEAMNRMGNITPLTGTQGEIRLNCRVVNSNSLLHDIVEVVDFVSSM 351
C1B_uniprot           FFNAFVEAMNRMGNITPLTGTQGEIRLNCRVVNSNSLLHDIVEVVDFVSSM 351
                      ***************************************************


C1C_uniprot           MLHASFSNAQLTPTFYDNSCPNVSNIVRDIIINELRSDPSIAASILRLHFHDCFVNGCDA 60
C1C_genbank           MLHASFSNAQLTPTFYDNSCPNVSNIVRDIIINELRSDPSIAASILRLHFHDCFVNGCDA 60
C1C_transcriptome     MLHASFSNAQLTPTFYDNSCPNVSNIVRDIIINELRSDPRIAASILRLHFHDCFVNGCDA 60
                      *************************************** ********************

C1C_uniprot           SILLDNTTSFRTEKDAFGNANSARGFPVVDRIKAAVERACPRTVSCADVLTIAAQQSVNL 120
C1C_genbank           SILLDNTTSFRTEKDAFGNANSARGFPVVDRIKAAVERACPRTVSCADVLTIAAQQSVNL 120
C1C_transcriptome     SILLDNTTSFRTEKDAFGNANSARGFPVVDRIKAAVERACPRTVSCADVLTIAAQQSVNL 120
                      ************************************************************

C1C_uniprot           AGGPSWRVPLGRRDSRQAFLDLANANLPAPSFTLPELKAAFANVGLNRPSDLVALSGGHT 180
C1C_genbank           AGGPSWRVPLGRRDSRQAFLDLANANLPAPSFTLPELKAAFANVGLNRPSDLVALSGGHT 180
C1C_transcriptome     AGGPSWRVPLGRRDSRQAFLDLANANLPAPSFTLPELKAAFANVGLNRPSDLVALSGGHT 180
                      ************************************************************

C1C_uniprot           FGKNQCRFIMDRLYNFSNTGLPDPTLNTTYLQTLRQQCPRNGNQSVLVDFDLRTPTVFDN 240
C1C_genbank           FGKNQCRFIMDRLYNFSNTGLPDPTLNTTYLQTLRQQCPRNGNQSVLVDFDLRTPTVFDN 240
C1C_transcriptome     FGKNQCRFIMDRLYNFSNTGLPDPTLNTTYLQTLRQQCPRNGNQSVLVDFDLRTPTVFDN 240
                      ************************************************************

C1C_uniprot           KYYVNLKEQKGLIQSDQELFSSPNATDTIPLVRSYADGTQTFFNAFVEAMNRMGNITPLT 300
C1C_genbank           KYYVNLKEQKGLIQSDQELFSSPNATDTIPLVRSYADGTQTFFNAFVEAMNRMGNITPLT 300
C1C_transcriptome     KYYVNLKEQKGLIQSDQELFSSPNATDTIPLVRSYADGTQTFFNAFVEAMNRMGNITPLT 300
                      ************************************************************

C1C_uniprot           GTQGEIRLNCRVVNSNSLLHDIVEVVDFVSSM 332
C1C_genbank           GTQGEIRLNCRVVNSNSLLHDIVEVVDFVSSM 332
C1C_transcriptome     GTQGEIRLNCRVVNSNSLLHDIVEVVDFVSSM 332
                      ********************************


C2_transcriptome    MHSSSSLIKLGFLLLLLNVSLSHAQLSPSFYDKTCPQVFDIATNTIKTALRSDPRIAASI 60
C2_genbank          MHSSSSLIKLGFLLLLLNVSLSHAQLSPSFYDKTCPQVFDIATNTIKTALRSDPRIAASI 60
C2_uniprot          MHSSSSLIKLGFLLLLLNVSLSHAQLSPSFYDKTCPQVFDIATNTIKTALRSDPRIAASI 60
                    ************************************************************

C2_transcriptome    LRLHFHDCFVNGCDASILLDNTTSFRTEKDAFGNARSARGFDVIDTMKAAVEKACPKTVS 120
```

```
C2_genbank          LRLHFHDCFVNGCDASILLDNTTSFRTEKDAFGNARSARGFDVIDTMKAAVEKACPKTVS 120
C2_uniprot          LRLHFHDCFVNGCDASILLDNTTSFRTEKDAFGNARSARGFDVIDTMKAAVEKACPKTVS 120
                    ************************************************************

C2_transcriptome    CADLLAIAAQKSVVLAGGPSWKVPSGRRDSLRGFMDLANDNLPGPSSTLQVLKDKFRNVG 180
C2_genbank          CADLLAIAAQKSVVLAGGPSWKVPSGRRDSLRGFMDLANDNLPGPSSTLQVLKDKFRNVG 180
C2_uniprot          CADLLAIAAQKSVVLAGGPSWKVPSGRRDSLRGFMDLANDNLPGPSSTLQVLKDKFRNVG 180
                    ************************************************************

C2_transcriptome    LDRPSDLVALSGGHTFGKNQCQFIMDRLYNFSNSGKPDPTLDKSYLSTLRKQCPRNGNLS 240
C2_genbank          LDRPSDLVALSGGHTFGKNQCQFIMDRLYNFSNSGKPDPTLDKSYLSTLRKQCPRNGNLS 240
C2_uniprot          LDRPSDLVALSGGHTFGKNQCQFIMDRLYNFSNSGKPDPTLDKSYLSTLRKQCPRNGNLS 240
                    ************************************************************

C2_transcriptome    VLVDFDLRTPTIFDNKYYVNLKENKGLIQSDQELFSSPDASDTIPLVRAYADGQGKFFDA 300
C2_genbank          VLVDFDLRTPTIFDNKYYVNLKENKGLIQSDQELFSSPDASDTIPLVRAYADGQGKFFDA 300
C2_uniprot          VLVDFDLRTPTIFDNKYYVNLKENKGLIQSDQELFSSPDASDTIPLVRAYADGQGKFFDA 300
                    ************************************************************

C2_transcriptome    FVEAMIRMGNLSPSTGKQGEIRLNCRVVNSKPKIMDVVDTNDFASSI 347
C2_genbank          FVEAMIRMGNLSPSTGKQGEIRLNCRVVNSKPKIMDVVDTNDFASSI 347
C2_uniprot          FVEAMIRMGNLSPSTGKQGEIRLNCRVVNSKPKIMDVVDTNDFASSI 347
                    ***********************************************


E5_transcriptome    MVVSPFFSCSAMGALILGCLLLQASNAQLRPDFYSRTCPSVFNIIKNVIVDELQTDPRIA 60
E5_uniprot          ---------------------------QLRPDFYSRTCPSVFNIIKNVIVDELQTDPRIA 33
                                               *********************************

E5_transcriptome    ASILRLHFHDCFVRGCDASILLDTSKSFRTEKDAAPNVNSARGFNVIDRMKTALERACPR 120
E5_uniprot          ASILRLHFHDCFVRGCDASILLDTSKSFRTEKDAAPNVNSARGFNVIDRMKTALERACPR 93
                    ************************************************************

E5_transcriptome    TVSCADILTIASQISVLLSGGPSWAVPLGRRDSVEAFFDLANTALPSPFFTLAQLKKAFA 180
E5_uniprot          TVSCADILTIASQISVLLSGGPSWAVPLGRRDSVEAFFDLANTALPSPFFTLAQLKKAFA 153
                    ************************************************************

E5_transcriptome    DVGLNRPSDLVALSGGHTFGRARCLFVTARLYNFNGTNRPDPTLNPSYLADLRRLCPRNG 240
E5_uniprot          DVGLNRPSDLVALSGGHTFGRARCLFVTARLYNFNGTNRPDPTLNPSYLADLRRLCPRNG 213
                    ************************************************************

E5_transcriptome    NGTVLVNFDVMTPNTFDNQFYTNLRNGKGLIQSDQELFSTPGADTIPLVNLYSSNTLSFF 300
E5_uniprot          NGTVLVNFDVMTPNTFDNQFYTNLRNGKGLIQSDQELFSTPGADTIPLVNLYSSNTLSFF 273
                    ************************************************************

E5_transcriptome    GAFADAMIRMGNLRPLTGTQGEIRQNCRVVNSRIRGMENDDGVVSSM 347
E5_uniprot          GAFADAMIRMGNLRPLTGTQGEIRQNCRVVNSR-------------- 306
                    *********************************


A2A_transcriptome   MAVTNLSITCDGLFIISLLVIVSSLFGTSSAQLNATFYSGTCPNASAIVRSTIQQAFQSD 60
A2_uniprot          -------------------------------QLNATFYSGTCPNASAIVRSTIQQAFQSD 29
                                                   *****************************

A2A_transcriptome   TRIGASLIRLHFHDCFVNGCDASILLDDSGSIQSEKNAGPNANSARGFNVVDNIKTALEN 120
A2_uniprot          TRIGASLIRLHFHDCFVDGCDASILLDDSGSIQSEKNAGPNANSARGFNVVDNIKTALEN 89
                    *****************:******************************************

A2A_transcriptome   TCPGVVSCSDILALASEASVSLTGGPSWTVLLGRRDSLTANLAGANSAIPSPFEGLSNIT 180
A2_uniprot          TCPGVVSCSDILALASEASVSLTGGPSWTVLLGRRDSLTANLAGANSAIPSPFEGLSNIT 149
                    ************************************************************

A2A_transcriptome   SKFSAVGLNTNDLVALSGAHTFGRARCGVFNNRLFNFSGTGNPDPTLNSTLLSSLQQLCP 240
A2_uniprot          SKFSAVGLNTNDLVALSGAHTFGRARCGVFNNRLFNFSGTNGPDPTLNSTLLSSLQQLCP 209
                    ****************************************..******************

A2A_transcriptome   QNGSASTITNLDLSTPDAFDNNYFANLQSNNGLLQSDQELFSTTGSATIAVVTSFASNQT 300
A2_uniprot          QNGSASTITNLDLSTPDAFDNNYFANLQSNNGLLQSDQELFSTLGSATIAVVTSFASNQT 269
                    ******************************************* ****************

A2A_transcriptome   LFFQAFAQSMINMGNISPLTGSNGEIRLDCKKVNGS 336
A2_uniprot          LFFQAFAQSMINMGNISPLTGSNGEIRLDCKKVDGS 305
                    *********************************:**
```

Figure 26

```
1.    5'GTAATACGACTCACTATAGGGCACGCGTGGTCGACGGCCCGGGCTGGT 3'
2.a   for XhoII, PsuI(BstYI), Bsp143I      3'-TCCCCGACCACTAG5'
2.b   for BsaWI                            3'-TCCCCGACCAGGCC5'
2.c   for HindIII                          3'-TCCCCGACCATCGA5'
```

Figure 27

```
                  *************************************************
08562.1           -------------------------------------------------GTAATGGCAAG 11
08562.4           -------------------------------------------------GTAATGGCAAG 11
contig08562       ATCACTCATCATCACAAAGTCTTCCCATTTCCTCATCTTCTTATTGAAAGTAATGGCAAG 60
contig08562_A     ATCACTCATCATCACAAAGTCTTCCCATTTCCTCATCTTCTTATTGAAAGTAATGGCAAG 60
                                                                   ***********

08562.1           ACTCACTAGCATTCTCCTTCTTCTTTCTCTTTTATGC--TTTTTCCCTCTCTGTCTCTGC 69
08562.4           ACTCACTAGCATTCTCCTTCTTCTTTCTCTTCTATGC--TTTTTCCCTCTCTGTCTCTGC 69
contig08562       ACTCACTAGCATTCTCCTTCTTCTTTCTCTTCTATGCXTTTTTTCCCTCTCTGTCTCTGC 120
contig08562_A     ACTCACTAGCATTCTCCTTCTTCTTTCTCTTCTATGCXTTTTTTCCCTCTCTGTCTCTGC 120
                   ****************************** *********** ****************

08562.1           GACAAGAGCTATGGAGGCAAACTCTTCCCTGGTTTTTACGCCCACTCATGCCCACAAGCC 129
08562.4           GACAAGAGCTATGGAGGCAAACTCTTCCCTGGTTTTTACGCCCACTCATGCCCACAAGCC 129
contig08562       GACAAGAGCTATGGAGGCAAACTCTTCCCTGGTTTTTACGCCCACTCATGCCCACAAGCC 180
contig08562_A     GACAAGAGCTATGGAGGCAAACTCTTCCCTGGTTTTTACGCCCACTCATGCCCACAAGCC 180
                  ************************************************************

08562.1           GGGGAAATCGTGAGATCAGTCGTAGCTAAAGCTGTTGCTAGAGAGACCCGTATGGCTGCT 189
08562.4           GGGGAAATCGTGAGATCAGTCGTAGCTAAAGCTGTTGCTAGAGAGACCCGTATGGCTGCT 189
contig08562       GGGGAAATCGTGAGATCAGTCGTAGCTAAAGCTGTTGCTAGAGAGACCCGTATGGCTGCT 240
contig08562_A     GCCCAAATCCTCACATCACTCCTACCTAAACCTCTTCCTACACACACCCCTATCCCTCCT 240
                  ************************************************************

08562.1           TCTTTGATGAGACTTCATTTCCACGACTGTTTCGTTCAGGTTTGGTTAATTTCTTCTACG 249
08562.4           TCTTTGATGAGACTTCATTTCCACGACTGTTTCGTTCAGGTTTGGTTAATTTCTTCTACG 249
contig08562       TCTTTGATGAGACTTCATTTCCACGACTGTTTCGTTCAG--------------------- 279
contig08562_A     TCTTTGATGAGACTTCATTTCCACGACTGTTTCGTTCAG--------------------- 279
                  ***************************************

08562.1           CCCACTATTCTAAAGATTTTTTTATTGAGCAAGGTAACTGTGAAATGCAGGGTTGTGATG 309
08562.4           CCCACTATTCTACACATTTTTTTATTCACCAACCTAACTCTCAAATCCAGCCTTCTCATC 309
contig08562       --------------------------------------------------GGTTGTGATG 289
contig08562_A     --------------------------------------------------GGTTGTGATG 289
                                                                    **********

08562.1           GCTCTTTGCTTCTAGACAGCAGTGGGAAAATAGTGAGTGAGAAAGGCTCAAACCCTAACA 369
08562.4           GCTCTTTGCTTCTAGACAGCAGTGGGAGAATAGTGAGTGAGAAAGGCTCAAACCCTAACA 369
contig08562       GCTCTTTGCTTCTAGACAGCAGTGGGAAAATAGTGAGTGAGAAAGGCTCAAACCCTAACA 349
contig08562_A     GCTCTTTGCTTCTAGACAGCAGTGGGAGAATAGTGAGTGAGAAAGGCTCAAACCCTAACA 349
                  ***************************.********************************

08562.1           GCAGATCAGCTCGTGGGTTCGACGTAGTTGACCAAATCAAAGCTGAATTGGAGAAACAAT 429
08562.4           GCAGATCAGCTCGTGGGTTCGACGTAGTTGACCAAATCAAAGCTGAATTGGAGAAACAAT 429
contig08562       GCAGATCAGCTCGTGGGTTCGACGTAGTTGACCAAATCAAAGCTGAATTGGAGAAACAAT 409
contig08562_A     GCACATCACCTCCTCGCTTCCACCTACTTCACCAAATCAAACCTCAATTCCACAAACAAT 409
                  ******************************************************** ***

08562.1           GCCCTGGAACTGTTTCTTGCGCTGATGCTCTAACTCTAGCCGCTAGAGACTCCTCTGTTC 489
08562.4           GCCCTGGAACTGTTTCTTGCGCTGATGCTCTAACTCTAGCCGCTAGAGACTCCTCTGTTC 489
contig08562       GCCCTGGAACTGTTTCTTGCGCTGATGCTCTAACTCTAGCCGCTAGAGACTCCTCTGTTC 469
contig08562_A     GCCCTGGAACTGTTTCTTGCGCTGATGTTCTAACTCTAGCTGCTAGAGACTCCTCTGTTC 469
                  *************************** ************ *******************
```

```
08562.1             TTGTAAGTCCCCTCCATAGTTTCCAAATCAAATTTAAAACATCAGCTAACTCGGTGTGGT 549
08562.4             TTGTAAGTCCCCTCCATAGTTTCCAAATCAAATTTAAAACATCAGCTAACTCGGTGTGGT 549
contig08562         TT---------------------------------------------------------- 471
contig08562_A       TT---------------------------------------------------------- 471
                    **

08562.1             TTTTGTTTTAGACCGGTGGACCGAGCTGGGTGGTTTCATTAGGAAGAAGAGATTCAAGAA 609
08562.4             TTTTGTTTTAGACCGGTGGACCGAGCTGGGTGGTTTCATTAGGAAGAAGAGATTCAAGAA 609
contig08562         -----------ACCGGTGGACCGAGCTGGGTGGTTTCATTAGGAAGAAGAGATTCAAGAA 520
contig08562_A       -----------ACCGGTGGACCAAGCTGGGTCGTTCCATTGGGAAGAAGAGATTCGAGAA 520
                               ***********.******** *** ****.*************.****

08562.1             GTGCAAGCTTGAGTGGTTCGAACAACAATATCCCTGCACCAAACAACACTTTCCAGACCA 669
08562.4             GTGCAAGCTTGAGTGGTTCGAACAACAATATCCCTGCACCAAACAACACTTTCCAGACCA 669
contig08562         GTGCAAGCTTGAGTGGTTCGAACAACAATATCCCTGCACCAAACAACACTTTCCAGACCA 580
contig08562_A       GTGCAAGCTTAAGTGGTTCTAACAACAACATCCCTGCACCAAATAACACTTTCCAGACCA 580
                    **********.******** ********* ************* ****************

08562.1             TTCTATCGAAGTTTAACCGTCAAGGACTCGATGTCACCGACCTTGTTGCTCTCTCCGGTA 729
08562.4             TTCTATCGAAGTTTAACCGTCAAGGACTCGATGTCACCGACCTTGTTGCTCTCTCCGGTA 729
contig08562         TTCTATCGAAGTTTAACCGTCAAGGACTCGATGTCACCGACCTTGTTGCTCTCTCCG--- 637
contig08562_A       TTCTATCGAAGTTTAATCGTCAAGGACTCGATGTCACCGACCTTGTTGCTCTCTCTG--- 637
                    **************** ************************************** *

08562.1             AGCTTTCTTCACTTGCACGCAACACAGTTAAAAGAAACCCCATTGCCTTACTTTTTTCTC 789
08562.4             AGCTTTCTTCACTTGCACCCAACACAGTTAAAAGAAACCCCATTGCTTAACTTCTTTCTC 789
contig08562         ------------------------------------------------------------
contig08562_A       ------------------------------------------------------------

08562.1             AACCCACCACACTTCTTAACTGTTTCTCAGGGAGCCACACCATTGGATTCTCGAGATGCA 849
08562.4             AAACCGCTACACTTCTTCACTGTTTCCCAGGGAGCCACACCATTGGATTCTCCAGATGCA 849
contig08562         ------------------------------GGAGCCACACCATTGGATTCTCCAGATGCA 667
contig08562_A       ------------------------------GGAGCCACACCATTGGATTCTCGAGATGCA 667
                                                  ********************** *******

08562.1             CGAGTTTCAGACAAAGATTGTACAACCAGTCCGGAAACGGACGTCCAGACATGACATTGG 909
08562.4             CGAGTTTCAGACAAAGGTTGTACAACCAGTCCGGAAACGGACGTCCAGACATGACACTGG 909
contig08562         CGAGTTTCAGACAAAGGTTGTACAACCAGTCCGGAAACGGACGTCCAGACATGACACTGG 727
contig08562_A       CGAGTTTCAGACAAAGATTGTACAACCAGTCCGGAAACGGACGTCCAGACATGACATTGG 727
                    ****************.*************************************** ***

08562.1             AACAATCCTTCGCTGCTAACTTGCGCCAAAGGTGTCCGAGATCCGGCGGAGACCAGATTC 969
08562.4             AACAATCCTTCGCTGCTAACTTGCGCCAAAGGTGTCCGAGATCCGGCGGGGACCAGATTC 969
contig08562         AACAATCCTTCGCTGCTAACTTGCGCCAAAGGTGTCCGAGATCCGGCGGGGACCAGATTC 787
contig08562_A       AACAATCCTTCGCTGCTAACTTGCGCCAAAGGTGTCCGAGATCCGGCGGAGACCAGATTC 787
                    *************************************************.**********

08562.1             TCTCAGTGTTGGACATCATCAGCGCCGCGAAATTCGACAACAGCTACTTCAAGAACTTGA 1029
08562.4             TCTCGGTGCTGGACATCATCAGCGCCGCGAAATTCGACAACAGCTACTTCAAGAACTTGA 1029
contig08562         TCTCGGTGCTGGACATCATCAGCGCCGCGAAATTCGACAACAGCTACTTCAAGAACTTGA 847
contig08562_A       TCTCAGTGTTGGACATCATCAGCGCCGCGAAATTCGACAACAGCTACTTCAAGAACTTGA 847
                    ****.*** ***************************************************

08562.1             TAGAAAACAAGGGTTTGTTGAACTCGGACCAGGTTTTGTTCAGCAGTAATGAGAAATCTA 1089
08562.4             TAGAGAACAAGGGTTTGTTGAACTCGGACCAGGTTCTGTTCAACAGTAACGAGAAATCTA 1089
contig08562         TAGAGAACAAGGGTTTGTTGAACTCGGACCAGGTTCTGTTCAACAGTAATGAGAAATCTA 907
contig08562_A       TAGAAAACAAGGGTTTGTTGAACTCGGACCAGGTTCTGTTCAGCAGTAATGAGAAATCTA 907
                    ****.****************************** ******.****** **********

08562.1             GAGAGCTTGTGAAGAAGTATGCAGAGGACCAAGGAGAGTTTTTTGAGCAGTTTGCGGAAT 1149
08562.4             GAGAGCTTGTGAAGAAGTATGCAGAGGACCAAGGAGAGTTTTTTGAACAGTTTGCGGAAT 1149
contig08562         GAGAGCTTGTGAAGAAGTATGCAGAGGACCAAGGAGAGTTTTTTGAGCAGTTTGCGGAAT 967
contig08562_A       GAGAGCTTGTGAAGAAGTATGCAGAGGACCAAGGAGAGTTTTTTGAGCAGTTTGCGGAAT 967
                    **********************************************.*************

08562.1             CGATGATCAAGATGGAAATATATCTCCCTTGACGGGTTCGAGTGGCGAAATCAGAAAGA 1209
08562.4             CAATGATCAAGATGGAAATATATCTCCCTTGACGGGTTCGAGTGGCGAAATCAGAAAGA 1209
contig08562         CAATGATCAAGATGGAAATATTTCTCCCATGACGGGTTCGAATGGCGAAATCAGGAAGA 1027
contig08562_A       CAATGATCAAGATGGAAATATTTCTCCCATGACGGGTTCGAATGGCGAAATCAGGAAGA 1027
                    *.*******************:******:************.************.****
```

```
08562.1            ATTGCAGGAAGATAAACTCTTGAATTCTTGAAATGAGGAAAGTATTGGG----------- 1258
08562.4            ATTGCAGGAAGATAAACTCTTGAATTCTTGAAATGAGGAAAGTATTGGG----------- 1258
contig08562        ATTGCAGGAAGATAAACTCTTGAATTCTTGAAATGAGGAAAGTATTGGGGCGAAAAAAAA 1087
contig08562_A      ATTGCAGGAAGATAAACTCTTGAATTCTTGAAATGAGGAAAGTATTGGGGCGAAAAAAAA 1087
                   *************************************************
```

## Figure 28

```
C1C_with_5'UTR     MHSPSSTSFTWATLITLGCLMLHASFSNAQLTPTFYDNSCPNVSNIVRDIIINELRSDPR 60
C1B                MHSPSSTSFTWI-LITLGCLAFYASLSDAQLTPTFYDTSCPNVSNIVRDIIINELRSDPR 59
                   *********** ******* ::**:*:*********.*********************
```

## Figure 29

```
synPDI             MQFNWDIKTVASILSALTLAQASDQEAIAPEDSHVVKLTEATFESFITSNPHVLAEFFAP 60
synPDI N314H       MQFNWDIKTVASILSALTLAQASDQEAIAPEDSHVVKLTEATFESFITSNPHVLAEFFAP 60
PDI704             MQFNWNIKTVASILSALTLAQANDQEAIAPEDSHVVKLTEATFESFITSNPHVLAEFFAP 60
                   *****:****************.*************************************

synPDI             WCGHCKKLGPELVSAAEILKDNEQVKIAQIDCTEEKELCQGYEIKGYPTLKVFHGEVEVP 120
synPDI N314H       WCGHCKKLGPELVSAAEILKDNEQVKIAQIDCTEEKELCQGYEIKGYPTLKVFHGEVEVP 120
PDI704             WCGHCKKLGPELVSAAETLKDNELVQIAQIDCTEEKDLCQSYEIKGYPTLKVFHGEVEVP 120
                   ***************** ***** *:**********:***.*******************

synPDI             SDYQGQRQSQSIVSYMLKQSLPPVSEINATKDLDDTIAEAKEPVIVQVLPEDASNLESNT 180
synPDI N314H       SDYQGQRQSQSIVSYMLKQSLPPVSEINATKDLDDTIAEAKEPVIVQVLPEDASNLESNT 180
PDI704             SDYQGQRQSQSIVSYMLKQSLPPVSEVDATKDLDDTIAEAKEAVILQVLPEGASNLESNT 180
                   **************************::**************.**:*****.********

synPDI             TFYGVAGTLREKFTFVSTKSTDYAKKYTSDSTPAYLLVRPGEEPSVYSGEELDETHLVHW 240
synPDI N314H       TFYGVAGTLREKFTFVSTKSTDYAKKYTSDSTPAYLLVRPGEEPSVYSGEELDETHLVHW 240
PDI704             TFYGVAGSLRERFTFVSTKSSDYAKKYTSDSTPAYVIVRPGEEPSLYSGEELDETHLVHW 240
                   *******:***:********:**************::********:**************

synPDI             IDIESKPLFGDIDGSTFKSYAEANIPLAYYFYENEEQRAAAADIIKPFAKEQRGKINFVG 300
synPDI N314H       IDIESKPLFGDIDGSTFKSYAEANIPLAYYFYENEEQRAAAADIIKPFAKEQRGKINFVG 300
PDI704             IDIESKPLFGDIDGSTFKSYAEANIPLAYYFYENEEQRAAAADIIIPFAKEQRGKINFVG 300
                   ********************************************* **************

synPDI             LDAVKFGKHAKNLNMDEEKLPLFVIHDLVSNKKFGVPQDQELTNKDVTELIEKFIAGEAE 360
synPDI N314H       LDAVKFGKHAKNLHMDEEKLPLFVIHDLVSNKKFGVPQDQELTNKDVTELIEKFIAGEAE 360
PDI704             LDAVKYGKHAKNLNMDDEKLPLFVIQDFVSNKKYGVPQDHELTNKDVTELIEKFIAGEAE 360
                   *****:*******:**:********:*:*****:*****:********************

synPDI             PIVKSEPIPEIQEEKVFKLVGKAHDEVVFDESKDVLVKYYAPWCGHCKRMAPAYEELATL 420
synPDI N314H       PIVKSEPIPEIQEEKVFKLVGKAHDEVVFDESKDVLVKYYAPWCGHCKRMAPAYEELATL 420
PDI704             PIVKSEPIPEIQEEKVFKLVGKAHDQVVFDESKDVLVKYYAPWCGHCKRMAPAYEELATL 420
                   *************************:**********************************

synPDI             YANDEDASSKVVIAKLDHTLNDVDNVDIQGYPTLILYPAGDKSNPQLYDGSRDLESLAEF 480
synPDI N314H       YANDEDASSKVVIAKLDHTLNDVDNVDIQGYPTLILYPAGDKSNPQLYDGSRDLESLAEF 480
PDI704             YANDENASSKIVIAKLDHTLNDVDNVDIQGYPTLILYPAGDKSNPQLYDGARDLESLAEF 480
                   *****:****:***************************************:*********

synPDI             VKERGTHKVDALALRPVEEEKEAEEEAESEADAHDEL 517
synPDI N314H       VKERGTHKVDALALRPVEEEKEAEEEAESEADAHDEL 517
PDI704             VKEKGTHKVDALALKPIEEEKEAEE---SEADAHDEL 514
                   ***:**********:*:********   *********
```

Europäisches Patentamt
European Patent Office
Office européen des brevets

# EUROPEAN SEARCH REPORT

Application Number
EP 11 18 5833

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KAZUHITO FUJIYAMA ET AL: "Structure of the horseradish peroxidase isozyme C genes", EUROPEAN JOURNAL OF BIOCHEMISTRY, vol. 173, no. 3, 1 May 1988 (1988-05-01), pages 681-687, XP055019896, ISSN: 0014-2956, DOI: 10.1111/j.1432-1033.1988.tb14052.x * abstract * * figure 3 * ----- | 1-15 | INV. C12N9/08 C12Q1/28 |
| Y | MORAWSKI BIRGIT ET AL: "Functional expression of horseradish peroxidase in Saccharomyces cerevisiae and Pichia pastoris", PROTEIN ENGINEERING, OXFORD UNIVERSITY PRESS, SURREY, GB, vol. 13, no. 5, 1 May 2000 (2000-05-01), pages 377-384, XP002158266, ISSN: 0269-2139, DOI: 10.1093/PROTEIN/13.5.377 * abstract * ----- | 1-15 | |
| Y | MORAWSKI B ET AL: "Functional expression and stabilization of horseradish peroxidase by directed evolution in Saccharomyces cerevisiae", BIOTECHNOLOGY AND BIOENGINEERING, WILEY & SONS, HOBOKEN, NJ, US, vol. 76, no. 2, 1 September 2001 (2001-09-01), pages 99-107, XP002478995, ISSN: 0006-3592, DOI: 10.1002/BIT.1149 * abstract * ----- -/-- | 1-15 | TECHNICAL FIELDS SEARCHED (IPC) C12N C12Q |

~~The present search report has been drawn up for all claims~~

2

| Place of search | Date of completion of the search | Examiner |
|---|---|---|
| Munich | 27 February 2012 | Behrens, Joyce |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 18 5833

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 01/72999 A1 (CALIFORNIA INST OF TECHN [US]; ARNOLD FRANCES H [US]; LIN ZHANGLIN [US]) 4 October 2001 (2001-10-04)<br>* claims 1-57 *<br>* examples 1-6 *<br>----- | 1-15 | |
| Y | SMITH A T ET AL: "EXPRESSION OF A SYNTHETIC GENE FOR HORSERADISH PEROXIDASE C IN ESCHERICHIA COLI AND FOLDING AND ACTIVATION OF THE RECOMBINANT ENZYME WITH CA2+ AND HEME",<br>JOURNAL OF BIOLOGICAL CHEMISTRY, THE AMERICAN SOCIETY OF BIOLOGICAL CHEMISTS, INC, US,<br>vol. 265, no. 22,<br>5 August 1990 (1990-08-05), pages 13335-13343, XP000801340,<br>ISSN: 0021-9258<br>* abstract *<br>----- | 1-15 | |

TECHNICAL FIELDS SEARCHED (IPC)

~~The present search report has been drawn up for all claims~~

2

| Place of search | Date of completion of the search | Examiner |
|---|---|---|
| Munich | 27 February 2012 | Behrens, Joyce |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

Europäisches Patentamt
European Patent Office
Office européen des brevets

Application Number
EP 11 18 5833

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 11 18 5833

The Search Division considers that the present European patent application does not comply with the requirements of unity of invention and relates to several inventions or groups of inventions, namely:

1. claims: 1-15(partially)

A recombinant heme-containing horseradish peroxidase isoenzyme encoded by a nucleic acid comprising the sequence corresponding to SEQ ID NO: 1.

---

2-49. claims: 1-15(partially)

A recombinant heme-containing horseradish peroxidase isoenzyme encoded by a nucleic acid comprising a sequence selected from the group consisting of SEQ ID NO: 2 to SEQ ID NO:49

---

## ANNEX TO THE EUROPEAN SEARCH REPORT ON EUROPEAN PATENT APPLICATION NO. EP 11 18 5833

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2012

| Patent document cited in search report | Publication date | Patent family member(s) | Publication date |
|---|---|---|---|
| WO 0172999 A1 | 04-10-2001 | AU 7576300 A | 08-10-2001 |
| | | WO 0172999 A1 | 04-10-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **VEITCH, N.C.** *Phytochemistry,* 2004, vol. 65, 249-259
- **PASSARDI, F. et al.** *Phytochemistry,* 2007, vol. 68, 1605-11
- **HOFRICHTER, M. et al.** *Applied microbiology and biotechnology,* 2010, vol. 87, 871-97
- **MORAWSKI et al.** *Protein engineering,* 2000, vol. 13, 377-84
- **CREGG, J. M. et al.** *Molecular Biotechnology,* 2000, vol. 16, 23-52
- **ABAD et al.** *Biotechnology journal,* 2010, vol. 5, 413-20
- **LIN-CEREGHINO et al.** *BioTechniques,* 2005, vol. 38, 44-48
- **WEIS et al.** *FEMS yeast research,* 2004, vol. 5, 179-89
- **HOFFMAN et al.** *Gene,* 1987, vol. 57, 267-272
- **HARJU et al.** *BMC biotechnology,* 2004, vol. 4, 8
- **ZULIDOV et al.** *Nucleic acid research,* 2004, vol. 32, e37
- **ABAD et al.** *Microbial cell factories,* 2010, vol. 9, 24
- *Journal of biological inorganic chemistry: JBIC: a publication of the Society of Biological Inorganic Chemistry,* vol. 6, 504-16

SEQUENCE LISTING

<110> Technische Universität Graz

<120> Horseradish Peroxidase Isoenzymes

<130> UG001EP

<160> 60

<170> PatentIn version 3.5

<210> 1
<211> 2512
<212> DNA
<213> Artificial Sequence

<220>
<223> na HRP isoenzym C1B

<400> 1
```
ttaattagtt ttctttttat ctttaaaaat atgcattccc cttcttctac ttcgtttact      60

tggatcttaa tcacattggg atgtcttgcg ttttatgcgt ctttgtccga tgctcagctt     120

acccctacct tctacgacac ttcatgtcct aatgtctcaa acatcgtacg agacatcatt     180

attaatgagc tacgatcgga ccctcgtatc accgcgagta tccttcgtct tcacttccac     240

gactgctttg ttaatgtaag ataatacttt ttcatatttc tattgcgtta tgaattattg     300

tgcgttttat cctttagata ttgataaatc acctcaagtc aaaatttaat aaaacattaa     360

ataaaatatg atacaaagag tcaatttgtt ttgtaggaat ataatagaaa tttcaacatg     420

tttttaaata tgggtccaaa atgttgaaaa cgacatttct tatgaaaaag agtgagtatg     480

tattaaatca taatttgcta taattatcgg gttgtgaaag tagttctatt cattttgaca     540

tgtagatggt tgcatagtac gttttgtcta caacattttt ttcttgattc attttacaaa     600

attacaagtt cacttgcctc cgaaaaatat gtatagcctt actatgacat aattacataa     660

tttacattca ataataattt ttattttta tataataatt tttattttta tttatataaa      720

aaagaaagat attattgttt gtggtgtcag ttgggtgaaa tcgtatccta aataaaagtc     780

actcgagtaa cggttctgat ccagattaaa aaatcagtac caaatttcac atggttagat     840

gtcgtgtgtt agattttgct gttgaataat taaatactta actctcgtcg acaatgataa     900

tgctaaaata tttatgaaat cggattcacg cccgtgttac agtattaaga gcatggtgcc     960

gtaccaaaca cgatacgaat ttaatggtga caaaaaatct ctgttaaatt gttaccggtg    1020

gtaaagagtt agctatggat gtaatacatg tactaataat ttgttaatta atattttgga    1080

tgtttgaaag ggttgtgacg catcgatatt gttagacaac acaacatcat ttctaacaga    1140

gaaagatgcg cttggaaacg caaactcggc tagaggattt cctacagttg acagaatcaa    1200

ggccgcggtg gagagggcat gcccaagaac agtttcatgc gcagatgtgc ttaccattgc    1260

agctcaacaa tctgttaatt tggtatgctc cattcattac aaacattgtt ttttaatttt    1320
```

```
aacatatttt ttagttgttt gagagcgtca caatctatat ttagtatcaa caactgttcg     1380
actatatgag gtattcatgg attaatcgag aaatattcaa aacgcgtggt cccgttaaga     1440
aaatttgaca agtttaatat cattggaaaa attaggtctc atcacaaggt tttaccttgg     1500
taggcaaagc tactttataa ttaaccaaaa aggataattt tcatttttc caaaaatagt      1560
ggagataaaa caaactccac tttgagtata tcatcaatca atttatacta catgtttatc     1620
tttttctctt tacatattga aacttccgag tgacaaatta atctcacaaa aataattatt     1680
ttgaatataa tggttactat tatattatag gcaggaggtc cttcttggag ggttcctttg     1740
ggaagaagag acagcttaca agcattttta gatcttgcta atgcaaatct tccagctcca     1800
ttctttacac ttccacaact taaggatgcc tttgcaaaag ttggcctcga ccgtccttct     1860
gatctcgttg ctctctccgg tagttaacaa aagaaaatta aacaccattt gatataagtt     1920
caattagata tttcattatt gatcttatta tatggtcttt cttttgttag gtggtcacac     1980
atttggaaaa aatcagtgta gatttattat ggacagatta tacaacttca gcaacaccgg     2040
actacccgac cctaccctca acactactta ccttcaaact cttcgtcaac aatgtcccct     2100
aaatggaaac caaagtgtat tggtggattt cgatctgcgt acgccaacgg ttttcgataa     2160
caaatactat gtgaatctta aagagcaaaa aggtctcatt cagagtgacc aagagttgtt     2220
ctctagcccc aatgccactg acacaatccc cttggtgagg tcatttgctg atggcacaca     2280
aaaattcttc aatgcgtttg tggaggctat gaataggatg ggaaatatta cacctcttac     2340
aggaactcaa ggagaaatca ggttgaattg tagggtggtg aactccaact ctctactcca     2400
tgatatagtg gaggtcgttg actttgttag ctctatgtga gaaaagttga gtcaatatct     2460
ggctaccaga gtacacgtta agataaataa agcgctctca agatgttact tg             2512
```

<210> 2
<211> 2625
<212> DNA
<213> Artificial Sequence

<220>
<223> na HRP isoenzym C1C

<400> 2

```
ttttttttt ctcttaaaaa atgcattccc cttcttctac ttcgtttact tgggcaacct        60
taatcacatt gggatgtctt atgcttcatg catcttttc caatgctcaa cttacccta        120
ccttctacga caattcatgt cctaacgtct caaacatagt acgggacatc attatcaatg      180
agttacgatc ggaccctcgt atcgccgcga gcatccttcg tcttcacttc cacgactgct      240
ttgttaatgt aagatattac ttttcatatt tctattgcgt tgtgaattat tgtgttttat      300
cttttttga agatatgtgt tttatctttt agatattgat atatcacacc tctagtcaaa       360
atttaataaa acataaaata aaatataata caaagaggag tataattaat aaaaaattta      420
acatgttttt aatctggctc aaaaatgtcg aaacgagatt tcttatgaaa aagagtaagt      480
```

atgtattaaa tcataatttg ctacaattat tgggtggtaa aagtaaggaa ttctattcat 540
tttgaaattt agctggttgc atagctttat tctacaacat ttttactgga ttcattttat 600
aaagttataa tttcaccttt tttttttgtt aaatagttat aatttcactt gccttcggaa 660
aatatgtata gccttactat aacataatta cattcaataa taatttttat tttatttata 720
taaaaagatt atattatttt ttgttttgtt cagttagagt gaaccgtatc ctaaataaaa 780
gtcactcgag taacgggtct gatccagaca aaaaaaaaaa attcaatacc aaatttcaca 840
tggttagatg tcgtatgttt aattttgctg gtgaataatt aatacttaac tctcgtcgac 900
aatgataatg ccaaaacatt tatgaaatcg gattcatgaa ttgtccgtgt tacagtatta 960
agagcatggt gccgtacccc aaacacgata tgaatttaat ggatgacaaa aaaaaaattc 1020
attgttaaat tgttaccggt ggtaaagatt tagctatgga tgtaatacat ttactaataa 1080
tttgttaatt aatattttgg atttttgata gggttgtgac gcatcgatct tgttagacaa 1140
cacaacatca tttcgaacag agaaagatgc gtttggaaac gcaaactcgg ctcgaggatt 1200
tcctgtggtt gacagaatca aggccgcggt ggagagggca tgcccaagaa ctgtttcatg 1260
cgcagatgtg cttaccattg cagctcaaca atctgttaat ttggtatgtt ccaataactt 1320
ctgtcattac aaacattgtt tttaattttt tttttttttt gttaaaatca ttgttttaaa 1380
ttttaatata tcaattcctc acttttacat atatcaaacg tacaacgata gcctaagttt 1440
gaaaagaaaa gtattcaaaa cgtcacaatc tatatattta gcatcaacaa cttttcaact 1500
atatatatga ggtatgtatg gattaatcta gaaatattca aaacgcgtgg tcgcgttatt 1560
tgacaagttt aataatatca ttgaaaaagt aggtatcatc acaagaattt accttgtaat 1620
aggcaaagcc tacctttata gttaaccaaa aagaataatt ttcatttttt tccaaaaatt 1680
agtggagata aaacaaagct ccactacttt gagtatatat tatcaatcaa tttatactac 1740
ttgtttatct ttttctcttt acatattgaa acttccgagt gacaaaatta atctcaaaaa 1800
aaataattat tttgaatata atggttatta tataggcagg aggtccttct tggagggttc 1860
ctttgggaag aagagacagc cgacaagcat ttttagatct cgctaatacg aatcttccag 1920
ctccatcctt cacacttcca caacttaagg ctgcttttgc aaatgttggc ctcaaccgtc 1980
cttctgatct cgttgctctc tctggtaatt aacaaaagaa aactaaacac catttggtat 2040
agtttaatga gcgatttcat tattaatctt attatggtct ttcttttgtt aggtggtcac 2100
acatttggta aaaatcaatg tcgatttatt atggacagat tatacaactt cagcaacacc 2160
ggactacccg accctaccct caacactact taccttcaaa ctcttcgtca acaatgtccc 2220
cgtaatggta accaaagcgt cttggtggat ttcgatctgc gtacgccaac agttttcgat 2280
aacaaatact atgtgaatct taaagagcaa aaaggtctca tccagagtga ccaagagttg 2340
ttctctagcc ccaatgccac tgacacaatc cccttggtga gatcatatgc tgatggcaca 2400

caaacattct tcaatgcctt tgtggaggcc atgaatagga tgggaaacat tacacctctt 2460

acaggaactc aaggagaaat caggttgaac tgtagggtgg tgaactccaa ctctctactc 2520

catgatatag tggaggtcgt tgactttgtt agctctatgt gagaaaagtt gactcaatat 2580

ctggctacca gagtatacgt taagataaat aaagcgctct caaga 2625

<210> 3
<211> 2631
<212> DNA
<213> Artificial Sequence

<220>
<223> na HRP isoenzym C2

<400> 3
aaacctaacc aaagaatttt atcttagaga gcaaagaaaa tgcattcctc ttccagtttg 60

ataaaattgg gatttcttct tcttcttctt aatgtatcat tgtctcacgc tcaactaagc 120

ccttcatttt acgataaaac atgtccacaa gtctttgaca ttgcaaccaa taccattaaa 180

actgcgctga gatcagaccc tcgcatcgct gcgagcatcc ttcgtcttca tttccacgac 240

tgctttgtta atgtaagata ccactagatt caatattttc acgtatatat taattaaggc 300

caaatgactt tttattaacg ctagtaaaaa acctgtttaa gtacttcaac tgtaaactac 360

catcttaaat ctagctaatc tatatgggtt acaatatttt tgtacacaag attatattag 420

atattatata aaattaaaat tattcatatg gtgtctaata cttgtcgccg ttcaccaata 480

tatccataaa ttattgtcct gtcgcagaaa aaaatacaaa agagttgaaa tatactaaaa 540

atataatctt actagagatg agtatttatc atagatttgt tgtcgttttc ggatatgatt 600

gattaagaaa ttttattata tagaatgtct tttggttccc tgatatatca caatcacaag 660

cttgatatta ggatatcaaa aagtgataga cattttgatt tgtggttcga ctcaaattgt 720

tttttttgct taaaatcgac tcaaattgtt cgtttctcgg tttattcatt tcaaatagaa 780

tatactcaat aaagtctcaa actgtataaa agtttgtgtt agttttggtt attgaaatgt 840

tgggtttctt ttggttcaga ttaaaacaaa agaaaacacc ggagttaaat aaaaatagaa 900

ataacaaaat aaaattatta tatcaacaaa aaatatgtct tttagttaaa ttattttata 960

agaacttgat tcgacttatt ttgactaatt ttggttcaat ttggaggggc agtatttata 1020

aatggaccaa tcgcttctta agcaaattaa tatgatttaa atagtacaac gtagattgct 1080

ttcttatctt aactctcaag tttcagattc tgtcgtcaaa gtatagtcaa agatgattag 1140

ttaataatta gcgaactcat ggctgtttca attaactcgg caaaaacaaa aacaagtatt 1200

ggacttttga tggattttta tttgggagat caaatcagtt tttttgtcca ccatcgaaat 1260

ttgaactcta aattttcaaa atctctctct tttttttttt ttttttgtta aacctcttta 1320

taattttttt ttctcacagc aaatcattat ttactgatta gatagctcag gaataggatg 1380

ggaaacacaa attcttgcac tgtttacgga tgttaagatg ttttgattta actatgaata 1440

tggtagttga ctaaatgtga agaactatat ttaattttga aacagggatg tgatgcatcg 1500

atattgctag acaacactac atcatttagg actgagaaag atgcgtttgg gaacgcaaga 1560

tcagctcgag gcttcgacgt aatcgacaca atgaaggctg ccgtggagaa agcatgtcct 1620

aaaaccgttt catgtgctga tttgctcgcc attgcagctc aaaaatctgt cgttttggta 1680

ttcttctttt acaatgctat gcatctatac atctttattt ttctttcctt tttcatttaa 1740

atggttttca tatcatctta ttgattttaa tttttaggag aaacaagtat ttaaaccatg 1800

caaatataat tgtttacttg aaaatgaaaa taaaataggc gggaggtcct tcatggaagg 1860

ttccaagtgg aagaagagac agcttaagag ggttcatgga tctcgctaat gataaccttc 1920

caggtccatc ctctacactt caagtactta aggacaaatt cagaaatgtc ggactcgacc 1980

gtccttctga tctcgttgct ctttctggta cattatagtt aaaaacattt tcattttcat 2040

atataaccta tagttgtagt cattaataaa ctctaaatta ttatttgctt tggtttatta 2100

attcttttga tatttttctt tggttaggtg gtcacacctt tggcaaaaac caatgtcagt 2160

tcataatgga tcggctttac aacttcagta actccggtaa acccgaccca accttgata 2220

aatcgtacct cagcacgcta agaaaacaat gcccacgtaa tggaaacctg agtgtattgg 2280

tagattttga tttacgtaca ccgacaatct ttgacaacaa atactatgtg aatctcaaag 2340

agaacaaagg tcttatccag agtgaccaag agttattctc tagccctgat gcttctgaca 2400

ctatcccttt ggtccgagca tacgctgatg gtcaaggaaa gtttttgat gcattcgtgg 2460

aggcaatgat aaggatggga aatctttcac cttcaactgg gaaacaagga gaaattagat 2520

tgaattgtag agtggtgaat tctaaaccta aaatcatgga tgtggttgat actaatgact 2580

ttgccagctc catctgaaga aatgactttc tcctaataat aaatgatcaa t 2631

<210> 4
<211> 1422
<212> DNA
<213> Artificial Sequence

<220>
<223> na HRP isoenzym A2A

<400> 4
atcatacctc taaaatcatt attttgtaaa acctaattaa tggctgtaac aaatctatct 60

actacttgtg atggtttgtt tatcatcagc cttcttgtta tcgtttcttc attgtttgga 120

acatcatctg cgcagctaaa tgcaacgttt tactccggga cttgccctaa cgcatctgcc 180

atcgtacgca gcactattca gcaagctttt caatccgata caagaatcgg agccagcctc 240

atccgccttc attttcacga ctgctttgtt aatgtatact aatcttccca atgcagctct 300

ttacataaag gcttcttgat atttttcgct ctaaaccgct actttgcttc tttatttttt 360

caaagggttg tgatgcgtcg atcttgcttg acgacagtgg aagcatccag agcgagaaga 420

acgctggtcc gaatgcaaac tcagctagag gattcaatgt tgtcgataac atcaagactg 480

cccttgaaaa cacttgccct ggtgttgtct cttgctctga cattttagcc cttgcctcag 540

aggcttccgt gtctttggta attagtaatt acactttctt tgtgaacata tgaaacaaaa 600

cataactaaa aatttcaaat ttgctcttaa tttcttgtta tatatatata atttgtctta 660

taaattatgt tttagtaata atatagatac gtatatgttc atatatatgt ttgatcatct 720

tcagacagga gggccatcat ggactgtatt attaggaaga cgagatagtc tcaccgcaaa 780

cctcgccggg gcaaattcgg ctattccttc tcccttcgaa ggccttagca atatcacatc 840

taaattttcg gctgtcgggc taaacacgaa cgatctagta gccttatctg gtaagttcat 900

ctacatgttt agttacttgc ggttcaagtt aattcaaaac cctgacgtca tctcttgtct 960

acgtaggtgc gcatacgttc gggcgtgctc gatgtggagt gttcaacaac agactattta 1020

acttcagcgg gacaggaaat ccagacccga ctctaaactc aacgctactg agcagtcttc 1080

aacagctatg tcctcaaaac ggcagcgcat caaccatcac caatctcgat ctgagcacac 1140

ctgatgcgtt cgataacaat tacttcgcca accttcagag caacaatggg cttcttcagt 1200

cagaccaaga gctgttctct accacgggtt cagccacaat agcggttgtt acctcctttg 1260

caagtaacca gactctgttt tttcaggcct ttgctcagtc catgatcaac atggggaata 1320

ttagtccctt gacagggagt aatggagaga ttagattaga ctgtaagaag gttaatggaa 1380

gttgatttcc ataaagctct tgtttttcaa aaaacacata at 1422

<210> 5
<211> 1847
<212> DNA
<213> Artificial Sequence

<220>
<223> na HRP isoenzym E5

<400> 5
catagtctat catcctccta aaaattaaag agaaatggtg gtttctcctt tcttttcttg 60

cagtgctatg ggagccctaa tattgggttg ccttctgctt caagcatcta atgctcagtt 120

gaggcctgac ttctactcta ggacttgccc atctgttttc aatattatta agaatgtcat 180

cgtcgatgaa ctgcagactg atcctcgcat tgccgctagt atccttcgcc ttcactttca 240

tgactgcttt gttcgtgtaa gtacttaact tatgttttta ttattaataa aaaaaaacca 300

tgacgaattc atattggacg aaactacttt ttataccatt taatttactt ataggttaga 360

ggccaaaagg catatttgaa tcaacaagta caatcgtcat ataacgtata atatctatgg 420

tttttgtagg gttgtgatgc atcgatcctg cttgacactt ccaaatcgtt ccgaaccgaa 480

aaagatgctg ctccaaacgt aaattcggct cgagggttca atgtcataga tagaatgaaa 540

acagcacttg agagagcttg tcctagaaca gtgtcttgcg cagatattct caccatcgcc 600

tctcaaatat cagtgctttt ggtatgtaca tatacctata tatgacttat aatatcgggt 660

gaaattaata aaaatatgtt atgaaatttt gacgtcaatg ctttatatgt tatagtcggg 720

aggtccatct tgggcagttc cgttggggag gagagacagc gtagaagctt tctttgacct 780

agctaataca gctcttccct ctccattttt cactcttgct caacttaaaa aagcttttgc 840

tgacgttggt ttaaaccgcc cctcagatct agtcgctctt tctggtaaac tatatatatt 900

catgttgttt ataatataaa gtgttttata taaaatgata gctaaccaca cccactccgc 960

gatcgatgag gtctagaatt tacacactaa tttataagtt atagaattac aaaattttat 1020

tattttgtat ttattaatat gatttgcttt tggctttgaa atacatgacc aagtataaaa 1080

tgaaaaacaa aatggataat ataatcaaat aataatacta tttcagacaa aacatcatct 1140

agcacgtgat tttgaattat aagataagat ggtaaaacga caaaacatga ctattatttt 1200

tttacctttt tttttttaa ttgtcaaact gacacataca atgtatctaa accttattgt 1260

caaaatggta ggtggtcaca catttggaag agcacgatgc ctatttgtga cagctcgtct 1320

ctacaacttc aacggtacaa acagaccaga cccaactctg aacccatctt acctcgccga 1380

cctccgtcga ttgtgccctc gaaacggaaa cggcaccgtt ctggtcaact tcgatgtcat 1440

gactccgaat actttcgata atcaattcta cactaatctt agaaatggga aaggtctgat 1500

tcagagtgac caagagctct tctcgactcc aggagccgac acgatcccac tagtaaacct 1560

atacagcagc aacacgttat cgttcttcgg agcattcgct gatgcaatga ttaggatggg 1620

aaatcttaga cctttgactg gaactcaagg cgagataaga cagaattgta gggttgtgaa 1680

ttcgcgaatt aggggtatgg agaacgatga tggagttgtg agttctatgt gattatgttg 1740

ggaatatata tcatatatgg ttatgtatca aatcataaaa tgtgtgggaa catgcatgtc 1800

gactaaataa aagttctaac gagttgtgaa atgactatga aattttg 1847

<210> 6
<211> 2901
<212> DNA
<213> Artificial Sequence

<220>
<223> na HRP isoenzym 01805

<400> 6
cttaaaccaa taaaagataa gtttcctctt accaaaaatg catttctcta cttcttcttc 60

ttccttgtct acttggacaa ccctaataac attggggtgt cttatgcttc attcatttaa 120

gtccagtgct caactaaccc ctacctttta cgacagtacc tgccccagcg tctttagcat 180

cgtacgggac accatcgtga acgagctaag atcagatcct cgaattgctg caagtatcct 240

tcgtcttcac ttccacgact gcttcgttaa tgtaagatat tatttttcag tttaattgat 300

gattttgtgg atacgctagt atcttttata attgaataaa agaattattt tatctaattt 360

tcacacatat aattgatata tttggtacta atataagttc ttcaaaaaca atttcaacga 420

```
tttgtgttgt caacttagtt gcaattcgga tcgacttgta gttaacgtat ttgttttctg      480
aaataagttt tttgggtggg gataatggcg ccactcaaga aaaaccagtt atttctttgt      540
aagttttaat tgttgagaaa gacaacaaaa aaaaacgcgt ggatacggca cttacattta      600
ttaaactaaa ggtttgtcag aattaattaa ttatagtaat agttgtttct gtggtattag      660
tttttttgtt tgttaaagcg tttctgtggt attagttacc atgacatgtt atcatcaata      720
caaaagttca cgtagttaga tgtaatattt ttggttttta ctgtcgagta tttttcttct      780
tgtcgtcgac tcatgacata aaataatgat tccaaaacat ctattaaact ttgacctccc      840
attcggcgaa catgaattat tcgtacaata atttaataaa agcatggacc cgtacaaagc      900
acgttatgaa aatttaaatc agtccctttt tttagcataa aaataatttt ttttgtgaaa      960
tcattgaaaa gtctcaagct tatcctcaac cccaaataac ctacagatat gaaaaagaaa     1020
cagaaatgta cgtcaaaacc tactatataa tttaatttat tatttttggg attttcaact     1080
ctcgaatctg atgtagttgt tagcatactt tatttgaact tttaatgttt ttattcgaaa     1140
acatagtata tacccagacc cagatattat agccaaataa tgtagaccaa caactttttg     1200
aattttgtgt gaaaagattt tagatttata tgtaaactaa tctgtttata attatgtata     1260
tattatcatg ccttgttgaa gaggaacaaa tggtttcaag gaatgaaaat gcaatctaac     1320
aatgtttata ggtagtaatt aaagatttga ttatgatttc aacttatttc tttgcaaaag     1380
ggctgtgatg catcgatttt gttagacaac acaacatcat ttcgaacaga gaaagatgca     1440
gctccaaacg caaactcagc tcgaggattt ccagtgattg atacaatgaa agctgcagtg     1500
gaaagagcat gtccaagaac cgtatcatgc gcagatttgc ttaccatcgc agctcaacaa     1560
tctgtgaatt tggtatgcaa cattaattta tctctttttt ttagttttaa ttttatctat     1620
tttttctcca cttcttgcaa acatggtctc aaattccttg ttgaagccgt aactgaaccc     1680
gttaaacatg actacatagc attgaaaaat gagatggact gttgcatgtt ttataattta     1740
gatttaatat taccattctt aaatattaat taccatactt gtcttgaaaa ttcattgtaa     1800
gaaataatca aaaacacatg gtaataatat tgtcttgttt ttgtaaaagg gttgggtttt     1860
ctaataatat aaagtctcat tacaataata aacttgatta gataaaaact cttttctgaa     1920
ttaactaaag tgaatataag caatatttgt aaaagaactg ggaaaataac agtacaattg     1980
agaacctaat tttgggattt atattttata tatccttttt aataattatg actttgacgt     2040
ggtaagaatt attttcatct ataaaatcat tattttaaga tatgggtatt ttaggcagga     2100
ggtccttctt ggagggttcc tttggggaga agagacagcg tacaagcatt ttttgatctt     2160
gccaatacaa atcttcccgc tccattcttc acgcttccac aacttaaggc cagctttagt     2220
aatgttggac ttgaccgtcc agaagatctc gttgcactct ctggtaatta tgaggagtaa     2280
tagtaacaac caaaactttt atttgatcta attagtttat aaaatattat taatttatca     2340
tcttttgatt aggtggtcac acatttggta aaaaccaatg ccaatttatt atggacagac     2400
```

tatacaactt tagtaacact ggtttacccg accctactct caacactact tatctccaga 2460

cacttcgtgt acaatgtccc cgtaatggta accagtccgt cttggtcgat ttcgatctac 2520

gcacaccgac agtttttgac aacaaatact atgtgaatct gaaagagcac aagggactta 2580

tccagaccga tcaagagttg ttctccagcc ctaatgccgc tgatacaatc cccttggtaa 2640

gatcatatgc tgatggcact cagaagttct tcaatgcttt tatggaggcc atgaacagaa 2700

tgggaaacat taccccctctc actggaactc aaggacagat caggcaaaat tgtagggtga 2760

tcaactccaa ctcgctgctc catgatattg ttgaaatcgt tgactttgtg agctctatgt 2820

aacaatagtt gtctcaatat atgtggcaac caaaattata tgttcttatg aaaataaaat 2880

gttctcgaaa cattacttaa g 2901

<210> 7
<211> 1819
<212> DNA
<213> Artificial Sequence

<220>
<223> na HRP isoenzym 22684

<400> 7
aaaatggggt tttctccttc attttcttcc agttctatag gagtcctaat attgggttgc 60

cttctgcttc aagcttcaaa ctctaatgct aagttgaggc ctgacttcta cttaaagaca 120

tgtccatcag ttttccaaat cattgggaat gtcatcgtcg atgaactgca gagtgatcct 180

cgtattgcag ctagtctcct tcgccttcac ttccatgact gttttgttcg tgtaaggact 240

taattactca acttatcttt ttatccgaaa aagaaaaaaa catgacgtgt tcaatggaca 300

aaatgacttt tcaatacgga agtagaggct aaaagcaatt tttaattaat aaatacaatc 360

ttcatgtatt ataatatggt tttgtagggt tgtgatgcat cggtcctgct cgacaattcc 420

acatcatttc agtccgagaa agatgctgct ccaaacgcaa attcggctcg agggttcgac 480

gtcgtagata gaatgaaagc agcccttgag aaagcttgtc ctggaacagt gtcttgtgca 540

gatgttcttg ccatctccgc tcaaatatca gtgcttttgg tatgtacata tacctatata 600

tgacttatat cgggtgaaac taatcaaaat atattatgaa attttgacgt tatgttatat 660

attatatagt cgggaggccc atggtggccg gttttgttgg ggaggagaga cggcgtagaa 720

gctttcttcg atttggctaa tacagctctt cccaatccat ttgcccctct tactgaactt 780

aaagaaaaat ttgctgacgt tggcctaaag cgcgcctcag atctagttgc tctttccggt 840

aaaattttca tatttttcaa tctttcttgt tttggtcaac catattgttt caatctatat 900

aacctactct atgtattatt gtttttttta taatctaact agatataatt cattttatct 960

cgataactag gtagatatcg agttagtctg gaattaactt agtaaagtta tcttctaggt 1020

agatatcgag ttagtctgga attaagatca aatttgcaat gtcaacacaa gaaaagtgac 1080

ttgaaattaa agatgagttg gtcaaacgac atgacatgag tcatctttaa taaagttaaa 1140

catatataca atgtatctaa actttacttt tttttgtggg gtcaaaatgg aaggtgctca 1200

cacatttgga agagcacaat gtctacttgt gacacctcgt ctctacaact tcagcggcac 1260

caataaacca gacccaactc tgaacccatc ttacctcgtc gaactccgtc gattgtgccc 1320

tcaaaacgga aacggcaccg ttctgctcaa cttcgatctc gtgactccaa atgctttcga 1380

tcgtcaatac tacaccaatc ttcgaaatgg gaaaggtctg attcagagtg accaagagct 1440

cttctcgact ccaggagccg acacgatccc actagtaaac ctatacagca agaacacgtt 1500

cgcgttcttc ggtgcattcg ttgacgcaat aattaggatg ggaaatattc aacctttgac 1560

tggaactcaa ggcgagataa gacagaattg tagggttgtg aattcgcgaa ttaggggtat 1620

ggagaacgat gatggagttg tgagttctat ttgattatgt tgggaatatg gttatgtaac 1680

aaatcataaa atgtgtggga acatgcatgt cgactaaata aaagctctca cgagttatga 1740

cttgtgagat tacaactgaa aaaaccaaag gaaagaaaag atcagatttt ggatcaccag 1800

cccgggccgt cgaccacgc 1819

<210> 8
<211> 1798
<212> DNA
<213> Artificial Sequence

<220>
<223> na HRP isoenzym 01350

<400> 8
caactccaac tccaagtcta ttcaaagtct ttgtttaacc taaacatggc ttcaaatcaa 60

cgtatttcca ttctagttct cgtagttaca tttttagtgc aaggtaatta caataacgtc 120

gttgaagcac aactgacgcc caatttctac tcaacctctt gccctaacct cctctccacc 180

gtccaatccg ccgttaagtc tgccgttaac agcgaggctc gaatgggtgc atctatcgta 240

cgccttttct tccacgattg cttcgtcaac gttagttttt ttttaacttt tttttttgtt 300

tttagttttc cttgcattcc aagaaactta gcgtaatgtt tttttttttt aattttcttt 360

tctaatcata gtcataactt gcaaatatat ataaaaatat agggatgcga tggttcgatt 420

ttactagatg acacatcaag cttcacggga gaacaaaatg cgaacccaaa ccgcaattcc 480

gctcgcgggt ttaatgtgat cgacaacatc aaagcagcgg tcgagaaagc atgtcccggg 540

gtcgtgtctt gtgctgatat cttagccatc gcagctagag actccgtcgt agtcgtaagc 600

tccctatgtc ctcctctctt agtccggttt tgcaaattta aaagattaat taaacgggtc 660

taaaaaatcg tctttgttct cgttgaaagc ttggagggcc taactggact gtgaaagtag 720

gaagaagaga tgcgagaacg gcgagtcaag cggcggcgaa tagcaacatt ccggcgccca 780

cttctagtct gagccaactc attagtagtt tcagtgccgt tggactctcc accagagata 840

tggttgctct ctccggtccg tttcatctct cttcttaact caaatttttt tttttatcat 900

atatgcaaat atttgtttca tagattaagg ctgattgcaa ttgttaacgg ttactaatat 960

atacattact aaaattggtg acaataaatt tcttttgtta atgtgtaatt gctttcacta 1020

atttgattta caaaaaagta aaaaacaaaa tcacaaaaat aaatattcaa aggactaaga 1080

gtcaacagaa tcgacataaa aataaactaa tatgattgtg tcgtttgatt tttgtcgact 1140

ctccccctt aagttcttgt ttctttgtga ttcttttttc aacacatttt ttgtaacgca 1200

ccaccgactt ttctgtttct taaccaaagt tacgtaatca tatagattac tgttacgcga 1260

caccgacaaa acaattgact gattaatcaa caaataatta agtccactat taaactaatt 1320

gctatatgtt cattttttc aggcgcgcac acgatcgggc aatcccgttg cacgagcttc 1380

cgaacgagaa tctataacga gacaaacatc aacgccgcat tcgccacaac acgtcaacga 1440

acttgcccta gaacctccgg ctccggcgac gggaatttag ctccacttga cgtcaccacg 1500

gcggcttctt tcgacaacaa ctatttcaag aatctcatga ctcaaagagg tcttctccat 1560

tccgaccaag agctcttcaa cggcggctcc actgactcca tagtccgtgg atacagcaac 1620

aatccgtcaa gctttagctc cgattttgcg gcggcgatga ttaaaatggg tgatattagc 1680

cccttgaccg gtagtagcgg tgagatccgg aaggtttgcg ggaggaccaa ctgatatttc 1740

tttttcccta ttggaatttg acttttgtta gttgattcgg tgagaataag atttgatc 1798

<210> 9
<211> 1485
<212> DNA
<213> Artificial Sequence

<220>
<223> na HRP isoenzym 02021

<400> 9
aagagtattg agaaacaaga tcgaggaaac taatcaatga ggacgatgaa gcgattgaac 60

gtggcggtgg cggttgcggt tacagcgacg gttcttatgg gaatgttagg atcatcagag 120

gctcagcttc aaatgaattt ctacgcgaag agctgtccaa acgcagagaa aataatttca 180

gatcatattc aaaagcatat ccctagtggt ccttctcttg cagctcctct catcagaatg 240

cacttccatg attgcttcgt cagggtattt aatctctaat ctatctacat atatagttgc 300

aagtgtttag atatattcga cttttatgta acatatgtag gaaattagta ttcacacatc 360

cagttcaata aatgatggga tagtccagaa gatgtactaa tgtatatttt aaaaaaatgg 420

taatttgata gtgaacatga ggtagatcta gaatatttga tatttattgc attttttaaa 480

tattgacaat gtttttgaaa aaaaaaacat aataatctag ggatgtgatg gatcggtgtt 540

gataaattcg acatcaggga acgcagagaa agattcagca ccaaatctaa cacttagagg 600

cttcggtttc gtagagagga ttaagactct tcttgaagca gagtgtccta agactgtttc 660

ttgcgccgac atcatcgcac tgaccgctag agacgcagtt gttgccaccg taagtaaaca 720

aattataact tcaagactca aaacattatt taatctaatt aatcgaaatt ataatctaat 780

ttttttaat agggaggtcc ttcatggaaa gttccgacag gaagaagaga cggtaggatc 840

tcaaatacga cggaggcttt gaacaacatt ccaccgccga cgagtaattt cacgacgtta 900

cagcgacttt tcgctaatca aggccttaat ctcaaagacc ttgttctgct ttccggtaag 960

tttagtaacc ggaaataacc agattgaatt taacaaccta acggtgttta actttttgtt 1020

gttgttgttg ttgtttagga gctcacacga tcggtgtctc gcattgttct tccatgaata 1080

ctcgtctcta caacttctcg acgacagtca aacaagatcc atctctggat agcgagtacg 1140

cagcaaatct aaaggctaac aaatgtaaga gtcttaacga taacaccacc atcctcgaga 1200

tggatcctgg tagtagcaaa acctttgatc tcagttatta taggcttgtc ttgaagagga 1260

gaggtttgtt tcagtctgat tctgccttaa cgacaaactc agctacgttg aagatgatca 1320

acgacttggt caacggtcct gaaaagaagt ttttaaaggc tttcgctaag tcaatggaga 1380

agatggggag agttaaagtg aagacgggct cagccggtgt gattaggaca cgttgttctg 1440

ttgccggaag ttagttagtt tggtcggaaa gtgatgtttt ctgtt 1485

<210> 10
<211> 1085
<212> DNA
<213> Artificial Sequence

<220>
<223> na HRP isoenzym 04791

<400> 10
tctcactttc tctcttccgc tacaacaatg gcggaactca aatctctctc cctcatcctc 60

ctcttcacac tcctcaccac caccatcgaa tctcgtttaa ccacaaactt ctactcaaaa 120

tcatgtccaa gattcttcga catagtcaga gatacaatct caaacaaaca aatcacaaca 180

ccaaccacgg cagccgccac aatccgtctc ttcttccacg actgtttccc caacggctgc 240

gacgcctcaa tcctaatctc ctcaactgcc ttcaacaccg cagaacgtga ctcatcaatc 300

aatctctcac ttcccggcga cggctttgac gtcatagtcc gagctaaaac cgcaatcgaa 360

ctcgcttgtc ccaacactgt ttcttgctcc gatataatca ccgtcgctac tcgtgacctt 420

cttgtcaccg tcggtggtcc ttactacgac gtttacctcg gccgtcgtga ttcaagaata 480

tctaaatcat ctcttttaac cgatcttctt cctcttcctt catctccgat ctcaaaaacc 540

attcgtcagt ttgaatctaa aggtttcact attcaagaaa tggttgctct tagtggggcc 600

cactcaatcg ggttttcaca ttgtaaagag tttgttaatc gggtcgccgg taataatacc 660

gggtataacc cgagatttgc tcaggcgttg aagcaagctt gttctaatta cccgaaagat 720

ccgacgttat ctgtgtttaa tgatattatg actccgaata ggtttgataa tatgtattat 780

cagaatattc caaagggtct tgggttactt gaatcggatc atgggttata ttctgacccg 840

agaacccgac cttttgttga tctttatgct agagatcaag atttgttctt taaagatttt 900

gctagagcta tgcagaagtt gagtctcttt ggtgttaaga ctggtcgacg aggagagatc 960

cgacgaaggt gcgatgcgat taactgagtt tatgtttttt cttttatgta tttatttttt 1020

ggattatatt tggggaagaa atgtgagatt tgatgaattg tgaatcttct gagttttttt 1080

ttttt 1085

<210> 11
<211> 1373
<212> DNA
<213> Artificial Sequence

<220>
<223> na HRP isoenzym 06117

<400> 11
cttcttcttt cttctggatc tagtggaaac tgacattatg gcaagaattg gaagctttct 60

cgttgttatc tctctcgctt gcgttcttac tctctgcatc tgcgacgacg agagtaatta 120

tggcggccaa gggaaactct tcccaggttt ctacagcagc tcgtgcccta aagctgagga 180

gatcgtgagg tctgttgtag ccaaagctgt tgcaagagag actcgtatgg ctgcttctct 240

catgaggctc cattttcacg actgttttgt tcaggtactc aagagctatt ttaagtaact 300

ttctcatcaa gctaacgact aattttgaga attttaatta attatagctg tttgcataaa 360

aatgtgtagg gttgtgatgg atcgttgctt ctagacagca gtggaagtat agttactgag 420

aagaactcta accctaacag cagatcagct cgtgggtttg aagttgttga cgagatcaaa 480

gctgcattgg agaatgaatg ccctaacact gtttcttgcg ctgacgccct aactctagcc 540

gctagagact cctctgttct tgtaagtcgc cttattccac tttctctctt taccgcttcc 600

ttaagttctc aagagatcta attttgttcg gtttgaatat agactggtgg accaagctgg 660

atggttcctt tgggaagaag agattcgaca agtgcaagct tgagtggatc aaataacaac 720

attcctgcac caaacaacac tttcaacaca attctctcga gatttaacag ccaaggtctc 780

gatctcacca atgtcgttgc tctctccggt aagcttactt aaaacacaag gaaaatttta 840

ctttccttgc tactcaagtt aaagttaaat cctaaaaagg attttagtat aacctgactc 900

aattgtttct cagggagcca cacaattgga ttctcaagat gtactagttt tagacagaga 960

ctttacaacc aatccggaaa cggaagtccc gacacaacct tagagcaatc ctacgctgct 1020

aacttgcgcc atcggtgccc tagatcaggt ggggaccaga acctgtcgga gcttgacatc 1080

aacagtgctg gaaggtttga taacagctac tttaagaatt tgatcgagaa catgggactg 1140

ttgaattccg accaggtctt gttctctagc aacgacgaat cgagagagct agtgaagaag 1200

tatgcagagg atcaagaaga gttcttcgag cagttcgcgg aatcgatggt caagatgggg 1260

aatatctctc ccttgactgg ttcaagtggt caaatcagga agaattgcag gaagattaac 1320

tcttgatttc ataatattga attgggcgaa ataaaaatga gagatgtttt ggg 1373

<210> 12
<211> 1405
<212> DNA
<213> Artificial Sequence

<220>
<223> na HRP isoenzym 17517

<400> 12
caacaacaac ttttacaaag ctcaaagagt ttcttattta ccaaaacaaa aacaaaatgg 60

gtcgtggtta taatttacta ttaattctag ttacgttttt agtattggtc gcagctgtaa 120

ccgctcgaag accacgagtt gggttttatg ggaatagatg ccgaaaggta gagtctatcg 180

tgagatcggt ggttcgatct catttccggt gtaatccggc aaatgcaccg ggaattttgc 240

gtatgtattt tcacgattgt tttgtcaatg gctgcgatgg ctcgatcctc ctcgctggta 300

acacttcgga gagaactgcg ggtcctaacc gttcattgag agggttcgaa gctattgaag 360

aagctaagac tcggcttgag aatgcttgtc ctaataccgt ttcttgtgcg gatatcctca 420

cccttgctgc tcgagacgcc gtcgtttggg taaaacatat tgaaattagt ttcagtttta 480

agaaatttta attatatatt gtgtgtttaa tggtaatata attttgggtg atgcagaccg 540

gtggaaaagg ttggtcggtg ccattgggac gtcttgacgg ccgaagatca gaagcctcag 600

atgtaaattt gcccggacca agcgaccccg tcgctaagca gaagcaagac tttgcagcta 660

aaaatctcaa caccttggac ctcgtaactc ttgttggttc gttgtaatta tatataatca 720

aacaatgtat attaatcaaa tagttacata tttggtcggt tgtattaaat tttcgataca 780

gttctgatta cgtcggtatg tatgaggtta tgaaatattt tttttcttga tttaagttta 840

gaaatttagt ttcaatttga accgaattcg gtttacatgt tcaattttat tagctaatat 900

tgcaaattga tattgatcag gtggacacac aattggaact gctggttgcg gtttggtaag 960

aggcagattc tttaacttca atggcacggg acaacctgac ccatcaatcg acccgagttt 1020

cgttcctcta gttcaggctc gttgccctca aaacggtaac gcaacgaccc gagtcgactt 1080

agacactgga agtgcaggtg atttcgatac atcgtaccta agtaacgtga ggtcaagccg 1140

cgtggttctc caatccgatc tagtcctgtg gaaggacacc gaaaccagag ccatcataga 1200

acgtttatta ggcttacgcc ggcccgtttt gaggttcgga tcagaatttg ggaagtcgat 1260

gaccaagatg agtctcatag aagttaaaac tagactatca gatggggaga ttcgtagggt 1320

ttgctctgcg atcaattaag tattaaaaac acacaaatgt ttggtttgat tttatcactt 1380

attttatgga ataagcttgc tatag 1405

<210> 13
<211> 1258
<212> DNA
<213> Artificial Sequence

<220>

<223> na HRP isoenzym 08562.1

<400> 13
gtaatggcaa gactcactag cattctcctt cttctttctc ttttatgctt tttccctctc 60

tgtctctgcg acaagagcta tggaggcaaa ctcttccctg gtttttacgc ccactcatgc 120

ccacaagccg gggaaatcgt gagatcagtc gtagctaaag ctgttgctag agagacccgt 180

atggctgctt ctttgatgag acttcatttc cacgactgtt tcgttcaggt ttggttaatt 240

tcttctacgc ccactattct aaagattttt ttattgagca aggtaactgt gaaatgcagg 300

gttgtgatgg ctctttgctt ctagacagca gtgggaaaat agtgagtgag aaaggctcaa 360

accctaacag cagatcagct cgtgggttcg acgtagttga ccaaatcaaa gctgaattgg 420

agaaacaatg ccctggaact gtttcttgcg ctgatgctct aactctagcc gctagagact 480

cctctgttct tgtaagtccc ctccatagtt tccaaatcaa atttaaaaca tcagctaact 540

cggtgtggtt tttgttttag accggtggac cgagctgggt ggtttcatta ggaagaagag 600

attcaagaag tgcaagcttg agtggttcga acaacaatat ccctgcacca aacaacactt 660

tccagaccat tctatcgaag tttaaccgtc aaggactcga tgtcaccgac cttgttgctc 720

tctccggtaa gctttcttca cttgcacgca acacagttaa aagaaacccc attgccttac 780

ttttttctca acccaccaca cttcttaact gtttctcagg gagccacacc attggattct 840

cgagatgcac gagtttcaga caaagattgt acaaccagtc cggaaacgga cgtccagaca 900

tgacattgga acaatccttc gctgctaact tgcgccaaag gtgtccgaga tccggcggag 960

accagattct ctcagtgttg gacatcatca gcgccgcgaa attcgacaac agctacttca 1020

agaacttgat agaaaacaag ggtttgttga actcggacca ggttttgttc agcagtaatg 1080

agaaatctag agagcttgtg aagaagtatg cagaggacca aggagagttt tttgagcagt 1140

ttgcggaatc gatgatcaag atgggaaata tatctccctt gacgggttcg agtggcgaaa 1200

tcagaaagaa ttgcaggaag ataaactctt gaattcttga aatgaggaaa gtattggg 1258

<210> 14
<211> 1258
<212> DNA
<213> Artificial Sequence

<220>
<223> na HRP isoenzym 08562.4

<400> 14
gtaatggcaa gactcactag cattctcctt cttctttctc ttctatgctt tttccctctc 60

tgtctctgcg acaagagcta tggaggcaaa ctcttccctg gtttttacgc ccactcatgc 120

ccacaagccg gggaaatcgt gagatcagtc gtagctaaag ctgttgctag agagacccgt 180

atggctgctt ctttgatgag acttcatttc cacgactgtt tcgttcaggt ttggttaatt 240

tcttctacgc ccactattct acagattttt ttattgagca aggtaactgt gaaatgcagg 300

gttgtgatgg ctctttgctt ctagacagca gtgggagaat agtgagtgag aaaggctcaa 360
accctaacag cagatcagct cgtgggttcg acgtagttga ccaaatcaaa gctgaattgg 420
agaaacaatg ccctggaact gtttcttgcg ctgatgctct aactctagcc gctagagact 480
cctctgttct tgtaagtccc ctccatagtt tccaaatcaa atttaaaaca tcagctaact 540
cggtgtggtt tttgttttag accggtggac cgagctgggt ggtttcatta ggaagaagag 600
attcaagaag tgcaagcttg agtggttcga acaacaatat ccctgcacca aacaacactt 660
tccagaccat tctatcgaag tttaaccgtc aaggactcga tgtcaccgac cttgttgctc 720
tctccggtaa gctttcttca cttgcacgca acacagttaa aagaaacccc attgcttaac 780
ttctttctca aaccgctaca cttcttcact gtttcccagg gagccacacc attggattct 840
ccagatgcac gagtttcaga caaaggttgt acaaccagtc cggaaacgga cgtccagaca 900
tgacactgga acaatccttc gctgctaact tgcgccaaag gtgtccgaga tccggcgggg 960
accagattct ctcggtgctg gacatcatca gcgccgcgaa attcgacaac agctacttca 1020
agaacttgat agagaacaag ggtttgttga actcggacca ggttctgttc aacagtaacg 1080
agaaatctag agagcttgtg aagaagtatg cagaggacca aggagagttt tttgaacagt 1140
ttgcggaatc aatgatcaag atgggaaata tatctccctt gacgggttcg agtggcgaaa 1200
tcagaaagaa ttgcaggaag ataaactctt gaattcttga aatgaggaaa gtattggg 1258

<210> 15
<211> 1375
<212> DNA
<213> Artificial Sequence

<220>
<223> na HRP isoenzym contig23190

<400> 15
ccctatagtg agtcgtatta cggccggggg aacaacaaga agcagagaag agagaggctt 60
cgactgaaac aacaaaaaat ggcaatgagt tattcgatac gtgtcctgac gtttctgatg 120
ttgatctcgt taatggcagt gacactgaac cttctgtcaa cggcggaagc aaagaagccg 180
aggagagatg ttcctatagt gaaaggtctc tcttggaact tttaccagag agcatgtccg 240
aaagtggaaa agattatcaa aaaagaactc aaaaaagtct tcaagagaga tattggttta 300
gccgcagcca tccttcgtat acatttccat gactgcttcg ttcaggggtg tgaagcatct 360
gtgctgctag ctggatcagc aagtggacca ggagaacaat catcgatccc gaacctaaca 420
ctccgtcaac aagcctttgt tgtcatcaat aacctgcgtg ccctcgtcca gaaacagtgt 480
ggtcaagtcg tctcttgctc cgacatcctc gctctcgccg ctcgcgattc catcgtcctt 540
tcaggagggc cagactatgc tgtgccactt ggccgacgtg actcgctagc gtttgcgacc 600
ccggaaacga cgttagctaa cttaccgcca ccgtttgcca acgcaagcca gctcatcagc 660
gacttcaacg acagaaacct caacatcacc gacttagtag cactttccgg tggtcacacc 720

atcggaattg cgcattgtcc gtctttcaca gaccggctct acccaaacca agatccaacc 780

atgaacaagt ctttcgccaa cagcctcaaa cgcacctgtc ccacggcgaa ctcgagcaac 840

acgcaagtga atgacataag gagtcctgac gtgtttgaca acaagtacta tgttgatctc 900

atgaaccgac aagggctgtt cacttccgac caggatctgt tcgttgacaa gaggacacgt 960

ggcatagtgg aaagctttgc gatcgaccag aacttgtttt ttgatcattt cacggtggca 1020

atgattaaga tgggtcagat gagtgtcttg acggggacac aaggggagat ccgttccaac 1080

tgttcagcca gaaacaccgc aagtttcata tccgttttgg tagaaggcat agtcgaggaa 1140

gctctttcca tgatctaaaa taaccataaa tctcagactt ttcttttctt taactttgtt 1200

tttatttagt tgtcgcactt gtggtttgtg gaatgcctaa gactatctca taaataagag 1260

cattgctttc atcttaattt cttcttcttt ttttttctc tagtttggtc aatgtctggg 1320

actataatga ataaagaatg ttgctacatc ttaaaaaaaa aaaaaaaaaa aaacg 1375

<210> 16
<211> 1283
<212> DNA
<213> Artificial Sequence

<220>
<223> na HRP isoenzym contig04663

<400> 16
aactaattag attaaagtat cataagttct gaatcaaaat ccgtcaaagg aaagattaat 60

caaagctcta tcattatttg caacaaactg attaatggct gcaacaagct cttctactac 120

ttgtgatggt ctcttcatca ttagccttct tgttatcgct tcttcattgt ttgggacatc 180

atctgcgcag ttaaacgcta cgttttactc cgggacgtgc cctaatgcct ctgccatcgt 240

acgcagcact atccagcaag ctcttcaatc cgacccgagg atcggagcca gcctcatccg 300

ccttcatttt cacgactgtt ttgttaatgg ctgcgacggg tcgctcttgc ttgacgacac 360

tggaagtatc cagagcgaga agaacgctcc tgccaacgca aactcagcta gaggatttaa 420

tgttgtcgac gatatcaaaa ctgccctcga gaatgcttgt cccggcattg tctcttgctc 480

tgacattcta gctcttgcct cagaggcttc cgtgtctttg gcaggaggtc cttcatggac 540

tgtgttagta ggaagaagag atggtctcac cgcaaacctg tccggggcca attcgtcgct 600

tccctctccc ttcgaaggcc ttaacaacat cacatctaaa tttttagctg tcgggctaaa 660

tacaaccgat gtagtagtct tgtctggagc tcatacgttt gggcgtggcc aatgtgtaac 720

cttcaacaat agacttttca acttcaacgg aacaggaagt cccgacccga ctctgaactc 780

aacacttctc agcagtcttc aacagatatg tcctcaaaac ggcagcggat cagcgatcac 840

caatctcgat ctgactacac ctgatgcatt tgatagcaac tactacacga accttcagag 900

taacaatggg cttcttcagt cagaccaaga actattctcc aacaccggtt cacccaccat 960

cgcgattgta atcctttgca agtaaccaaa ccctgttttt tgaggctttt gctcagtcta 1020

tgatcaagat gggtaacatt agtcccctga ctgggactag tggagagatt agacaagatt 1080

gtaaggcggt taatggacag tcatcagcca ctaaagcaga ggacattcag atgcaatctg 1140

acggaccagt gagtttagca gatatgtgaa caataatggg atcagtttca ccgtttgttt 1200

atgatacatg aataattact cctaagctga atcatttgtg taaaagaata agtgttgttc 1260

gggaaaaaaa aaaaaaaaaa aaa 1283

<210> 17
<211> 1180
<212> DNA
<213> Artificial Sequence

<220>
<223> na HRP isoenzym contig06351

<400> 17
aattaatctg attacaagat ttaagataga aaataataag atggttaggg caaatttagt 60

gagcgtgatt ctgttaatgc atgttattgt tgggtttcct tttcatgcga ggggcttaag 120

tatgacttat tacatgatga gctgtcctat ggctgaacaa attgtgaaaa acagtgttaa 180

caatgctctt caagccgatc ccactttagc cgcaggtctt atacgtatgt tgttccacga 240

ctgtttcatt gagggatgtg atgcgtcgat tctgctagat tcaacaaaag acaacactgc 300

ggaaaaggat tctcctgcga atctgagtct acgtggctac gagatcatag atgatgcaaa 360

agagaaagtt gagaatatgt gtccaggagt tgtatcttgc gcagatattg ttgccatggc 420

tgctagagat gctgtctttt gggctggtgg tccatattat gacataccaa aaggaagatt 480

tgatggtaaa agatcgaaga tagaagatac aagaaacctt ccttcacctt ttctcaatgc 540

ctctcaactc attcaaacct ttggcaaccg tggcttctct ccgcaagatg ttgttgctct 600

ctctggagca catacccttg gagttgcacg atgctcctcc ttcaaggcta gacttaccac 660

tccagattct tcactggact ccacttttgc aaacactctc actagaactt gcaatgcggg 720

ggacaatgca gagcaaccct ttgatgcgac ccgcaacgat ttcgacaatg cctacttcaa 780

tgcgcttcag aggaaatcag gagtcctctt ttcagaccag accttattca acactccaag 840

gaccaggaat cttgttaatg gttatgccct taatcaagct aagtttttct ttgatttcca 900

acaggccatg cgcaaaatga gcaatcttga tgttaaactt ggctctcaag gtgaaatacg 960

tcaaaattgc cggactatta actaagccta ggccgatttt tgtatttatg ctcccacctt 1020

taattatact tacctactct gtcattaatt cgagtcataa tgtcctatgc taccatgtaa 1080

aattagtgtg cctaatgtga tatgcagctg tattgtactt attgtttgtg ggtttcagat 1140

gtccatcatc aaaacgtaat atatatactt ggtgatcttg 1180

<210> 18
<211> 1212

<212> DNA
<213> Artificial Sequence

<220>
<223> na HRP isoenzym contig06117

<400> 18
cggccggggg accatcacaa agtcacctcc cttcttcttt cttctggatc tagtggaaac 60

tgacattatg gcaagaattg gaagctttct cgttgtcatc tctctcgctt gcgttcttac 120

tctctgcatc tgcgacgacg agagtaatta tggcggccaa gggaaactct tcccaggttt 180

ctacagcagc tcgtgcccta aagctgagga gatcgtgagg tctgttgtag ccaaagctgt 240

tgcaagagag actcgtatgg ctgcttctct catgaggctc cattttcacg actgttttgt 300

tcagggttgt gatggatcgt tgcttctaga cagcagtgga agtatagtta ctgagaagaa 360

ctctaaccct aacagcagat cagctcgtgg gtttgaagtt gttgacgaga tcaaagctgc 420

attggagaat gaatgcccta acactgtttc ttgcgctgac gccctaactc tagccgctag 480

agactcctct gttcttactg gtggaccaag ctggatggtt cctttgggaa gaagagattc 540

gacaagtgca agcttgagtg gatcaaataa caacattcct gcaccaaaca acactttcaa 600

cacaattctc tcgagattta acagccaagg tctcgatctc accaatgtcg ttgctctctc 660

cgggagccac acaattggat tctcaagatg tactagtttt agacagagac tttacaacca 720

atccggaaac ggaagtcccg acacaacctt agagcaatcc tacgctgcta acttgcgcca 780

tcggtgccct agatcaggtg gggaccagaa cctgtcggag cttgacatca acagtgctgg 840

aaggtttgat aacagctact ttaagaattt gattgagaac atgggactgt tgaattccga 900

ccaggtcttg ttctctagca acgacgaatc gagagagcta gtgaagaagt atgcagagga 960

tcaagaagag ttcttcgagc agttcgcgga atcgatggtc aagatgggga atatctctcc 1020

cttgactggt tcaagtggtc aaatcaggaa gaattgcagg aagattaact cttgatttca 1080

taatattgaa ttgggcgaaa taaaaatgag agatgttttg ggaagaaatt tatgtttgtg 1140

ttctttgttt tgtatgagtc atgagtgtat ttccttgtgt caaaaatttg aattttatga 1200

gtttttttc ag 1212

<210> 19
<211> 1066
<212> DNA
<213> Artificial Sequence

<220>
<223> na HRP isoenzym contig03523

<400> 19
gacccaacaa atctcacttt ctctcttccg ctacaacaat ggcggaactc aaatctctct 60

ccctcatcct cctcttcaca ctcctcacca ccaccatcga atctcgttta accacaaact 120

tctactcaaa atcatgtcca agattcttcg acatagtcag agatacaatc tcaaacaaac 180

aaatcacaac accaaccacg gcagccgcca caatccgtct cttcttccac gactgtttcc 240

ccaacggctg cgacgcctca atcctaatct cctcaactgc cttcaacacc gcagaacgtg 300

actcatcaat caatctctca cttcccggcg acggctttga cgtcatagtc cgagctaaaa 360

ccgcaatcga actcgcttgt cccaacactg tttcttgctc cgatataatc accgtcgcta 420

ctcgtgacct tcttgtcacc gtcggtggtc cttactacga cgtttacctc ggccgtcgtg 480

attcaagaat atctaaatca tctcttttaa ccgatcttct tcctcttcct tcatctccga 540

tctcaaaaac cattcgtcag tttgaatcta aaggtttcac tattcaagaa atggttgctc 600

ttagtggggc ccactcaatc gggttttcac attgtaaaga gtttgttaat cgggtcgccg 660

gtaataatac cgggtataac ccgagatttg ctcaggcgtt gaagcaagct tgttctaatt 720

acccgaaaga tccgacgtta tctgtgttta atgatattat gactccgaat aggtttgata 780

atatgtatta tcagaatatt ccaaagggtc ttgggttact tgaatcggat catgggttat 840

attctgaccc gagaacccga ccttttgttg atctttatgc tagagatcaa gatttgttct 900

ttaaagattt tgctagagct atgcagaagt tgagtctctt tggtgttaag actggtcgac 960

gaggagagat ccgacgaagg tgcgatgcga ttaactgagt ttatgttttt tcttttatgt 1020

atttattttt tggtattata ttggggaaga aatgtgagat ttgatg 1066

<210> 20
<211> 1215
<212> DNA
<213> Artificial Sequence

<220>
<223> na HRP isoenzym contig17517

<400> 20
gagcgtatta cggccgggga cacaacaaac aacaacaact tttacaaagc tcaaagagtt 60

tcttatttac caaaacaaaa acaaaatggg tcgtggttat aatttactat taattctagt 120

tacgttttta gtattggtcg cagctgtaac cgctcgaaga ccacgagttg ggttttatgg 180

gaatagatgc cgaaaggtag agtctatcgt gagatcggtg gttcgatctc atttccggtg 240

taatccggca aatgcaccgg gaattttgcg tatgcatttt cacgattgtt ttgtcaatgg 300

ctgcgatggc tcgatcctcc tcgctggtaa cacttcggag agaactgcgg gtcctaaccg 360

ttcattgaga gggttcgaag ctattgaaga agctaagact cggcttgaga atgcttgtcc 420

taataccgtt tcttgtgcgg atatcctcac ccttgctgct cgagacgccg tcgtttggac 480

cggtggaaaa ggttggtcgg tgccattggg acgtcttgac ggccgaagat cagaagcctc 540

agatgtaaat ttgcccggac caagcgaccc cgtcgctaag cagaagcaag actttgcagc 600

taaaaatctc aacaccttgg acctcgtaac tcttgttggt ggacacacaa ttggaactgc 660

tggttgcggt ttggtaagag gcagattctt taacttcaat ggcacgggac aacctgaccc 720

atcaatcgac ccgagtttcg ttcctctagt tcaggctcgt tgccctcaaa acggtaacgc 780

```
aacgacccga gtcgacttag acactggaag tgcaggtgat ttcgatacat cgtacctaag      840

taacgtgagg tcaagccgcg tggttctcca atccgatcta gtcctgtgga aggacaccga      900

aaccagagcc atcatagaac gtttattagg tttacgccgg cccgttttga ggttcggatc      960

agaatttggg aagtcgatga ccaagatgag tctcatagaa gttaagacta gactatcaga     1020

tggggagatt cgtagggttt gctctgcgat caattaagta ttaaaaacac acaaatgttt     1080

ggtttgattt tatcacttat tttatggaat aagcttgcta tagcattttg tggtcgatgg     1140

ttacatgttg aatttcaagt gtaagattgt acttgtaacg aaaatgtatt tataaaaatg     1200

tcgtttgttt cttta                                                      1215
```

```
<210>  21
<211>  1308
<212>  DNA
<213>  Artificial Sequence

<220>
<223>  na HRP isoenzym contig01350

<400>  21
cggccggggg aagtacaata atcaactcca actccaagtc tattcaaagt ctttgtttaa       60

cctaaacatg gcttcaaatc aacgtatttc cattctagtt ctcgtagtta catttttagt      120

gcaaggtaat tacaataacg tcgttgaagc acaactgacg cccaatttct actcaacctc      180

ttgccctaac ctcctctcca ccgtccaatc cgccgttaag tctgccgtta acagcgaggc      240

tcgaatgggt gcatctatcg tacgcctttt cttccacgat tgcttcgtca acggatgcga      300

tggttcgatt ttactagatg acacatcaag cttcacggga gaacaaaatg cgaacccaaa      360

ccgcaattcc gctcgcgggt ttaatgtgat cgacaacatc aaagcagcgg tcgagaaagc      420

atgtcccggg gtcgtgtctt gtgctgatat cttagccatc gcagctagag actccgtcgt      480

agtccttgga gggcctaact ggactgtgaa agtaggaaga agagatgcga gaacggcgag      540

tcaagcggcg gcgaatagca acattccggc gcccacttct agtctgagcc aactcattag      600

tagtttcagt gccgttggac tctccaccag agatatggtt gctctctccg gcgcgcacac      660

gatcgggcaa tcccgttgca cgagcttccg aacgagaatc tataacgaga caaacatcaa      720

cgccgcattc gccacaacac gtcaacgaac ttgccctaga acctccggct ccggcgacgg      780

gaatttagct ccacttgacg tcaccacggc ggcttctttc gacaacaact atttcaagaa      840

tctcatgact caaagaggtc ttctccattc cgaccaagag ctcttcaacg gcggctccac      900

tgactccata gtccgtggat acagcaacaa tccgtcaagc tttagctccg attttgcggc      960

ggcgatgatt aaaatgggtg atattagccc cttgaccggt agtagcggtg agatccggaa     1020

ggtttgcggg aggaccaact gatatttctt tttccctatt ggaatttgac ttttgttagt     1080

tgattcggtg agaataagat ttgatcttcc cccaagaaaa atcgaagaat gtttgttttc     1140
```

cttgttttgt gttcaagtgt gtattttcgc attaaaatct aatgataatt agctatgatt 1200

tccctttacg aaattttttt tattcgcatt tttaattagt gattacgtgt ctgaattcca 1260

ttggttatca taaataatta atcttttgaa gcgaaaaaaa aaaaaaaa 1308

<210> 22
<211> 1032
<212> DNA
<213> Artificial Sequence

<220>
<223> na HRP isoenzym contig01350_ALTERNATIVE

<400> 22
aagtacaata atcaactcca actccaagtc tattcaaagt ctttgtttat cttaaacatg 60

gcttcaaatc aacgtatttc cattctagtt ctcgtagtta cacttttagt gcaaggtaat 120

tacaataacg tcgttgaagc acaactgacg cccaatttct actcaacctc ttgccctaac 180

ctcctctcca ccgtccaatc cgccgttaag tctgccgtta acagcgaggc tcgaatgggt 240

gcatctatcg tacgcctttt cttccacgat tgcttcgtca acggatgcga tggttcgatt 300

ttactagatg acacatcaag cttcacggga gaacaaaacg cgaacccaaa ccgcaattcc 360

gctcgcgggt ttaatgtgat cgacaacatc aaagcagcgg tcgacaaagc atgtcccggg 420

gtcgtgtctt gtgctgatat cttagccatc gcagctagag actccgtcgt agtccttgga 480

gggcctaact ggactgtgaa agtaggaaga agagatgcga gaacggcgag tcaagcggcg 540

gcgaatagca acattccggc gcccacttct agtctgagcc aactcattag tagtttcagt 600

gccgttggac tctccaccag agatatggtt gctctctccg gcgcgcacac gatcgggcaa 660

tcccgttgcc cgagcttccg aacgagaatc tataacgaga caaacatcaa cgccgcattc 720

gccacaacac gtcaacgaac ttgccctaga acctccggct ccggcgacgg gaatttagct 780

ccacttgacg tcaccacggc ggcttctttc gacaacaact atttcaagaa tctcatgact 840

caaagaggtc ttctccattc cgaccaagag ctcttcaacg gcggctccac tgactccata 900

gtccgtggat acagcaacaa tccgtcaagc tttagctccg attttgcggc ggcgatgatt 960

aaaatgggtg atattagccc cttgaccggt agtagcggtg agatccggaa ggtttgcggg 1020

aggaccaact ga 1032

<210> 23
<211> 1051
<212> DNA
<213> Artificial Sequence

<220>
<223> na HRP isoenzym contig05508

<400> 23
tcactacaac taaaaacaca cttgatcttc tctaaaacat cgaaacataa atacaagatg 60

ggtttgatta gatcattatg cgtattcata actttcctca gttgtatcat cagctcggcc 120

catggccaag ccatctcgat ttctatcaca attaggatcg ggttttactt gaccacgtgt 180

cccacagctg aaatcattgt tcgaaacgcc gtgagagctg gtttcaattc tgacccgaga 240

atcgcacccg gaatattgag aatgcatttc cacgactgct tcgttcaagg ttgtgacggt 300

tcagtcctta tatcaggaag taacaccgag agaaccgccg ttccaaacct cagcctccgt 360

ggatttgaag tcatagaaaa cgccaaaacg cagctcgaag ccgcgtgccc aggagttgtc 420

tcttgtgctg atattttagc cttagctgct cgtgatactg tagtccttac gagagggata 480

ggctggcaag taccaacggg acgtagagat ggtcgagttt ctgtggcctc gaacgctaat 540

aatcttccag gtccccgtga ctccgtcgcc gttcaacaac agaaattctc cgctctcgga 600

ctcaataccc gcgatctcgt cgtcctcgcc ggaggacaca cgctcggaac agctggatgc 660

ggtgtattca gggacagact attcaataac acggatccta acgtcgacca gccatttttg 720

acgcagcttc aaacaaaatg tccccgaaac ggagacggtt cagtgcgcgt ggatctcgat 780

accggaagcg gaaccacttt tgataattcc tacttcatca acctaagtcg tggccgcgga 840

gtcctcgaat ccgatcatgt actttggacc gatccagcca ctagacccat cgtgcaacag 900

ttgatgagtt ctagtggcaa cttcaacgct gaatttgcga ggtcaatggt caagatgagt 960

aatatcggtg tggttacggg gactaatggg gaaattcgta aggtttgctc tgcgattaat 1020

taattaaccg attaaaatca gtggtgataa a 1051

<210> 24
<211> 1131
<212> DNA
<213> Artificial Sequence

<220>
<223> na HRP isoenzym contig08562

<220>
<221> misc_feature
<222> (98)..(98)
<223> n is a, c, g, or t

<400> 24
atcactcatc atcacaaagt cttcccattt cctcatcttc ttattgaaag taatggcaag 60

actcactagc attctccttc ttctttctct tctatgcntt ttttccctct ctgtctctgc 120

gacaagagct atggaggcaa actcttccct ggtttttacg cccactcatg cccacaagcc 180

ggggaaatcg tgagatcagt cgtagctaaa gctgttgcta gagagacccg tatggctgct 240

tctttgatga gacttcattt ccacgactgt ttcgttcagg gttgtgatgg ctctttgctt 300

ctagacagca gtgggaaaat agtgagtgag aaaggctcaa accctaacag cagatcagct 360

cgtgggttcg acgtagttga ccaaatcaaa gctgaattgg agaaacaatg ccctggaact 420

gtttcttgcg ctgatgctct aactctagcc gctagagact cctctgttct taccggtgga 480

```
ccgagctggg tggtttcatt aggaagaaga gattcaagaa gtgcaagctt gagtggttcg      540
aacaacaata tccctgcacc aaacaacact ttccagacca ttctatcgaa gtttaaccgt      600
caaggactcg atgtcaccga ccttgttgct ctctccggga gccacaccat tggattctcc      660
agatgcacga gtttcagaca aaggttgtac aaccagtccg gaaacggacg tccagacatg      720
acactggaac aatccttcgc tgctaacttg cgccaaaggt gtccgagatc cggcggggac      780
cagattctct cggtgctgga catcatcagc gccgcgaaat tcgacaacag ctacttcaag      840
aacttgatag agaacaaggg tttgttgaac tcggaccagg ttctgttcaa cagtaatgag      900
aaatctagag agcttgtgaa gaagtatgca gaggaccaag gagagttttt tgagcagttt      960
gcggaatcaa tgatcaagat gggaaatatt tctcccatga cgggttcgaa tggcgaaatc     1020
aggaagaatt gcaggaagat aaactcttga attcttgaaa tgaggaaagt attggggcga     1080
aaaaaaaata ttttggagaa gaaatgtgtg acatttgttt ttactttggt t              1131
```

```
<210>  25
<211>  1117
<212>  DNA
<213>  Artificial Sequence

<220>
<223>  na HRP isoenzym contig08562_ALTERNATIVE


<220>
<221>  misc_feature
<222>  (98)..(98)
<223>  n is a, c, g, or t

<400>  25
atcactcatc atcacaaagt cttcccattt cctcatcttc ttattgaaag taatggcaag       60
actcactagc attctccttc ttctttctct tctatgcntt ttttccctct ctgtctctgc      120
gacaagagct atggaggcaa actcttccct ggtttttacg cccactcatg cccacaagcc      180
ggggaaatcg tgagatcagt cgtagctaaa gctgttgcta gagagacccg tatggctgct      240
tctttgatga gacttcattt ccacgactgt ttcgttcagg gttgtgatgg ctctttgctt      300
ctagacagca gtgggagaat agtgagtgag aaaggctcaa accctaacag cagatcagct      360
cgtgggttcg acgtagttga ccaaatcaaa gctgaattgg agaaagaatg ccctggaact      420
gtttcttgcg ctgatgttct aactctagct gctagagact cctctgttct taccggtgga      480
ccaagctggg tcgttccatt gggaagaaga gattcgagaa gtgcaagctt aagtggttct      540
aacaacaaca tccctgcacc aaataacact ttccagacca ttctatcgaa gtttaatcgt      600
caaggactcg atgtcaccga ccttgttgct ctctctggga gccacaccat tggattctcg      660
agatgcacga gtttcagaca aagattgtac aaccagtccg gaaacggacg tccagacatg      720
acattggaac aatccttcgc tgctaacttg cgccaaaggt gtccgagatc cggcggagac      780
cagattctct cagtgttgga catcatcagc gccgcgaaat tcgacaacag ctacttcaag      840
```

aacttgatag aaaacaaggg tttgttgaac tcggaccagg ttctgttcag cagtaatgag 900

aaatctagag agcttgtgaa gaagtatgca gaggaccaag gagagttttt tgagcagttt 960

gcggaatcaa tgatcaagat gggaaatatt tctcccatga cgggttcgaa tggcgaaatc 1020

aggaagaatt gcaggaagat aaactcttga attcttgaaa tgaggaaagt attggggcga 1080

aaaaaaaata ttttggagaa gaaatgtgtg acatttg 1117

<210> 26
<211> 1066
<212> DNA
<213> Artificial Sequence

<220>
<223> na HRP isoenzym contig22489

<400> 26
actacaacta aaaacacact tgatcttctc taaaacacta aaattatata tccaatatgg 60

agtttgttag atcattatgc gtattcataa ctttcctcgg ttgtctcatc agctcggccc 120

atggccaagc cgccgcaagg cgacctggtc cgatttctgg cacaaggatc gggttttact 180

tgaccacgtg tcccaccgct gaaatcattg tccgaaacgc cgtgagagct ggtttcaatt 240

ctgacccaag aatcgcaccc ggaatattga gaatgcattt ccacgactgc ttcgttctag 300

gttgtgacgg ttcagtcctt atatcaggaa gtaacactga gagaaccgcc gttccgaacc 360

tcaacctccg tggatttgaa gtcatagaca acgccaaaac gcagctcgaa gccacatgcc 420

caggagttgt ctcttgtgct gatattttag ccttagctgc tcgtgatact gtagtcctta 480

cgagagggtt aggctggcaa gtaccaacgg gacgtagaga tggtcgagtt tctgtggcct 540

cgaacgctaa taatcttcca ggtccccgtg actccgtcgc cgttcaacaa cagaaattct 600

ccgctgtcgg actcaatacc cgcgatcttg tcgtcctcgc cggaggacac acgatcggaa 660

cagctggatg cggtgttttc agggacaggc tattcaataa cacggatcct aacgtcaacc 720

agctattttt gacgcagctt caaacacaat gtccccaaaa cggagacggt gcagtgcgcg 780

tggatctcga caccggaagt ggaaccactt ttgacaattc ctacttcatc aacctaagcc 840

gtggccgcgg agtcctcgaa tccgaccatg tactttggac cgatccagcc actagaccga 900

tcgtgcaaca gttgatgagt cctagaggca acttcaacgc tgaatttgcg aggtcaatgg 960

tcaggatgag taatatcggt gtggttacgg gggctaatgg ggaaattcgt agggtttgct 1020

ctgcggttaa ttaattaacc gattaaaatc agtggtgata aacaga 1066

<210> 27
<211> 1111
<212> DNA
<213> Artificial Sequence

<220>
<223> na HRP isoenzym contig02021

<400> 27
atcctgtctc tcttcactga cacaaaacca aagaagagta ttgagaaaca agatcgagga 60

aactactcaa tgaggacgat gaagcgattg aacgtggcgg tggcggttgc ggttacagcg 120

acggttctta tgggaatgtt aggatcatca gaggctcagc ttcaaatgaa tttctacgcg 180

aagagctgtc caaacgcaga gaaaataatt tcagatcata ttcaaaagca tatccctagt 240

ggtccttctc ttgcagctcc tctcatcaga atgcacttcc atgattgctt cgtcagggga 300

tgtgatggat cggtgttgat aaattcgaca tcagggaacg cagagaaaga ttcagcacca 360

aatctaacac ttagaggctt cggtttcgta gagaggatta agactcttct tgaagcagag 420

tgtcctaaga ctgtttcttg cgccgacatc atcgcactga ccgctagaga cgcagttgtt 480

gccaccggag gtccttcatg gaaagttccg acaggaagaa gagacggtag gatctcaaat 540

acgacggagg ctttgaacaa cattccaccg ccgacgagta atttcacgac gttacagcga 600

cttttcgcta atcaaggcct taatctcaaa gaccttgttc tgctttccgg agctcacacg 660

atcggtgtct cgcattgttc ttccatgaat actcgtctct acaacttctc gacgacagtc 720

aaacaagatc catctctgga tagcgagtac gcagcaaatc taaaggctaa caaatgtaag 780

agtcttaacg ataacaccac catcctcgag atggatcctg gtagtagcaa aacctttgat 840

ctcagttatt ataggcttgt cttgaagagg agaggtttgt ttcagtctga ttctgcctta 900

acgacaaact cagctacgtt gaagatgatc aacgacttgg tcaacggtcc tgaaaagaag 960

tttttaaagg ctttcgctaa gtcaatggag aagatgggga gagttaaagt gaagacgggc 1020

tcagccggtg tgattaggac acgttgttct gttgccggaa gttagttagt ttggtcggaa 1080

agtgatgttt tctgttcatt gttgttctgt t 1111

<210> 28
<211> 1399
<212> DNA
<213> Artificial Sequence

<220>
<223> na HRP isoenzym contig15901

<400> 28
attctcggtt aaacacaatt aattagtttt ctttttatct ttaaaaatat gcattcccct 60

tcttctactt cgtttacttg gatcttaatc acattgggat gtcttgcgtt ttatgcgtct 120

ttgtccgatg ctcagcttac ccctaccttc tacgacactt catgtcctaa tgtctcaaac 180

atcgtacgag acatcattat taatgagcta cgatcggacc ctcgtatcac cgcgagtatc 240

cttcgtcttc acttccacga ctgctttgtt aatggttgtg acgcatcgat attgttagac 300

aacacaacat catttctaac agagaaagat gcgcttggaa acgcaaactc ggctagagga 360

tttcctacag ttgacagaat caaggccgcg gtggagaggg catgcccaag aacagtttca 420

tgcgcagatg tgcttaccat tgcagctcaa caatctgtta atttggcagg aggtccttct 480

tggagggttc ctttgggaag aagagacagc ttacaagcat ttttagatct tgctaatgca 540

aatcttccag ctccattctt tacacttcca caacttaagg atgcctttgc aaaagttggc 600

ctcgaccgtc cttctgatct cgttgctctc tccggtggtc acacatttgg aaaaaatcag 660

tgtagattta ttatggacag attatacaac ttcagcaaca ccggactacc cgaccctacc 720

ctcaacacta cttaccttca aactcttcgt caacaatgtc ccctaaatgg aaaccaaagt 780

gtattggtgg atttcgatct gcgtacgcca acggttttcg ataacaaata ctatgtgaat 840

cttaaagagc aaaaaggtct cattcagagt gaccaagagt tgttctctag ccccaatgcc 900

actgacacaa tccccttggt gaggtcattt gctgatggca cacaaaaatt cttcaatgcg 960

tttgtggagg ctatgaatag gatgggaaat attacacctc ttacaggaac tcaaggagaa 1020

atcaggttga attgtagggt ggtgaactcc aactctctac tccatgatat agtggaggtc 1080

gttgactttg ttagctctat gtgagaaaag ttgagtcaat atctggctac cagagtacac 1140

gttaagataa ataaagcgct ctcaagatgt tacttgagaa ggagaagata tttattggtg 1200

tgtagagagt atctaagttg ttctctgttt ttatgtttga gttggccttt gaatgcgttt 1260

cgtgaatcgg tctaaacttt tatgggtttg tgtgtagaga gtatctaagt tgttctctgt 1320

ttttatgttt gagttggcct ttgaaatgcg tttcgtgaat cggtctaaac ttttatgggt 1380

ttgggacgtt ctatcctga 1399

<210> 29
<211> 1301
<212> DNA
<213> Artificial Sequence

<220>
<223> na HRP isoenzym contig25148

<400> 29
atgcattccc cttcttctac ttcgtttact tgggcaacct taatcacatt gggatgtctt 60

atgcttcatg catctttttc caatgctcaa cttaccccta ccttctacga caattcatgt 120

cctaacgtct caaacatagt acgggacatc attatcaatg agttacgatc ggaccctcgt 180

atcgccgcga gcatccttcg tcttcacttc cacgactgct ttgttaatgg ttgtgacgca 240

tcgatcttgt tagacaacac aacatcattt cgaacagaga aagatgcgtt tggaaacgca 300

aactcggctc gaggatttcc tgtggttgac agaatcaagg ccgcggtgga gagggcatgc 360

ccaagaactg tttcatgcgc agatgtgctt accattgcag ctcaacaatc tgttaatttg 420

gcaggaggtc cttcttggag ggttcctttg ggaagaagag acagccgaca agcattttta 480

gatctcgcta atgcgaatct tccagctcca tccttcacac ttccagaact taaggctgct 540

tttgcaaatg ttggcctcaa ccgtccttct gatctcgttg ctctctctgg tggtcacaca 600

tttggtaaaa atcaatgtcg atttattatg gacagattat acaacttcag caacaccgga 660

```
ctacccgacc ctaccctcaa cactacttac cttcaaactc ttcgtcaaca atgtccccgt      720
aatggtaacc aaagcgtctt ggtggatttc gatctgcgta cgccaacagt tttcgataac      780
aaatactatg tgaatcttaa agagcaaaaa ggtctcatcc agagtgacca agagttgttc      840
tctagcccca atgccactga cacaatcccc ttggtgagat catatgctga tggcacacaa      900
acattcttca atgcctttgt ggaggccatg aataggatgg gaaacattac acctcttaca      960
ggaactcaag gagaaatcag gttgaactgt agggtggtga actccaactc tctactccat     1020
gatatagtgg aggtcgttga ctttgttagc tctatgtgag aaaagttgac tcaatatctg     1080
gctaccagag tatacgttaa gataaataaa gcgctctcaa gatgttactt gagaaggaga     1140
atatctttat tggtgtgtag tgtgtagggt atctaagttg ttctctgttt ttatgtttgt     1200
gttggccttt gaatgcgttt cgtgaatcgg tctaaacttg tatgggtttg gacgttctat     1260
cctaataaag atgataaaat aaaataaaaa aatgttactc g                         1301
```

<210>  30
<211>  1166
<212>  DNA
<213>  Artificial Sequence

<220>
<223>  na HRP isoenzym contig04627

<400>  30

```
cggccgggga ctcaacttca aacctaacca aagaatttta tcttagagag caaagaaaat       60
gcattcctct tccagtttga taaaattggg atttcttctt cttcttctta atgtatcatt      120
gtctcacgct caactaagcc cttcatttta cgataaaaca tgtccacaag tctttgacat      180
tgcaaccaat accattaaaa ctgcgctgag atcagaccct cgcatcgctg cgagcatcct      240
tcgtcttcat ttccacgact gctttgttaa tggatgtgat gcatcgatat tgctagacaa      300
cactacatca tttaggactg agaaagatgc gtttgggaac gcaagatcag ctcgaggctt      360
cgacgtaatc gacacaatga aggctgccgt ggagaaagca tgtcctaaaa ccgtttcatg      420
tgctgatttg ctcgccattg cagctcaaaa atctgtcgtt ttggcgggag gtccttcatg      480
gaaggttcca agtggaagaa gagacagctt aagagggttc atggatctcg ctaatgataa      540
ccttccaggt ccatcctcta cacttcaagt acttaaggac aaattcagaa atgtcggact      600
cgaccgtcct tctgatctcg ttgctctttc tggtggtcac acctttggca aaaaccaatg      660
tcagttcata atggatcggc tttacaactt cagtaactcc ggtaaacccg acccaaccct      720
tgataaatcg tacctcagca cgctaagaaa acaatgccca cgtaatggaa acctgagtgt      780
attggtagat tttgatttac gtacaccgac aatctttgac aacaaatact atgtgaatct      840
caaagagaac aaaggtctta tccagagtga ccaagagtta ttctctagcc ctgatgcttc      900
tgacactatc cctttggtcc gagcatacgc tgatggtcaa ggaaagtttt ttgatgcatt      960
cgtggaggca atgataagga tgggaaatct ttcaccttca actgggaaac aaggagaaat     1020
```

tagattgaat tgtagagtgg tgaattctaa acctaaaatc atggatgtgg ttgatactaa 1080

tgactttgcc agctccatct gaagaaatga ctttctccta ataataaatg atcaatgtta 1140

tctttttctt tcaaaaaaaa aaaaaa 1166

<210> 31
<211> 1201
<212> DNA
<213> Artificial Sequence

<220>
<223> na HRP isoenzym contig01805

<400> 31
atcttctcaa cttaaaccaa taaaagataa gtttcctctt accaaaaatg catttctcta 60

cttcttcttc ttccttgtct acttggacaa ccctaataac attggggtgt cttatgcttc 120

attcatttaa gtccagtgct caactaaccc ctacctttta cgacagtacc tgccccagcg 180

tctttagcat cgtacgggac accatcgtga acgagctaag atcagatcct cgaattgctg 240

caagtatcct tcgtcttcac ttccacgact gcttcgttaa tggctgtgat gcatcgattt 300

tgttagacaa cacaacatca tttcgaacag agaaagatgc agctccaaac gcaaactcag 360

ctcgaggatt tccagtgatt gatacaatga aagctgcagt ggaaagagca tgtccaagaa 420

ccgtatcatg cgcagatttg cttaccatcg cagctcaaca atctgtgaat ttggcaggag 480

gtccttcttg gagggttcct ttggggagaa gagacagcgt acaagcattt tttgatcttg 540

ccaatacaaa tcttcccgct ccattcttca cgcttccaca acttaaggcc agctttagta 600

atgttggact tgaccgtcca gaagatctcg ttgcactctc tggtggtcac acatttggta 660

aaaaccaatg ccaatttatt atggacagac tatacaactt tagtaacact ggtttacccg 720

accctactct caacactact tatctccaga cacttcgtgt acaatgtccc cgtaatggta 780

accagtccgt cttggtcgat ttcgatctac gcacaccgac agtttttgac aacaaatact 840

atgtgaatct gaaagagcac aagggactta tccagaccga tcaagagttg ttctccagcc 900

ctaatgccgc tgatacaatc cccttggtaa gatcatatgc tgatggcact cagaagttct 960

tcaatgcttt tatggaggcc atgaacagaa tgggaaacat tacccctctc actggaactc 1020

aaggacagat caggcaaaat tgtagggtga tcaactccaa ctcgctgctc catgatattg 1080

ttgaaatcgt tgactttgtg agctctatgt aacaatagtt gtctcaatat atgtggcaac 1140

caaaattata tgttcttatg aaaataaaat gttctcgaaa cattacttaa gaatatttgg 1200

t 1201

<210> 32
<211> 1211
<212> DNA
<213> Artificial Sequence

<220>
<223> na HRP isoenzym contig00938

<400> 32
aggccggggg accatcagcc acactcccaa ctcaaatcat agtctatcat cctcctaaaa 60

attaaagaga aatggtggtt tctcctttct tttcttgcag tgctatggga gccctaatat 120

tgggttgcct tctgcttcaa gcatctaatg ctcagttgag gcctgacttc tactctagga 180

cttgcccatc tgttttcaat attattaaga atgtcatcgt cgatgaactg cagactgatc 240

ctcgcattgc cgctagtatc cttcgccttc actttcatga ctgctttgtt cgtggttgtg 300

atgcatcgat cctgctcgat acttccaaat cgttccgaac cgaaaaagat gctgctccaa 360

acgtaaattc ggctcgaggg ttcaatgtca tagatagaat gaaaacagca cttgagagag 420

cttgtcctag aacagtgtct tgtgcagata ttctcaccat cgcctctcaa atatcagtgc 480

ttttgtcggg aggtccatct tgggcagttc cgttggggag gagagacagc gtagaagctt 540

tctttgacct agctaataca gctcttccct ctccattttt cactcttgct caacttaaaa 600

aagcttttgc tgacgttggt ttaaaccgcc cctcagatct agtcgctctt tctggtggtc 660

acacatttgg aagagcacga tgcctatttg tgacagctcg tctctacaac ttcaacggta 720

caaacagacc agacccaact ctgaacccat cttacctcgc cgacctccgt cgattgtgcc 780

ctcgaaacgg aaacggcacc gttctggtca acttcgatgt catgactccg aatactttcg 840

ataatcaatt ctacactaat cttagaaatg ggaaaggtct gattcagagt gaccaagagc 900

tcttctcgac tccaggagcc gacacgatcc cactagtaaa cctatacagc agcaacacgt 960

tatcgttctt cggagcattc gctgatgcaa tgattaggat gggaaatctt agacctttga 1020

ctggaactca aggcgagata agacagaatt gtagggttgt gaattcgcga attaggggta 1080

tggagaacga tgatggagtt gtgagttcta tgtgattatg ttgggaatat atatcatata 1140

tggttatgta acaaatcata aaatgtgtgg gaacatgcat gtcgactaaa taaaagttct 1200

aacgagttgt g 1211

<210> 33
<211> 1173
<212> DNA
<213> Artificial Sequence

<220>
<223> na HRP isoenzym contig22684

<400> 33
aaaaaaaaaa aaaaacggaa ccgcctccct ataggtagtc gtattacggc cggggaccat 60

cagcaaaact cacgactcaa accatagtct gtcttcctct tgaaaaaaga aaaatggggt 120

tttctccttc attttcttcc agttctatag gagtcctaat attgggttgc cttctgcttc 180

aagcttcaaa ctctaatgct aagttgaggc ctgacttcta cttaaagaca tgtccatcag 240

ttttccaaat cattgggaat gtcatcgtcg atgaactgca gagtgatcct cgtattgcag 300

```
ctagtctcct tcgccttcac ttccatgact gttttgttcg tggttgtgat gcatcggtcc      360

tgctcgacaa ttccacatca tttcagtccg agaaagatgc tgctccaaac gcaaattcgg      420

ctcgagggtt cgacgtcgta gatagaatga aagcagccct tgagaaagct tgtcctggaa      480

cagtgtcttg tgcagatgtt cttgccatct ccgctcaaat atcagtgctt ttgtcgggag      540

gcccatggtg gccggttttg ttggggagga gagacggcgt agaagctttc ttcgatttgg      600

ctaatacagc tcttcccaat ccatttgccc ctcttactga acttaaagaa aaatttgctg      660

acgttggcct aaagcgcgcc tcagatctag ttgctctttc cggtgctcac acatttggaa      720

gagcacaatg tctacttgtg acacctcgtc tctacaactt cagcggcacc aataaaccag      780

acccaactct gaacccatct tacctcgtcg aactccgtcg attgtgccct caaaacggaa      840

acggcaccgt tctgctcaac ttcgatctcg tgactccaaa tgctttcgat cgtcaatact      900

acaccaatct tcgaaatggg aaaggtctga ttcagagtga ccaagagctc ttctcgactc      960

caggagccga cacgatccca ctagtaaacc tatacagcaa gaacacgttc gcgttcttcg     1020

gtgcattcgt tgacgcaata attaggatgg gaaatattca acctttgact ggaactcaag     1080

gcgagataag acagaattgt agggttgtga attcgcgaat taagggtatg gagaacgacg     1140

gtggagttgt gagttctatt tgattatgtt ggg                                  1173
```

<210> 34
<211> 477
<212> DNA
<213> Artificial Sequence

<220>
<223> na HRP isoenzym contig01351

<400> 34

```
aaatacaata atcaactcca agtctattca aagtctttgt ttaacctaac catgacttca       60

aatcaactta tttccattct agttctcgta gttacacttt tagtgcaagg taattacaat      120

aacatcgtcg aagcacaact cacggccaat ttctactcaa cctcttgccc taatctcctc      180

tctaccgtcc aagccgccgt taagtctgcc gttaacagcg aggctcgaat gggtgcatcg      240

atcgtacgcc ttttcttcca cgattgcttc gtcaacggct gcgacggttc tattctacta      300

gatgacacat caagcttcac gggagaacaa aacgcgaacc caaaccgcaa ttccgctcgc      360

gggtttaatg tgatcgacaa catcaaatca gcggttgaga aagcatgtcc cggggtcgtg      420

tcgtgtgctg atatcttagc catcgcagct agagactccg tcgtactact tggaggg         477
```

<210> 35
<211> 1199
<212> DNA
<213> Artificial Sequence

<220>
<223> na HRP isoenzym HRPC1AsynK232Q_K241N

<400> 35
aacgatgaga ttcccatcta ttttcaccgc tgtcttgttc gctgcctcct ctgcattggc 60
tgcccctgtt aacactacca ctgaagacga gactgctcaa attccagctg aagcagttat 120
cggttactct gaccttgagg gtgatttcga cgtcgctgtt ttgcctttct ctaactccac 180
taacaacggt ttgttgttca ttaacaccac tatcgcttcc attgctgcta aggaagaggg 240
tgtctctctc gagaagagag aggccgaagc tcaacttact ccaaccttct acgataactc 300
ttgtcctaat gtgtccaaca tcgttagaga caccattgtc aatgaattga gatcagatcc 360
acgtattgct gcatctatct tgagacttca ctttcatgac tgcttcgtca acggttgtga 420
tgcttccatc ttgctggaca acactacctc tttcagaact gagaaggacg ctttcggtaa 480
tgccaactct gctagaggat ttccagtcat tgacagaatg aaggctgccg ttgaatctgc 540
atgtcctaga actgtgtcat gtgctgacct tctgactatt gccgctcagc aatctgttac 600
cttagctggt ggaccatcct ggagagttcc attgggtcgt agagactccc ttcaagcctt 660
tctggacctt gcaaatgcta acttgcctgc tccattcttt accttacctc aattgaaaga 720
ctctttcaga aacgttggtc ttaacagatc atccgacttg gttgccttat ctggaggtca 780
cacctttggt aagaaccaat gtagattcat catggatcgt ctgtacaact tctctaacac 840
cggtttgcca gatcctactc tgaacaccac ttacttgcaa accttaagag gtttgtgccc 900
acttaacgga aatctgtctg ctctggttga cttcgatttg cgtactccta ccatcttcga 960
caaccaatac tatgtcaact tggaggaaca gaacggtctt atccaatctg accaggagtt 1020
gttctcctct cctaacgcta ctgataccat tccattggtg agatccttcg caaactccac 1080
tcaaaccttc tttaacgctt tcgtcgaggc aatggacaga atgggtaaca ttactccttt 1140
gaccggtact caaggacaga ttagattgaa ctgccgtgtt gtcaactcta actcataat 1199

<210> 36
<211> 1199
<212> DNA
<213> Artificial Sequence

<220>
<223> na HRP isoenzym HRPC1AsynK232Q_K241F

<400> 36
aacgatgaga ttcccatcta ttttcaccgc tgtcttgttc gctgcctcct ctgcattggc 60
tgcccctgtt aacactacca ctgaagacga gactgctcaa attccagctg aagcagttat 120
cggttactct gaccttgagg gtgatttcga cgtcgctgtt ttgcctttct ctaactccac 180
taacaacggt ttgttgttca ttaacaccac tatcgcttcc attgctgcta aggaagaggg 240
tgtctctctc gagaagagag aggccgaagc tcaacttact ccaaccttct acgataactc 300
ttgtcctaat gtgtccaaca tcgttagaga caccattgtc aatgaattga gatcagatcc 360
acgtattgct gcatctatct tgagacttca ctttcatgac tgcttcgtca acggttgtga 420

```
tgcttccatc ttgctggaca acactacctc tttcagaact gagaaggacg ctttcggtaa      480

tgccaactct gctagaggat ttccagtcat tgacagaatg aaggctgccg ttgaatctgc      540

atgtcctaga actgtgtcat gtgctgacct tctgactatt gccgctcagc aatctgttac      600

cttagctggt ggaccatcct ggagagttcc attgggtcgt agagactccc ttcaagcctt      660

tctggacctt gcaaatgcta acttgcctgc tccattcttt accttacctc aattgaaaga      720

ctctttcaga aacgttggtc ttaacagatc atccgacttg gttgccttat ctggaggtca      780

cacctttggt aagaaccaat gtagattcat catggatcgt ctgtacaact tctctaacac      840

cggtttgcca gatcctactc tgaacaccac ttacttgcaa accttaagag gtttgtgccc      900

acttaacgga aatctgtctg ctctggttga cttcgatttg cgtactccta ccatcttcga      960

caaccaatac tatgtcaact tggaggaaca gttcggtctt atccaatctg accaggagtt     1020

gttctcctct cctaacgcta ctgataccat tccattggtg agatccttcg caaactccac     1080

tcaaaccttc tttaacgctt tcgtcgaggc aatggacaga atgggtaaca ttactccttt     1140

gaccggtact caaggacaga ttagattgaa ctgccgtgtt gtcaactcta actcataat      1199


<210>  37
<211>  1199
<212>  DNA
<213>  Artificial Sequence

<220>
<223>  na HRP isoenzym HRPC1AsynK232N

<400>  37
aacgatgaga ttcccatcta ttttcaccgc tgtcttgttc gctgcctcct ctgcattggc       60

tgcccctgtt aacactacca ctgaagacga gactgctcaa attccagctg aagcagttat      120

cggttactct gaccttgagg gtgatttcga cgtcgctgtt ttgcctttct ctaactccac      180

taacaacggt ttgttgttca ttaacaccac tatcgcttcc attgctgcta aggaagaggg      240

tgtctctctc gagaagagag aggccgaagc tcaacttact ccaaccttct acgataactc      300

ttgtcctaat gtgtccaaca tcgttagaga caccattgtc aatgaattga gatcagatcc      360

acgtattgct gcatctatct tgagacttca ctttcatgac tgcttcgtca acggttgtga      420

tgcttccatc ttgctggaca acactacctc tttcagaact gagaaggacg ctttcggtaa      480

tgccaactct gctagaggat ttccagtcat tgacagaatg aaggctgccg ttgaatctgc      540

atgtcctaga actgtgtcat gtgctgacct tctgactatt gccgctcagc aatctgttac      600

cttagctggt ggaccatcct ggagagttcc attgggtcgt agagactccc ttcaagcctt      660

tctggacctt gcaaatgcta acttgcctgc tccattcttt accttacctc aattgaaaga      720

ctctttcaga aacgttggtc ttaacagatc atccgacttg gttgccttat ctggaggtca      780

cacctttggt aagaaccaat gtagattcat catggatcgt ctgtacaact tctctaacac      840
```

cggtttgcca gatcctactc tgaacaccac ttacttgcaa accttaagag gtttgtgccc 900

acttaacgga aatctgtctg ctctggttga cttcgatttg cgtactccta ccatcttcga 960

caacaactac tatgtcaact tggaggaaca gaagggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 38
<211> 1199
<212> DNA
<213> Artificial Sequence

<220>
<223> na HRP isoenzym HRPC1AsynK232N_K241N

<400> 38
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

tgtctctctc gagaagagag aggccgaagc tcaacttact ccaaccttct acgataactc 300

ttgtcctaat gtgtccaaca tcgttagaga caccattgtc aatgaattga gatcagatcc 360

acgtattgct gcatctatct tgagacttca ctttcatgac tgcttcgtca acggttgtga 420

tgcttccatc ttgctggaca acactacctc tttcagaact gagaaggacg ctttcggtaa 480

tgccaactct gctagaggat ttccagtcat tgacagaatg aaggctgccg ttgaatctgc 540

atgtcctaga actgtgtcat gtgctgacct tctgactatt gccgctcagc aatctgttac 600

cttagctggt ggaccatcct ggagagttcc attgggtcgt agagactccc ttcaagcctt 660

tctggacctt gcaaatgcta acttgcctgc tccattcttt accttacctc aattgaaaga 720

ctctttcaga aacgttggtc ttaacagatc atccgacttg gttgccttat ctggaggtca 780

cacctttggt aagaaccaat gtagattcat catggatcgt ctgtacaact tctctaacac 840

cggtttgcca gatcctactc tgaacaccac ttacttgcaa accttaagag gtttgtgccc 900

acttaacgga aatctgtctg ctctggttga cttcgatttg cgtactccta ccatcttcga 960

caacaactac tatgtcaact tggaggaaca gaacggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 39
<211> 1199

<212> DNA
<213> Artificial Sequence

<220>
<223> na HRP isoenzym HRPC1AsynK232N_K241F

<400> 39
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

tgtctctctc gagaagagag aggccgaagc tcaacttact ccaaccttct acgataactc 300

ttgtcctaat gtgtccaaca tcgttagaga caccattgtc aatgaattga gatcagatcc 360

acgtattgct gcatctatct tgagacttca ctttcatgac tgcttcgtca acggttgtga 420

tgcttccatc ttgctggaca acactacctc tttcagaact gagaaggacg ctttcggtaa 480

tgccaactct gctagaggat ttccagtcat tgacagaatg aaggctgccg ttgaatctgc 540

atgtcctaga actgtgtcat gtgctgacct tctgactatt gccgctcagc aatctgttac 600

cttagctggt ggaccatcct ggagagttcc attgggtcgt agagactccc ttcaagcctt 660

tctggacctt gcaaatgcta acttgcctgc tccattcttt accttacctc aattgaaaga 720

ctctttcaga aacgttggtc ttaacagatc atccgacttg gttgccttat ctggaggtca 780

cacctttggt aagaaccaat gtagattcat catggatcgt ctgtacaact tctctaacac 840

cggtttgcca gatcctactc tgaacaccac ttacttgcaa accttaagag gtttgtgccc 900

acttaacgga aatctgtctg ctctggttga cttcgatttg cgtactccta ccatcttcga 960

caacaactac tatgtcaact tggaggaaca gttcggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 40
<211> 1199
<212> DNA
<213> Artificial Sequence

<220>
<223> na HRP isoenzym HRPC1AsynK174R_K241N

<400> 40
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

```
tgtctctctc gagaagagag aggccgaagc tcaacttact ccaaccttct acgataactc      300
ttgtcctaat gtgtccaaca tcgttagaga caccattgtc aatgaattga gatcagatcc      360
acgtattgct gcatctatct tgagacttca ctttcatgac tgcttcgtca acggttgtga      420
tgcttccatc ttgctggaca acactacctc tttcagaact gagaaggacg ctttcggtaa      480
tgccaactct gctagaggat ttccagtcat tgacagaatg aaggctgccg ttgaatctgc      540
atgtcctaga actgtgtcat gtgctgacct tctgactatt gccgctcagc aatctgttac      600
cttagctggt ggaccatcct ggagagttcc attgggtcgt agagactccc ttcaagcctt      660
tctggacctt gcaaatgcta acttgcctgc tccattcttt accttacctc aattgaaaga      720
ctctttcaga aacgttggtc ttaacagatc atccgacttg gttgccttat ctggaggtca      780
cacctttggt cgtaaccaat gtagattcat catggatcgt ctgtacaact tctctaacac      840
cggtttgcca gatcctactc tgaacaccac ttacttgcaa accttaagag gtttgtgccc      900
acttaacgga aatctgtctg ctctggttga cttcgatttg cgtactccta ccatcttcga      960
caacaagtac tatgtcaact tggaggaaca gaacggtctt atccaatctg accaggagtt     1020
gttctcctct cctaacgcta ctgataccat tccattggtg agatccttcg caaactccac     1080
tcaaaccttc tttaacgctt tcgtcgaggc aatggacaga atgggtaaca ttactccttt     1140
gaccggtact caaggacaga ttagattgaa ctgccgtgtt gtcaactcta actcataat      1199
```

<210> 41
<211> 1199
<212> DNA
<213> Artificial Sequence

<220>
<223> na HRP isoenzym HRPC1AsynK174R_K241F

<400> 41

```
aacgatgaga ttcccatcta ttttcaccgc tgtcttgttc gctgcctcct ctgcattggc       60
tgcccctgtt aacactacca ctgaagacga gactgctcaa attccagctg aagcagttat      120
cggttactct gaccttgagg gtgatttcga cgtcgctgtt ttgcctttct ctaactccac      180
taacaacggt ttgttgttca ttaacaccac tatcgcttcc attgctgcta aggaagaggg      240
tgtctctctc gagaagagag aggccgaagc tcaacttact ccaaccttct acgataactc      300
ttgtcctaat gtgtccaaca tcgttagaga caccattgtc aatgaattga gatcagatcc      360
acgtattgct gcatctatct tgagacttca ctttcatgac tgcttcgtca acggttgtga      420
tgcttccatc ttgctggaca acactacctc tttcagaact gagaaggacg ctttcggtaa      480
tgccaactct gctagaggat ttccagtcat tgacagaatg aaggctgccg ttgaatctgc      540
atgtcctaga actgtgtcat gtgctgacct tctgactatt gccgctcagc aatctgttac      600
cttagctggt ggaccatcct ggagagttcc attgggtcgt agagactccc ttcaagcctt      660
tctggacctt gcaaatgcta acttgcctgc tccattcttt accttacctc aattgaaaga      720
```

```
ctctttcaga aacgttggtc ttaacagatc atccgacttg gttgccttat ctggaggtca      780

cacctttggt cgtaaccaat gtagattcat catggatcgt ctgtacaact tctctaacac      840

cggtttgcca gatcctactc tgaacaccac ttacttgcaa accttaagag gtttgtgccc      900

acttaacgga aatctgtctg ctctggttga cttcgatttg cgtactccta ccatcttcga      960

caacaagtac tatgtcaact tggaggaaca gttcggtctt atccaatctg accaggagtt     1020

gttctcctct cctaacgcta ctgataccat tccattggtg agatccttcg caaactccac     1080

tcaaaccttc tttaacgctt tcgtcgaggc aatggacaga atgggtaaca ttactccttt     1140

gaccggtact caaggacaga ttagattgaa ctgccgtgtt gtcaactcta actcataat      1199


<210>  42
<211>  1199
<212>  DNA
<213>  Artificial Sequence

<220>
<223>  na HRP isoenzym HRPC1AsynK174R_K232Q

<400>  42
aacgatgaga ttcccatcta ttttcaccgc tgtcttgttc gctgcctcct ctgcattggc       60

tgcccctgtt aacactacca ctgaagacga gactgctcaa attccagctg aagcagttat      120

cggttactct gaccttgagg gtgatttcga cgtcgctgtt ttgcctttct ctaactccac      180

taacaacggt ttgttgttca ttaacaccac tatcgcttcc attgctgcta aggaagaggg      240

tgtctctctc gagaagagag aggccgaagc tcaacttact ccaaccttct acgataactc      300

ttgtcctaat gtgtccaaca tcgttagaga caccattgtc aatgaattga gatcagatcc      360

acgtattgct gcatctatct tgagacttca ctttcatgac tgcttcgtca acggttgtga      420

tgcttccatc ttgctggaca acactacctc tttcagaact gagaaggacg ctttcggtaa      480

tgccaactct gctagaggat ttccagtcat tgacagaatg aaggctgccg ttgaatctgc      540

atgtcctaga actgtgtcat gtgctgacct tctgactatt gccgctcagc aatctgttac      600

cttagctggt ggaccatcct ggagagttcc attgggtcgt agagactccc ttcaagcctt      660

tctggacctt gcaaatgcta acttgcctgc tccattcttt accttacctc aattgaaaga      720

ctctttcaga aacgttggtc ttaacagatc atccgacttg gttgccttat ctggaggtca      780

cacctttggt cgtaaccaat gtagattcat catggatcgt ctgtacaact tctctaacac      840

cggtttgcca gatcctactc tgaacaccac ttacttgcaa accttaagag gtttgtgccc      900

acttaacgga aatctgtctg ctctggttga cttcgatttg cgtactccta ccatcttcga      960

caaccaatac tatgtcaact tggaggaaca gaagggtctt atccaatctg accaggagtt     1020

gttctcctct cctaacgcta ctgataccat tccattggtg agatccttcg caaactccac     1080

tcaaaccttc tttaacgctt tcgtcgaggc aatggacaga atgggtaaca ttactccttt     1140
```

gaccggtact caaggacaga ttagattgaa ctgccgtgtt gtcaactcta actcataat 1199

<210> 43
<211> 1199
<212> DNA
<213> Artificial Sequence

<220>
<223> na HRP isoenzym HRPC1AsynK174R_K232N

<400> 43
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

tgtctctctc gagaagagag aggccgaagc tcaacttact ccaaccttct acgataactc 300

ttgtcctaat gtgtccaaca tcgttagaga caccattgtc aatgaattga gatcagatcc 360

acgtattgct gcatctatct tgagacttca ctttcatgac tgcttcgtca acggttgtga 420

tgcttccatc ttgctggaca acactacctc tttcagaact gagaaggacg ctttcggtaa 480

tgccaactct gctagaggat ttccagtcat tgacagaatg aaggctgccg ttgaatctgc 540

atgtcctaga actgtgtcat gtgctgacct tctgactatt gccgctcagc aatctgttac 600

cttagctggt ggaccatcct ggagagttcc attgggtcgt agagactccc ttcaagcctt 660

tctggacctt gcaaatgcta acttgcctgc tccattcttt accttacctc aattgaaaga 720

ctctttcaga aacgttggtc ttaacagatc atccgacttg gttgccttat ctggaggtca 780

cacctttggt cgtaaccaat gtagattcat catggatcgt ctgtacaact tctctaacac 840

cggtttgcca gatcctactc tgaacaccac ttacttgcaa accttaagag gtttgtgccc 900

acttaacgga aatctgtctg ctctggttga cttcgatttg cgtactccta ccatcttcga 960

caacaattac tatgtcaact tggaggaaca gaagggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 44
<211> 1199
<212> DNA
<213> Artificial Sequence

<220>
<223> na HRP isoenzym HRPC1AsynK174Q_K241N

<400> 44
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

tgtctctctc gagaagagag aggccgaagc tcaacttact ccaaccttct acgataactc 300

ttgtcctaat gtgtccaaca tcgttagaga caccattgtc aatgaattga gatcagatcc 360

acgtattgct gcatctatct tgagacttca ctttcatgac tgcttcgtca acggttgtga 420

tgcttccatc ttgctggaca acactacctc tttcagaact gagaaggacg ctttcggtaa 480

tgccaactct gctagaggat ttccagtcat tgacagaatg aaggctgccg ttgaatctgc 540

atgtcctaga actgtgtcat gtgctgacct tctgactatt gccgctcagc aatctgttac 600

cttagctggt ggaccatcct ggagagttcc attgggtcgt agagactccc ttcaagcctt 660

tctggacctt gcaaatgcta acttgcctgc tccattcttt accttacctc aattgaaaga 720

ctctttcaga aacgttggtc ttaacagatc atccgacttg gttgccttat ctggaggtca 780

cacctttggt cagaaccaat gtagattcat catggatcgt ctgtacaact tctctaacac 840

cggtttgcca gatcctactc tgaacaccac ttacttgcaa accttaagag gtttgtgccc 900

acttaacgga aatctgtctg ctctggttga cttcgatttg cgtactccta ccatcttcga 960

caacaagtac tatgtcaact tggaggaaca gaacggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 45
<211> 1199
<212> DNA
<213> Artificial Sequence

<220>
<223> na HRP isoenzym HRPC1AsynK174Q_K241F

<400> 45
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

tgtctctctc gagaagagag aggccgaagc tcaacttact ccaaccttct acgataactc 300

ttgtcctaat gtgtccaaca tcgttagaga caccattgtc aatgaattga gatcagatcc 360

acgtattgct gcatctatct tgagacttca ctttcatgac tgcttcgtca acggttgtga 420

tgcttccatc ttgctggaca acactacctc tttcagaact gagaaggacg ctttcggtaa 480

tgccaactct gctagaggat ttccagtcat tgacagaatg aaggctgccg ttgaatctgc 540

atgtcctaga actgtgtcat gtgctgacct tctgactatt gccgctcagc aatctgttac 600

cttagctggt ggaccatcct ggagagttcc attgggtcgt agagactccc ttcaagcctt 660

tctggacctt gcaaatgcta acttgcctgc tccattcttt accttacctc aattgaaaga 720

ctctttcaga aacgttggtc ttaacagatc atccgacttg gttgccttat ctggaggtca 780

cacctttggt cagaaccaat gtagattcat catggatcgt ctgtacaact tctctaacac 840

cggtttgcca gatcctactc tgaacaccac ttacttgcaa accttaagag gtttgtgccc 900

acttaacgga aatctgtctg ctctggttga cttcgatttg cgtactccta ccatcttcga 960

caacaagtac tatgtcaact tggaggaaca gttcggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 46
<211> 1199
<212> DNA
<213> Artificial Sequence

<220>
<223> na HRP isoenzym HRPC1AsynK174Q_K232Q

<400> 46
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

tgtctctctc gagaagagag aggccgaagc tcaacttact ccaaccttct acgataactc 300

ttgtcctaat gtgtccaaca tcgttagaga caccattgtc aatgaattga gatcagatcc 360

acgtattgct gcatctatct tgagacttca ctttcatgac tgcttcgtca acggttgtga 420

tgcttccatc ttgctggaca acactacctc tttcagaact gagaaggacg ctttcggtaa 480

tgccaactct gctagaggat ttccagtcat tgacagaatg aaggctgccg ttgaatctgc 540

atgtcctaga actgtgtcat gtgctgacct tctgactatt gccgctcagc aatctgttac 600

cttagctggt ggaccatcct ggagagttcc attgggtcgt agagactccc ttcaagcctt 660

tctggacctt gcaaatgcta acttgcctgc tccattcttt accttacctc aattgaaaga 720

ctctttcaga aacgttggtc ttaacagatc atccgacttg gttgccttat ctggaggtca 780

cacctttggt cagaaccaat gtagattcat catggatcgt ctgtacaact tctctaacac 840

cggtttgcca gatcctactc tgaacaccac ttacttgcaa accttaagag gtttgtgccc 900

acttaacgga aatctgtctg ctctggttga cttcgatttg cgtactccta ccatcttcga 960

caaccagtac tatgtcaact tggaggaaca gaagggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 47
<211> 1199
<212> DNA
<213> Artificial Sequence

<220>
<223> na HRP isoenzym HRPC1AsynK174Q_K232N

<400> 47
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

tgtctctctc gagaagagag aggccgaagc tcaacttact ccaaccttct acgataactc 300

ttgtcctaat gtgtccaaca tcgttagaga caccattgtc aatgaattga gatcagatcc 360

acgtattgct gcatctatct tgagacttca ctttcatgac tgcttcgtca acggttgtga 420

tgcttccatc ttgctggaca acactacctc tttcagaact gagaaggacg ctttcggtaa 480

tgccaactct gctagaggat ttccagtcat tgacagaatg aaggctgccg ttgaatctgc 540

atgtcctaga actgtgtcat gtgctgacct tctgactatt gccgctcagc aatctgttac 600

cttagctggt ggaccatcct ggagagttcc attgggtcgt agagactccc ttcaagcctt 660

tctggacctt gcaaatgcta acttgcctgc tccattcttt accttacctc aattgaaaga 720

ctctttcaga aacgttggtc ttaacagatc atccgacttg gttgccttat ctggaggtca 780

cacctttggt cagaaccaat gtagattcat catggatcgt ctgtacaact tctctaacac 840

cggtttgcca gatcctactc tgaacaccac ttacttgcaa accttaagag gtttgtgccc 900

acttaacgga aatctgtctg ctctggttga cttcgatttg cgtactccta ccatcttcga 960

caacaattac tatgtcaact tggaggaaca gaagggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 48
<211> 1199
<212> DNA
<213> Artificial Sequence

<220>
<223> na HRP isoenzym HRPC1Asyn_T110V

<400> 48
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

tgtctctctc gagaagagag aggccgaagc tcaacttact ccaaccttct acgataactc 300

ttgtcctaat gtgtccaaca tcgttagaga caccattgtc aatgaattga gatcagatcc 360

acgtattgct gcatctatct tgagacttca ctttcatgac tgcttcgtca acggttgtga 420

tgcttccatc ttgctggaca acactacctc tttcagaact gagaaggacg ctttcggtaa 480

tgccaactct gctagaggat ttccagtcat tgacagaatg aaggctgccg ttgaatctgc 540

atgtcctaga actgtgtcat gtgctgacct tctgactatt gccgctcagc aatctgttgt 600

gttagctggt ggaccatcct ggagagttcc attgggtcgt agagactccc ttcaagcctt 660

tctggacctt gcaaatgcta acttgcctgc tccattcttt accttacctc aattgaaaga 720

ctctttcaga aacgttggtc ttaacagatc atccgacttg gttgccttat ctggaggtca 780

cacctttggt aagaaccaat gtagattcat catggatcgt ctgtacaact tctctaacac 840

cggtttgcca gatcctactc tgaacaccac ttacttgcaa accttaagag gtttgtgccc 900

acttaacgga aatctgtctg ctctggttga cttcgatttg cgtactccta ccatcttcga 960

caacaagtac tatgtcaact tggaggaaca gaagggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 49
<211> 1199
<212> DNA
<213> Artificial Sequence

<220>
<223> na HRP isoenzym HRPC1Asyn_K241F

<400> 49
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

tgtctctctc gagaagagag aggccgaagc tcaacttact ccaaccttct acgataactc 300

ttgtcctaat gtgtccaaca tcgttagaga caccattgtc aatgaattga gatcagatcc 360

acgtattgct gcatctatct tgagacttca ctttcatgac tgcttcgtca acggttgtga 420

tgcttccatc ttgctggaca acactacctc tttcagaact gagaaggacg ctttcggtaa 480

tgccaactct gctagaggat ttccagtcat tgacagaatg aaggctgccg ttgaatctgc 540

atgtcctaga actgtgtcat gtgctgacct tctgactatt gccgctcagc aatctgttac 600

cttagctggt ggaccatcct ggagagttcc attgggtcgt agagactccc ttcaagcctt 660

tctggacctt gcaaatgcta acttgcctgc tccattcttt accttacctc aattgaaaga 720

ctctttcaga aacgttggtc ttaacagatc atccgacttg gttgccttat ctggaggtca 780

cacctttggt aagaaccaat gtagattcat catggatcgt ctgtacaact tctctaacac 840

cggtttgcca gatcctactc tgaacaccac ttacttgcaa accttaagag gtttgtgccc 900

acttaacgga aatctgtctg ctctggttga cttcgatttg cgtactccta ccatcttcga 960

caacaagtac tatgtcaact tggaggaaca gttcggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 50
<211> 1199
<212> DNA
<213> Artificial Sequence

<220>
<223> na HRP isoenzym HRPC1Asyn

<400> 50
aacgatgaga ttcccatcta ttttcaccgc tgtcttgttc gctgcctcct ctgcattggc 60

tgcccctgtt aacactacca ctgaagacga gactgctcaa attccagctg aagcagttat 120

cggttactct gaccttgagg gtgatttcga cgtcgctgtt ttgcctttct ctaactccac 180

taacaacggt ttgttgttca ttaacaccac tatcgcttcc attgctgcta aggaagaggg 240

tgtctctctc gagaagagag aggccgaagc tcaacttact ccaaccttct acgataactc 300

ttgtcctaat gtgtccaaca tcgttagaga caccattgtc aatgaattga gatcagatcc 360

acgtattgct gcatctatct tgagacttca ctttcatgac tgcttcgtca acggttgtga 420

tgcttccatc ttgctggaca acactacctc tttcagaact gagaaggacg ctttcggtaa 480

tgccaactct gctagaggat ttccagtcat tgacagaatg aaggctgccg ttgaatctgc 540

atgtcctaga actgtgtcat gtgctgacct tctgactatt gccgctcagc aatctgttac 600

cttagctggt ggaccatcct ggagagttcc attgggtcgt agagactccc ttcaagcctt 660

tctggacctt gcaaatgcta acttgcctgc tccattcttt accttacctc aattgaaaga 720

ctctttcaga aacgttggtc ttaacagatc atccgacttg gttgccttat ctggaggtca 780

cacctttggt aagaaccaat gtagattcat catggatcgt ctgtacaact tctctaacac 840

cggtttgcca gatcctactc tgaacaccac ttacttgcaa accttaagag gtttgtgccc 900

acttaacgga aatctgtctg ctctggttga cttcgatttg cgtactccta ccatcttcga 960

caacaagtac tatgtcaact tggaggaaca gaagggtctt atccaatctg accaggagtt 1020

gttctcctct cctaacgcta ctgataccat tccattggtg agatccttcg caaactccac 1080

tcaaaccttc tttaacgctt tcgtcgaggc aatggacaga atgggtaaca ttactccttt 1140

gaccggtact caaggacaga ttagattgaa ctgccgtgtt gtcaactcta actcataat 1199

<210> 51
<211> 2065
<212> DNA
<213> Artificial Sequence

<220>
<223> na HRP isoenzym C1A

<400> 51
tcatcttctc agtaatatag ttttcccctt taaaaatgca tttctcttct tcttctactt 60

tgttcacttg tataacctta atcccattgg tatgtcttat tcttcatgct tctttgtctg 120

atgctcaact tacccctacc ttctacgaca attcatgtcc taatgtctct aacatcgtac 180

gggatactat tgtcaatgag ctaagatcag accctcgtat tgccgcgagc atccttcgtc 240

ttcacttcca cgactgcttt gttaatgtaa attactactt ttcatatttc tatttcgtta 300

cgaattatta tatgtttcat gtaactgatt tttaaatgct tatctattca tactgtctaa 360

ttacatttat catttgaatt tgattattaa atcagtttta atgtgattaa tataaatttt 420

aaaaaaatgt gattaatata taaacatttt gatgataaaa tttttaattt gtttattttt 480

cttttttttt actgaataaa atttttttta gtggatgaca aaaatgttac attgttgccg 540

tggtaaagat tcaactgtat atggatgtaa tacattgaat aataatttat taaatgatat 600

attggatttt tgaaagggtt gtgacgcatc gatcttgtta gacaacacaa catcatttcg 660

aacagagaaa gatgcgtttg gaaacgcaaa ctcggcaaga ggatttccag tgattgatag 720

aatgaaagcc gcggtggaga gtgcatgccc aagaaccgtt tcatgcgcag atttgctcac 780

cattgcagct caacaatctg tcactttggt atgctccatt gatcccacta acttttattc 840

attacaatta ttgcttttaa ttttaatata tcaaatagtc actttacata tcaacacggc 900

aacctaagtt tagaaaaaca aaaatcggga tatttttagc tgtttgagaa cgacatggaa 960

attatttagt tgtttcagaa gggtcacaat caatatatag tatcaacttt ccaatcatat 1020

gactagtatt caaaattaat cgggagatat ttgaaacgcg tggtccccgt agagaaaact 1080

tgaaatgttt aatatcactg aaaaaaatta atctcattaa caagaatata cttacttggc 1140

aatactacgt ttttagttaa ccaaaaagaa tggttttcat atttttcaag aaacagtgga 1200

gacttaaaaa cttcacttca agcatatata tcatcaatga atttaaatta cacatatttt 1260

tctcttaaca cattgaaact tctaaatgag gaaaataata atcaaaacaa aatggttatt 1320

attataggcg ggaggtcctt cttggagagt tcctttgggc agaagagata gcttacaagc 1380

atttctggat cttgctaatg caaatcttcc agctccattc ttcacacttc cacaacttaa 1440

agacagcttt agaaatgttg gcctcaaccg ttcttctgat ctcgttgcac tgtccggtaa 1500

ttaacaaaaa tatattaaac acaccatttg atatagttgt atttagtgag tttattaaag 1560

atctctcttt cttttgttag gggccacac atttggtaaa aatcagtgtc ggtttattat 1620

ggacagatta tacaacttca gcaacaccgg tttacccgat cctactctca acactactta 1680

tctccaaact cttcgtggac tatgtcccct caatggtaat ctaagcgctt tggtggattt 1740

tgatctacgt acgccaacga tttttgacaa caaatactat gtgaatctcg aagagcaaaa 1800

aggacttatc caaagcgacc aagagttgtt ctctagcccc aatgccactg acacaatccc 1860

tttggtgaga tcatttgcta atagcacaca aacattcttc aatgcgtttg tggaggcgat 1920

ggataggatg ggaaacatta cacctcttac aggaactcaa ggacagatca ggttgaattg 1980

tagggtggtg aactccaact ctctactcca tgatatggtg gaggtcgttg actttgttag 2040

ctctatgtga gcatagtcga cgcca 2065

<210> 52
<211> 1554
<212> DNA
<213> Artificial Sequence

<220>
<223> na HRP isoenzym SynPDI_N314H_DNA

<400> 52
atgcaattca actgggacat caagacagtt gcatccatcc tttctgcctt gactcttgca 60

caagcttctg accaagaagc tattgctcca gaagactctc acgttgtgaa gttgactgag 120

gctactttcg agtccttcat cacctctaac cctcatgtat tggcagagtt tttcgcacct 180

tggtgtggtc actgcaagaa gttaggtcct gagttggtat ctgctgctga gattttgaag 240

gacaatgagc aagtcaagat tgcccagatt gactgcactg aggaaaagga gttgtgtcaa 300

ggttacgaga ttaagggtta ccctaccttg aaggtgtttc acggtgaagt tgaggttcca 360

tctgactacc aaggacaaag acagtcccaa tccatcgttt cctacatgct gaagcaatct 420

cttccacctg tatccgagat caacgcaact aaggacttgg atgacaccat tgcagaggct 480

aaagagccag ttatcgtcca agtccttcca gaagatgcat ccaacttgga gtccaatacg 540

acctttacg gagttgcagg tacattgcgt gagaagttca cctttgtctc taccaagtct 600

actgactacg ctaagaagta cacctctgac tctactccag cttacctgtt ggttagacct 660

ggagaagagc catccgtcta ctctggtgag gaattggatg agactcacct tgttcactgg 720

atagacatcg agtccaaacc tctgtttgga gacattgatg gttccacctt caagtcttat 780

gctgaggcta acattcctct tgcctactat ttctacgaga acgaagagca aagagcagca 840

gctgctgaca tcattaagcc atttgctaag gagcaaagag gcaagattaa cttcgttggt 900

ttggatgcag tcaagttcgg caaacatgcc aagaaccttc acatggatga ggagaagttg 960

ccactgttcg ttatccatga cttggtttcc aacaagaaat tcggtgttcc tcaagaccaa 1020

gaactgacca acaaggacgt tacagagctg attgagaagt tcattgcagg tgaagcagaa 1080

cccattgtca agtctgagcc tattccagag atacaagagg agaaggtctt caagttggta 1140

ggtaaagctc acgatgaagt cgttttcgac gaatccaagg acgttttggt caagtactat 1200

gctccttggt gtggtcactg taagagaatg gcaccagctt acgaagagct tgctactttg 1260

tacgccaatg atgaggatgc atcttccaag gtggttattg ccaagcttga tcacactctt 1320

aacgacgttg ataacgtgga catacaggga tacccaacct tgatccttta tcctgctggt 1380

gacaagtcta acccacagtt atacgacggt tccagagacc ttgagtcttt ggctgagttt 1440

gtcaaggaaa gaggtactca caaggttgac gctttagctt tgagacctgt tgaagaggag 1500

aaagaagctg aggaagaagc tgagtctgaa gctgatgctc acgacgaatt gtaa 1554

<210> 53
<211> 517
<212> PRT
<213> Artificial Sequence

<220>
<223> aa HRP isoenzym SynPDI_N314H_Protein

<400> 53

Met Gln Phe Asn Trp Asp Ile Lys Thr Val Ala Ser Ile Leu Ser Ala
1               5                   10                  15

Leu Thr Leu Ala Gln Ala Ser Asp Gln Glu Ala Ile Ala Pro Glu Asp
            20                  25                  30

Ser His Val Val Lys Leu Thr Glu Ala Thr Phe Glu Ser Phe Ile Thr
        35                  40                  45

Ser Asn Pro His Val Leu Ala Glu Phe Phe Ala Pro Trp Cys Gly His
    50                  55                  60

Cys Lys Lys Leu Gly Pro Glu Leu Val Ser Ala Ala Glu Ile Leu Lys
65                  70                  75                  80

Asp Asn Glu Gln Val Lys Ile Ala Gln Ile Asp Cys Thr Glu Glu Lys
                85                  90                  95

Glu Leu Cys Gln Gly Tyr Glu Ile Lys Gly Tyr Pro Thr Leu Lys Val
            100                 105                 110

Phe His Gly Glu Val Glu Val Pro Ser Asp Tyr Gln Gly Gln Arg Gln
        115                 120                 125

Ser Gln Ser Ile Val Ser Tyr Met Leu Lys Gln Ser Leu Pro Pro Val
    130                 135                 140

Ser Glu Ile Asn Ala Thr Lys Asp Leu Asp Asp Thr Ile Ala Glu Ala
145                 150                 155                 160

Lys Glu Pro Val Ile Val Gln Val Leu Pro Glu Asp Ala Ser Asn Leu
                165                 170                 175

Glu Ser Asn Thr Thr Phe Tyr Gly Val Ala Gly Thr Leu Arg Glu Lys
            180                 185                 190

Phe Thr Phe Val Ser Thr Lys Ser Thr Asp Tyr Ala Lys Lys Tyr Thr
        195                 200                 205

Ser Asp Ser Thr Pro Ala Tyr Leu Leu Val Arg Pro Gly Glu Glu Pro
    210                 215                 220

Ser Val Tyr Ser Gly Glu Glu Leu Asp Glu Thr His Leu Val His Trp
225                 230                 235                 240

Ile Asp Ile Glu Ser Lys Pro Leu Phe Gly Asp Ile Asp Gly Ser Thr
                245                 250                 255

Phe Lys Ser Tyr Ala Glu Ala Asn Ile Pro Leu Ala Tyr Tyr Phe Tyr
            260                 265                 270

Glu Asn Glu Glu Gln Arg Ala Ala Ala Ala Asp Ile Ile Lys Pro Phe
        275                 280                 285

Ala Lys Glu Gln Arg Gly Lys Ile Asn Phe Val Gly Leu Asp Ala Val
    290                 295                 300

Lys Phe Gly Lys His Ala Lys Asn Leu His Met Asp Glu Glu Lys Leu
305                 310                 315                 320

Pro Leu Phe Val Ile His Asp Leu Val Ser Asn Lys Lys Phe Gly Val
                325                 330                 335

Pro Gln Asp Gln Glu Leu Thr Asn Lys Asp Val Thr Glu Leu Ile Glu
            340                 345                 350

Lys Phe Ile Ala Gly Glu Ala Glu Pro Ile Val Lys Ser Glu Pro Ile
        355                 360                 365

Pro Glu Ile Gln Glu Glu Lys Val Phe Lys Leu Val Gly Lys Ala His
    370                 375                 380

Asp Glu Val Val Phe Asp Glu Ser Lys Asp Val Leu Val Lys Tyr Tyr
385 390 395 400

Ala Pro Trp Cys Gly His Cys Lys Arg Met Ala Pro Ala Tyr Glu Glu
405 410 415

Leu Ala Thr Leu Tyr Ala Asn Asp Glu Asp Ala Ser Ser Lys Val Val
420 425 430

Ile Ala Lys Leu Asp His Thr Leu Asn Asp Val Asp Asn Val Asp Ile
435 440 445

Gln Gly Tyr Pro Thr Leu Ile Leu Tyr Pro Ala Gly Asp Lys Ser Asn
450 455 460

Pro Gln Leu Tyr Asp Gly Ser Arg Asp Leu Glu Ser Leu Ala Glu Phe
465 470 475 480

Val Lys Glu Arg Gly Thr His Lys Val Asp Ala Leu Ala Leu Arg Pro
485 490 495

Val Glu Glu Glu Lys Glu Ala Glu Glu Glu Ala Glu Ser Glu Ala Asp
500 505 510

Ala His Asp Glu Leu
515

<210> 54
<211> 22
<212> DNA
<213> Artificial Sequence

<220>
<223> na Adaptor Primer1

<400> 54
gtaatacgac tcactatagg gc 22

<210> 55
<211> 19
<212> DNA
<213> Artificial Sequence

<220>
<223> na Adaptor Primer2

<400> 55
actatagggc acgcgtggt 19

<210> 56
<211> 31
<212> PRT
<213> Artificial Sequence

<220>
<223> aa N-terminal signal peptide 01805

<400> 56

Met His Phe Ser Thr Ser Ser Ser Ser Leu Ser Thr Trp Thr Thr Leu
1 5 10 15

Ile Thr Leu Gly Cys Leu Met Leu His Ser Phe Lys Ser Ser Ala
20 25 30

<210> 57
<211> 29
<212> PRT
<213> Artificial Sequence

<220>
<223> na signal peptide 22684

<400> 57

Met Gly Phe Ser Pro Ser Phe Ser Ser Ser Ser Ile Gly Val Leu Ile
1 5 10 15

Leu Gly Cys Leu Leu Leu Gln Ala Ser Asn Ser Asn Ala
20 25

<210> 58
<211> 27
<212> PRT
<213> Artificial Sequence

<220>
<223> aa signal peptide E5

<400> 58

Met Val Val Ser Pro Phe Phe Ser Cys Ser Ala Met Gly Ala Leu Ile
1 5 10 15

Leu Gly Cys Leu Leu Leu Gln Ala Ser Asn Ala
20 25

<210> 59
<211> 15
<212> PRT
<213> Artificial Sequence

<220>
<223> na C-terminal peptide

<400> 59

Leu Leu His Asp Met Val Glu Val Val Asp Phe Val Ser Ser Met
1 5 10 15

<210> 60

```
<211>  1218
<212>  DNA
<213>  Artificial Sequence

<220>
<223>  na A2ACstrep

<400>  60
gaattcgaaa cgatgagatt cccatctatt ttcaccgctg tcttgttcgc tgcctcctct        60
gcattggctg cccctgttaa cactaccact gaagacgaga ctgctcaaat tccagctgaa       120
gcagttaggt tactctgacc ttgagggtga tttcgacgtc gctgttttgc ctttctctaa       180
ctccactaac aacggtttgt tgttcattaa caccactatc gcttccattg ctgctaagga       240
agagggtgtc tcttgagaag agagaggccg aagctcaatt gaatgcaact ttctattccg       300
gaacctgccc aaacgcctct gcaatcgtta gatccactat tcaacaggcc ttccaatcag       360
acactagaat tggtgcatct tattagactg cactttcacg attgtttcgt gaacggttgt       420
gacgcctcta tcttgctgga tgactcagga tctattcagt cagagaagaa tgctggtcca       480
aacgccaatt ccgctagagg tttcaatgtg gttgataaca tcaagaccgc tttggaaaac       540
acttgccctg gtgtggtttc tttccgacat cttggctttg gcatctgaag catccgtttc       600
tttgactgga ggtccatcct ggactgtctt attgggtaga cgtgactctc ttaccgctaa       660
cttagctggt gccaactcag ctattcctcc catttgaagg tctttctaac attacctcta       720
agttctccgc tgtcggactg aacaccaacg accttgttgc actgtctggt gcccatacct       780
ttggacgtgc tagatgtggt gtcttcaaca ataatgttca acttttctgg aactggtaac       840
ccagacccta ccttgaactc cactttgctg tcttccttgc agcaactttg tccacagaac       900
ggttctgctt caactatcac taacctggac ttatctaccc tgcgctttcg acaacaatta       960
ctttgccaac cttcaatcta acaacggttt gttacaatcc gatcaagagt tgttctctac      1020
cactggttct gctactatcg cagttgtcac ctctttcgct tctaacaaat ttgttctttc      1080
aagccttcgc tcaatcaatg atcaacatgg gtaacatttc tcctttgacc ggttctaacg      1140
gtgagattag acttgattgc aagaaagtta acggatcctc tgcatggtcc ccccacgttc      1200
gagaagtaat gcggccgc                                                    1218
```